(19) Europäisches Patentamt
European Patent Office
Office européen des brevets

(11) EP 0 626 939 B1

(12) EUROPEAN PATENT SPECIFICATION

(45) Date of publication and mention of the grant of the patent: **07.11.2001 Bulletin 2001/45**

(21) Application number: **94902931.8**

(22) Date of filing: **22.12.1993**

(51) Int Cl.[7]: **C07C 43/253**, A61K 31/44, C07C 43/235, C07D 213/30

(86) International application number: **PCT/GB93/02625**

(87) International publication number: **WO 94/14742 (07.07.1994 Gazette 1994/15)**

(54) **TRI-SUBSTITUTED PHENYL DERIVATIVES AS PHOSPHODIESTERASE INHIBITORS**

TRi-SUBSTITUIERTE PHENYLDERIVATE ALS PHOSPHODIESTERASEINHIBITOREN

DERIVES DU PHENYLE TRI-SUBSTITUE UTILISES COMME INHIBITEURS DE LA PHOSPHODIESTERASE

(84) Designated Contracting States:
**AT BE CH DE DK ES FR GR IE IT LI LU MC NL PT SE**

(30) Priority: **23.12.1992 GB 9226831**
**02.08.1993 GB 9315966**

(43) Date of publication of application:
**07.12.1994 Bulletin 1994/49**

(73) Proprietor: **CELLTECH THERAPEUTICS LIMITED**
**Slough, Berkshire SL1 4EN (GB)**

(72) Inventors:
- **WARRELLOW, Graham John**
  **Northwood, Middlesex HA6 3QU (GB)**
- **BOYD, Ewan Campbell**
  **Slough, Berkshire SL1 9HB (GB)**
- **ALEXANDER, Rikki Peter**
  **Bucks HP12 3HY (GB)**

(74) Representative: **Marsden, John Christopher et al**
**Frank B. Dehn & Co.,**
**European Patent Attorneys,**
**179 Queen Victoria Street**
**London EC4V 4EL (GB)**

(56) References cited:
**WO-A-91/15451**
**WO-A-92/19594**
**US-A- 4 971 959**
**US-A- 5 128 358**

- **JOURNAL OF THE INDIAN CHEMICAL SOCIETY vol. 58, no. 3 , 1981 P.B. SATTUR ET AL. 'SYNTHESIS AND PHARMACOLOGY OF 2,5-DISUBSTITUTED 1,3,4-OXADIAZOLES'**
- **CHEMICAL ABSTRACTS. REGISTRY HANDBOOK - NUMBER SECTION. PRINTED ISSUES COLUMBUS US**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 0 626 939 B1

## Description

This invention relates to a novel series of tri-substituted phenyl derivatives, to processes for their preparation, to pharmaceutical compositions containing them, and to their use in medicine.

Many hormones and neurotransmitters modulate tissue function by elevating intra-cellular levels of adenosine 3', 5'-cyclic monophosphate (cAMP). The cellular levels of cAMP are regulated by mechanisms which control synthesis and breakdown. The synthesis of cAMP is controlled by adenyl cyclase which may be directly activated by agents such as forskolin or indirectly activated by the binding of specific agonists to cell surface receptors which are coupled to adenyl cyclase. The breakdown of cAMP is controlled by a family of phosphodiesterase (PDE) isoenzymes, which also control the breakdown of guanosine 3',5'-cyclic monophosphate (cGMP). To date, seven members of the family have been described (PDE I-VII) the distribution of which varies from tissue to tissue. This suggests that specific inhibitors of PDE isoenzymes could achieve differential elevation of cAMP in different tissues, [for reviews of PDE distribution, structure, function and regulation, see Beavo & Reifsnyder (1990) TIPS, 11: 150-155 and Nicholson et al (1991) TIPS, 12: 19-27].

There is clear evidence that elevation of cAMP in inflammatory leukocytes leads to inhibition of their activation. Furthermore, elevation of cAMP in airway smooth muscle has a spasmolytic effect. In these tissues, PDE IV plays a major role in the hydrolysis of cAMP. It can be expected, therefore, that selective inhibitors of PDE IV would have therapeutic effects in inflammatory diseases such as asthma, by achieving both anti-inflammatory and bronchodilator effects.

The design of PDE IV inhibitors has met with limited success to date, in that many of the potential PDE IV inhibitors which have been synthesised have lacked potency and/or have been capable of inhibiting more than one type of PDE isoenzyme in a non-selective manner. Lack of a selective action has been a particular problem given the widespread role of cAMP in vivo and what is needed are potent selective PDE IV inhibitors with an inhibitory action against PDE IV and little or no action against other PDE isoenzymes.

WO 92/19594 describes N-substituted pyrrolidinones as PDE IV inhibitors.

We have now found a novel series of tri-substituted phenyl derivatives, members of which compared to known structurally similar compounds are potent inhibitors of PDE IV at concentrations at which they have little or no inhibitory action on other PDE isoenzymes. These compounds inhibit the human recombinant PDE IV enzyme and also elevate cAMP in isolated leukocytes. Certain compounds prevent inflammation in the lungs induced by carrageenan, platelet-activating factor (PAF), interleukin-5 (IL-5) or antigen challenge. These compounds also suppress the hyperresponsiveness of airway smooth muscle seen in inflamed lungs. Advantageously, compounds according to the invention have good oral activity and at orally effective doses exhibit little or none of the side-effects associated with known PDE IV inhibitors, such as rolipram. The compounds of the invention are therefore of use in medicine, especially in the prophylaxis and treatment of asthma.

Thus according to one aspect of the invention, we provide a compound of formula (1) which is an inhibitor of PDE IV:

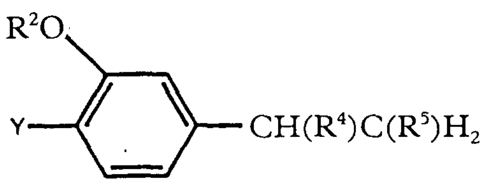

(1)

wherein

Y represents a group $OR^1$, where $R^1$ is a $C_{1-6}$ alkyl group optionally substituted by one or more halogen atoms;
$R^2$ is a $C_{1-6}$ alkyl or $C_{2-6}$ alkenyl group (each optionally substituted by one , two or three halogen atoms or hydroxyl or $C_{1-6}$ alkoxy groups) or a $C_{3-8}$ cycloalkyl or $C_{3-8}$ cycloalkenyl group (each optionally substituted by one, two or three halogen atoms or $C_{1-6}$ alkyl, hydroxyl or $C_{1-6}$ alkoxy groups);
$R^4$ and $R^5$, which may be the same or different, is each a group Ar, where Ar is an optionally substituted monocyclic or bicyclic aryl group optionally containing one or more heteroatoms selected from oxygen, sulphur or nitrogen atoms;

and the salts, solvates, hydrates and N-oxides thereof; provided that -Y and $-OR^2$ are not both methoxy groups.

It will be appreciated that the compounds of formula (1) may have one or more chiral centres, depending on the nature of the groups $R^4$ and $R^5$. Where one or more chiral centres is present, enantiomers or diastereomers may exist, and the invention is to be understood to extend to all such enantiomers, diastereomers and mixtures thereof, including racemates.

$R^1$ is an optionally substituted straight or branched $C_{1-6}$alkyl group, such as a methyl, ethyl, n-propyl or i-propyl group. Optional substitutents which may be present on $R^1$ groups include one or more halogen atoms, e.g. fluorine, or chlorine atoms. Particular substituted alkyl groups include for example $-CH_2F$, $-CH_2Cl$, $-CHF_2$, $-CHCl_2$, $-CF_3$ or $-CCl_3$ groups.

Alkyl groups represented by $R^2$ in the compounds of formula (1) include optionally substituted straight or branched $C_{1-6}$ alkyl groups, e.g. $C_{1-3}$ alkyl groups such as methyl or ethyl groups. Optional substituents on these groups include one, two or three substituents selected from halogen atoms, e.g. fluorine, chlorine, bromine or iodine atoms, or hydroxyl or $C_{1-6}$ alkoxy e.g. $C_{1-3}$ alkoxy such as methoxy or ethoxy groups.

Alkenyl groups represented by $R^2$ in the compounds of formula (1) include optionally substituted straight or branched $C_{2-6}$alkenyl groups such as ethenyl, propen-1-yl and 2-methylpropen-1-yl. Optional substituents include those described above in relation to the group $R^2$.

When $R^2$ in the compounds of formula (1) is an optionally substituted cycloalkyl or cycloalkenyl group it may be for example a $C_{3-8}$cycloalkyl group such as a cyclobutyl, cyclopentyl or cyclohexyl group or a $C_{3-8}$ cycloalkenyl group containing for example one or two double bonds such as a 2-cyclobuten-1-yl, 2-cyclopenten-1-yl, 3-cyclopenten-1-yl, 2,4-cyclopentadien-1-yl, 2-cyclohexen-1-yl, 3-cyclohexen-1-yl, 2,4-cyclohexadien-1-yl or 3,5-cyclohexadien-1-yl group, each cycloalkyl or cycloalkenyl group being optionally substituted by one, two or three substituents selected from halogen atoms, e.g. fluorine, chlorine, bromine or iodine atoms, straight or branched $C_{1-6}$alkyl e.g. $C_{1-3}$alkyl such as methyl or ethyl, hydroxyl or $C_{1-6}$alkoxy e.g. $C_{1-3}$alkoxy such as methoxy or ethoxy groups.

In the compounds of formula (1) the groups $R^4$ and $R^5$ may each independently be a group -Ar.

Monocyclic or bicyclic aryl groups represented by the group Ar in compounds of formula (1) include for example C6-12 optionally substituted aryl groups, for example optionally substituted phenyl, 1-or 2-naphthyl, indenyl or isoindenyl groups.

When the monocyclic or bicyclic aryl group Ar contains one or more heteroatoms it may be for example a $C_{1-9}$ optionally substituted heteroaryl group containing for example one, two, three or four heteroatoms selected from oxygen, sulphur or nitrogen atoms. In general, Ar heteroaryl groups may be for example monocyclic or bicyclic heteroaryl groups. Monocyclic heteroaryl groups include for example five- or six-membered heteroaryl groups containing one, two, three or four heteroatoms selected from oxygen, sulphur or nitrogen atoms.

Examples of heteroaryl groups represented by Ar include pyrrolyl, furyl, thienyl, imidazolyl, N-methylimidazolyl, N-ethylimidazolyl, oxazolyl, isoxazolyl, thiazolyl, isothiazolyl, pyrazolyl, 1,2,3-triazolyl, 1,2,4-triazolyl, 1,2,3-oxadiazolyl, 1,2,4-oxadiazolyl, 1,2,5-oxadiazolyl, 1,3,4-oxadiazolyl, pyridyl, pyrimidinyl, pyridazinyl, pyrazinyl, 1,3,5-triazinyl, 1,2,4-triazinyl, 1,2,3-triazinyl, benzofuryl, isobenzofuryl, benzothienyl, isobenzothienyl, indolyl, isoindolyl, benzimidazolyl, benzothiazolyl, benzoxazolyl, quinazolinyl, naphthyridinyl, pyrido[3,4-b]pyridyl, pyrido[3,2-b]pyridyl, pyrido [4,3-b]pyridyl, quinolinyl, isoquinolinyl, tetrazolyl, 5,6,7,8-tetrahydroquinolinyl and 5,6,7,8-tetrahydroisoquinolinyl.

The heteroaryl group represented by Ar may be attached to the remainder of the molecule of formula (1) through any ring carbon or heteroatom as appropriate. Thus, for example, when the group Ar is a pyridyl group it may be a 2-pyridyl, 3-pyridyl or 4-pyridyl group. When it is a thienyl group it may be a 2-thienyl or 3-thienyl group, and, similarly, when it is a furyl group it may be a 2-furyl or 3-furyl group.

When in compounds of formula (1) the Ar group is a nitrogen-containing heterocycle it may be possible to form quaternary salts, for example N-alkyl quaternary salts and the invention is to be understood to extend to such salts. Thus for example when the group Ar is a pyridyl group, pyridinium salts may be formed, for example N-alkylpyridinium salts such as N-methylpyridinium.

The aryl or heteroaryl groups represented by Ar in compounds of formula (1) may each optionally be substituted by one, two, three or more substituents $[R^{10}]$. The substituent $R^{10}$ may be selected from an atom or group $R^{13}$ or $-Alk^1(R^{13})_m$ wherein $R^{13}$ is a halogen atom, or an amino ($-NH_2$), substituted amino, nitro, cyano, hydroxyl (-OH), substituted hydroxyl, cycloalkoxy, formyl [HC(O)-], carboxyl ($-CO_2H$), esterified carboxyl, thiol (-SH), substituted thiol, $-C(O)Alk^1$, $-SO_3H$, $-SO_2Alk^1$, $-SO_2NH_2$, $-SO_2NHAlk^1$, $-SO_2N[Alk^1]_2$, $-CONH_2$, $-CONHAlk^1$, $-CON[Alk^1]_2$, $-NHSO_2H$, $-NHSO_2Alk^1$, $-N[SO_2Alk^1]_2$, $-NHSO_2NH_2$, $-NHSO_2NHAlk^1$, $-NHSO_2N[Alk^1]_2$, $-NHC(O)Alk^1$, or $-NHC(O)OAlk^1$ group; $Alk^1$ is a straight or branched $C_{1-6}$alkylene, $C_{2-6}$alkenylene, or $C_{2-6}$alkynylene chain optionally interrupted by one, two, or three -O-, or -S- atoms or -S(O)p-, [where p is an integer 1 or 2] or $-N(R^8)$- groups; and m is zero or an integer 1, 2 or 3.

When in the group $-Alk^1(R^{13})_m$ m is an integer 1, 2 or 3, it is to be understood that the substituent or substituents $R^{13}$ may be present on any suitable carbon atom in $-Alk^1$. Where more than one $R^{13}$ substitutent is present these may be the same or different and may be present on the same or different carbon atom in $Alk^1$. Clearly, when m is zero and no substituent $R^{13}$ is present or when $Alk^1$ forms part of a group such as $-SO_2Alk^1$ the alkylene, alkenylene or alkynylene

chain represented by $Alk^1$ becomes an alkyl, alkenyl or alkynyl group.

When $R^{13}$ is a substituted amino group it may be a group $-NH[Alk^1(R^{13a})_m]$ [where $Alk^1$ and m are as defined above and $R^{13a}$ is as defined above for $R^{13}$ but is not a substituted amino, a substituted hydroxyl or a substituted thiol group] or a group $-N[Alk^1(R^{13a})_m]_2$ wherein each $-Alk^1(R^{13a})_m$ group is the same or different.

When $R^{13}$ is a halogen atom it may be for example a fluorine, chlorine, bromine, or iodine atom.

When $R^{13}$ is a cycloalkoxy group it may be for example a $C_{5-7}$cycloalkoxy group such as a cyclopentyloxy or cyclohexyloxy group.

When $R^{13}$ is a substituted hydroxyl or substituted thiol group it may be a group $-OAlk^1(R^{13a})_m$ or $-SAlk^1(R^{13a})_m$ respectively, where $Alk^1$, $R^{13a}$ and m are as just defined.

Esterified carboxyl groups represented by the group $R^{13}$ include groups of formula $-CO_2Alk^2$ wherein $Alk^2$ is a straight or branched, optionally substituted $C_{1-8}$alkyl group such as a methyl, ethyl, n-propyl, i-propyl, n-butyl, i-butyl, s-butyl or t-butyl group; a $C_{6-12}$aryl$C_{1-8}$alkyl group such as an optionally substituted benzyl, phenylethyl, phenylpropyl, 1-naphthylmethyl or 2-naphthylmethyl group; a $C_{6-12}$aryl group such as an optionally substituted phenyl, 1-naphthyl or 2-naphthyl group; a $C_{6-12}$aryloxy$C_{1-8}$alkyl group such as an optionally substituted phenyloxymethyl, phenyloxyethyl, 1-naphthyloxymethyl, or 2-naphthyloxymethyl group; an optionally substituted $C_{1-8}$alkanoyloxy$C_{1-8}$alkyl group, such as a pivaloyloxymethyl, propionyloxyethyl or propionyloxypropyl group; or a $C_{6-12}$aroyloxy$C_{1-8}$alkyl group such as an optionally substituted benzoyloxyethyl or benzoyloxypropyl group. Optional substituents present on the $Alk^2$ group include $R^{10}$ substituents described above.

When $Alk^1$ is present in or as a substituent $R^{10}$ it may be for example a methylene, ethylene, n-propylene, i-propylene, n-butylene, i-butylene, s-butylene, t-butylene, ethenylene, 2-propenylene, 2-butenylene, 3-butenylene, ethynylene, 2-propynylene, 2-butynylene or 3-butynylene chain, optionally interrupted by one, two, or three -O- or -S-, atoms or -S(O)-, $-S(O)_2-$ or $-N(R^8)-$ groups.

Particularly useful atoms or groups represented by $R^{10}$ include fluorine, chlorine, bromine or iodine atoms, or $C_{1-6}$alkyl, e.g. methyl or ethyl, $C_{1-6}$alkylamino, e.g. methylamino or ethylamino, $C_{1-6}$ hydroxyalkyl, e.g. hydroxymethyl or hydroxyethyl, $C_{1-6}$alkylthiol e.g. methylthiol or ethylthiol, $C_{1-6}$alkoxy, e.g. methoxy or ethoxy, $C_{5-7}$cycloalkoxy, e.g. cyclo-pentyloxy, halo$C_{1-6}$alkyl, e.g. trifluoromethyl, $C_{1-6}$alkylamino, e.g. methylamino or ethylamino, amino ($-NH_2$), amino$C_{1-6}$alkyl, e.g. aminomethyl or aminoethyl, $C_{1-6}$dialkylamino, e.g. dimethylamino or diethylamino, nitro, cyano, hydroxyl (-OH), formyl [HC(O)-], carboxyl ($-CO_2H$), $-CO_2Alk^2$ [where $Alk^2$ is as defined above], $C_{1-6}$ alkanoyl e.g. acetyl, thiol (-SH), thio$C_{1-6}$alkyl, e.g. thiomethyl or thioethyl, sulphonyl ($-SO_3H$), $C_{1-6}$alkylsulphonyl, e.g. methylsulphonyl, aminosulphonyl ($-SO_2NH_2$), $C_{1-6}$alkylaminosulphonyl, e.g. methylaminosulphonyl or ethylaminosulphonyl, $C_{1-6}$dialkylaminosulphonyl, e.g. dimethylaminosulphonyl or diethylaminosulphonyl, carboxamido ($-CONH_2$), $C_{1-6}$alkylaminocarbonyl, e.g. methylaminocarbonyl or ethylaminocarbonyl, $C_{1-6}$dialkylaminocarbonyl, e.g. dimethylaminocarbonyl or diethylaminocarbonyl, sulphonylamino ($-NHSO_2H$), $C_{1-6}$alkylsulphonylamino, e.g. methylsulphonylamino or ethylsulphonylamino, $C_{1-6}$dialkylsulphonylamino, e.g. dimethylsulphonylamino or diethylsulphonylamino, aminosulphonylamino ($-NHSO_2NH_2$), $C_{1-6}$alkylaminosulphonylamino, e.g. methylaminosulphonylamino or ethylaminosulphonylamino, $C_{1-6}$dialkylaminosulphonylamino, e.g. dimethylaminosulphonylamino or diethylaminosulphonylamino, $C_{1-6}$alkanoylamino, e.g. acetylamino, $C_{1-6}$alkanoylamino $C_{1-6}$alkyl, e.g. acetylaminomethyl or $C_{1-6}$alkoxycarbonylamino, e.g. methoxycarbonylamino, ethoxycarbonylamino or t-butoxycarbonylamino groups.

Where desired, two $R^{10}$ substituents may be linked together to form a cyclic group such as a cyclic ether, e.g. a $C_{2-6}$alkylenedioxy group such as ethylenedioxy.

It will be appreciated that where two or more $R^{10}$ substituents are present, these need not necessarily be the same atoms and/or groups. The $R^{10}$ substituents may be present at any ring carbon atom away from that attached to the rest of the molecule of formula (1). Thus, for example, in phenyl groups represented by Ar any substituent may be present at the 2-, 3-, 4-, 5- or 6- positions relative to the ring carbon atom attached to the remainder of the molecule.

In the compounds of formula (1), when an ester group is present, for example a group $-CO_2Alk^2$ this may advantageously be a metabolically labile ester.

The presence of certain substituents in the compounds of formula (1) may enable salts of the compounds to be formed. Suitable salts include pharmaceutically acceptable salts, for example acid addition salts derived from inorganic or organic acids, and salts derived from inorganic and organic bases.

Acid addition salts include hydrochlorides, hydrobromides, hydroiodides, alkylsulphonates, e.g. methanesulphonates, ethanesulphonates, or isethionates, arylsulphonates, e.g. p-toluenesulphonates, besylates or napsylates, phosphates, sulphates, hydrogen sulphates, acetates, trifluoroacetates, propionates, citrates, maleates, fumarates, malonates, succinates, lactates, oxalates, tartrates and benzoates.

Salts derived from inorganic or organic bases include alkali metal salts such as sodium or potassium salts, alkaline earth metal salts such as magnesium or calcium salts, and organic amine salts such as morpholine, piperidine, dimethylamine or diethylamine salts.

Particularly useful salts of compounds according to the invention include pharmaceutically acceptable salts, especially acid addition pharmaceutically acceptable salts.

In the compounds of formula (1), the group $R^1$ is preferably an optionally substituted ethyl group or, especially, an optionally substituted methyl group. Especially useful substitutents which may be present on $R^1$ groups include one, two or three fluorine or chlorine atoms.

A particularly useful group of compounds of formula (1) has the formula (2):

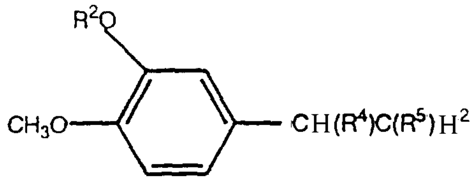

(2)

where $R^2$ is an optionally substituted cycloalkyl group; $R^4$ and $R^5$ are as defined for formula (1); and the salts, solvates, hydrates and N-oxides thereof.

In the compounds of formulae (1) or (2) $R^2$ is preferably an optionally substituted methyl or cyclopentyl group. In particular, $R^2$ is a cyclopentyl group.

The groups $R^4$ and $R^5$ in compounds of formulae (1) or (2) is each, independently, preferably an -Ar group.

Particularly useful $R^4$ or $R^5$ groups in the compounds of formulae (1) or (2) include those $R^4$ or $R^5$ groups in which Ar is a monocyclic aryl group optionally containing one or more heteroatoms selected from oxygen, sulphur, or, in particular, nitrogen atoms, and optionally substituted by one, two, three or more $R^{10}$ substituents. In these compounds, when the group represented by Ar is a heteroaryl group it is preferably a nitrogen-containing monocyclic heteroaryl group, especially a six-membered nitrogen-containing heteroaryl group. Thus, in one preferred example, the groups $R^4$ and $R^5$ may each be a six-membered nitrogen-containing heteroaryl group. In another preferred example $R^4$ may be a monocyclic aryl group or monocyclic heteroaryl group containing an oxygen or sulphur atom and $R^5$ may be a six-membered nitrogen-containing heteroaryl group. In these examples, the six-membered nitrogen-containing heteroaryl group may be an optionally substituted pyridyl, pyridazinyl, pyrimidinyl or pyrazinyl group. Particular examples include optionally substituted 2-pyridyl, 3-pyridyl or, especially, 4-pyridyl, 3-pyridazinyl, 4-pyridazinyl, 5-pyridazinyl, 2-pyrimidinyl, 4-pyrimidinyl, 5-pyrimidinyl, 2-pyrazinyl or 3-pyrazinyl. The monocyclic aryl group may be a phenyl group or a substituted phenyl group, and the monocyclic heteroaryl group containing an oxygen or sulphur atom may be an optionally substituted 2-furyl, 3-furyl, 2-thienyl or 3-thienyl group.

One particularly useful group of compounds of formulae (1) or (2) is that wherein $R^4$ and $R^5$ is each a pyridyl or, especially, a monosubstituted pyridyl, or preferably a disubstituted pyridyl group, or $R^4$ is a phenyl, thienyl or furyl, or substituted phenyl, thienyl or furyl group and $R^5$ is a pyridyl or, especially a monosubstituted pyridyl, or preferably a disubstituted pyridyl group.

In this particular group of compounds and also in general in compounds of formulae (1) or (2), when $R^4$ and/or $R^5$ is a substituted phenyl group it may be for example a mono-, di- or trisubstituted phenyl group in which the substituent is an atom or group $R^{10}$ as defined above. When the $R^4$ and/or $R^5$ group is a monosubstituted phenyl group the substituent may be in the 2-, or preferably 3-, or especially 4-position relative to the ring carbon atom attached to the remainder of the molecule.

When in compounds of formulae (1) or (2) $R^4$ and/or $R^5$ is a substituted pyridyl group it may be for example a mono-or disubstituted pyridyl group, such as a mono- or disubstituted 2-pyridyl, 3-pyridyl or especially 4-pyridyl group substituted by one or two atoms or groups $R^{10}$ as defined above, in particular one or two halogen atoms such as fluorine or chlorine atoms, or methyl, methoxy, hydroxyl or nitro groups. Particularly useful pyridyl groups of these types are 3-monosubstituted-4-pyridyl or 3,5-disubstituted-4-pyridyl, or 2- or 4-monosubstituted-3-pyridyl or 2,4-disubstituted-3-pyridyl groups.

Compounds in which $R^2$ is a cycloalkyl or substituted cycloalkyl group, especially a substituted cyclopentyl or in particular a cyclopentyl group are also particularly useful. In this group of compounds, $R^4$ is preferably a monocyclic aryl group, particularly a phenyl or substituted phenyl group or $R^4$ is a six-membered nitrogen-containing monocyclic heteroaryl group, particularly a pyridyl or substituted pyridyl group and $R^5$ is a six-membered nitrogen-containing monocyclic heteroaryl group, especially a pyridyl or substituted pyridyl group in particular a 4-pyridyl or substituted 4-pyridyl group.

Particularly useful compounds according to the invention are:

(±)-4-[2-(3-Cyclopentyloxy-4-methoxyphenyl)-2-(2-furyl) ethyl]pyridine;
(±)-4-[2-(3-Cyclopentyloxy-4-methoxyphenyl)-2-(2-thienyl)ethyl] pyridine;
(±)-4-[2-(3-Cyclopentyloxy-4-methoxyphenyl)-2-phenylethyl]-3-methylimidazole;

(±)-4-[2-(3-Cyclopentyloxy-4-methoxyphenyl)-2-phenylethyl] pyridine;
(±)-4-[1-(3-Cyclopentyloxy-4-methoxyphenyl)-2-(4-pyridyl)ethyl] pyridine;
(±)-4-[2-(3-Cyclopentyloxy-4-methoxyphenyl)-2-(4-fluorophenylethyl] pyridine;
(±)-4-[2-(3-Cyclopentyloxy-4-methoxyphenyl)-2-(4-trifluoromethylphenyl)ethyl]pyridine;
(±)-4-[2-(3-Cyclopentyloxy-4-methoxyphenyl)-2-(2-methoxyphenylethyl)]pyridine;
(±)-4-[2-(3-Cyclopentyloxy-4-methoxyphenyl)-2-(4-methoxyphenyl)-ethyl]pyridine;
(±)-4-[2-(3-Cyclopentyloxy-4-methoxyphenyl)-2-(4-methylphenyl) ethyl]pyridine;
(±)-4-[2-(3-Cyclopentyloxy-4-methoxyphenyl)-2-(3-methylphenyl)-ethyl]pyridine;
(±)-4-[2-(3-Cyclopentyloxy-4-methoxyphenyl)-2-(3-cyclopentyloxy-4-methoxyphenyl)ethyl]pyridine;
(±)-4-[2-(3-Cyclopentyloxy-4-methoxyphenyl)-2-phenylethyl]-3,5-dichloropyridine;
(±)-2-[2-(3-Cyclopentyloxy-4-methoxyphenyl)-2-phenylethyl] pyridine;
(±)-4-[1-(3-Cyclopentyloxy-4-methoxyphenyl)-2-(4-pyridyl) ethyl] aniline;
(±) -4-[1-(3-Cyclopenxyloxy-4-methoxyphenyl)-2-(4-pyridyl)ethyl] benzoic acid;
(±) Ethyl N-{4-[1-(3-Cyclopentyloxy-4-methoxyphenyl)-2-(4-pyridyl) ethyl]phenyl)carbamate;
(±) N-{4-[1-(3-Cyclopentyloxy-4-methoxyphenyl)-2(4-pyridyl)ethyl] phenyl}N'-ethylurea;
(±) N-{4-[1-(3-Cyclopentyloxy-4-methoxyphenyl)]-2-(4-pyridyl) ethyl}phenylacetamide;
(±)-3-[2-(3-Cyclopentyloxy-4-methoxyphenyl)-2-phenylethyl] pyridine;
(±) -4-[2-(3-Cyclopentyloxy-4-methoxyphenyl)-2-phenylethyl] pyrimidine;
(±) -4-[2-(3-Cyclopentyloxy-4-methoxyphenyl)-2-(4-hydroxymethylphenyl)ethyl]pyridine;
(±) -4-[1 -(3-Cyclopentyloxy-4-methoxyphenyl)-2-(4-pyridyl)ethyl] benzamide;
(±) Ethyl-4-[1-(3-Cyclopentyloxy-4-methoxyphenyl)-2-(4-phenylethyl]benzoate;
(±) N-{4-[1-(3-Cyclopentyloxy-4-methoxyphenyl)-2-(4-pyridyl)ethyl] phenyl}methanesulphonamide; or

the resolved enantiomers thereof; and the salts, solvates, hydrates and N-oxides thereof.

The above specifically mentioned compounds exist in two enantiomeric forms. Each enantiomer is useful, as are mixtures of both enantiomers.

Compounds according to the invention are selective and potent inhibitors of PDE IV. The ability of the compounds to act in this way may be simply determined by the tests described in the Examples hereinafter.

The compounds according to the invention are thus of particular use in the prophylaxis and treatment of human diseases where an unwanted inflammatory response or muscular spasm (for example bladder or alimentary smooth muscle spasm) is present and where the elevation of cAMP levels may be expected to prevent or alleviate the inflammation and relax muscle.

Particular uses to which the compounds of the invention may be put include the prophylaxis and treatment of asthma, especially inflamed lung associated with asthma, cystic fibrosis, or in the treatment of inflammatory airway disease, chronic bronchitis, eosinophilic granuloma, psoriasis and other benign and malignant proliferative skin diseases, endotoxic shock, septic shock, ulcerative colitis, Crohn's disease, reperfusion injury of the myocardium and brain, inflammatory arthritis, chronic glomerulonephritis, atopic dermatitis, urticaria, adult respiratory distress syndrome, diabetes insipidus, allergic rhinitis, allergic conjunctivitis, vernal conjunctivitis, arterial restenosis and artherosclerosis.

Compounds of the invention also suppress neurogenic inflammation through elevation of cAMP in sensory neurones. They are, therefore, analgesic, anti-tussive and anti-hyperalgesic in inflammatory diseases associated with irritation and pain.

Compounds according to the invention may also elevate cAMP in lymphocytes and thereby suppress unwanted lymphocyte activation in immune-based diseases such as rheumatoid arthritis, ankylosing spondylitis, transplant rejection and graft versus host disease.

Compounds according to the invention have also been found to reduce gastric acid secretion and therefore can be used to treat conditions associated with hypersecretion.

Compounds of the invention suppress cytokine synthesis by inflammatory cells in response to immune or infectious stimulation. They are, therefore, useful in the treatment of bacterial, fungal or viral induced sepsis and septic shock in which cytokines such as tumour necrosis factor (TNF) are key mediators. Also compounds of the invention suppress inflammation and pyrexia due to cytokines and are, therefore, useful in the treatment of inflammation and cytokine-mediated chronic tissue degeneration which occurs in diseases such as rheumatoid or osteo-arthritis.

Over-production of cytokines such as TNF in bacterial, fungal or viral infections or in diseases such as cancer, leads to cachexia and muscle wasting. Compounds of the invention ameliorate these symptoms with a consequent enhancement of quality of life.

Compounds of the invention also elevate cAMP in certain areas of the brain and thereby counteract depression and memory impairment.

Compounds of the invention suppress cell proliferation in certain tumour cells and can be used, therefore, to

prevent tumour growth and invasion of normal tissues.

For the prophylaxis or treatment of disease the compounds according to the invention may be administered as pharmaceutical compositions, and according to a further aspect of the invention we provide a pharmaceutical composition which comprises a compound of formula (1) together with one or more pharmaceutically acceptable carriers, excipients or diluents.

Pharmaceutical compositions according to the invention may take a form suitable for oral, buccal, parenteral, nasal, topical or rectal administration, or a form suitable for administration by inhalation or insufflation.

For oral administration, the pharmaceutical compositions may take the form of, for example, tablets, lozenges or capsules prepared by conventional means with pharmaceutically acceptable excipients such as binding agents (e.g. pregelatinised maize starch, polyvinylpyrrolidone or hydroxypropyl methylcellulose); fillers (e.g. lactose, microcrystalline cellulose or calcium hydrogen phosphate); lubricants (e.g. magnesium stearate, talc or silica); disintegrants (e.g. potato starch or sodium glycollate); or wetting agents (e.g. sodium lauryl sulphate). The tablets may be coated by methods well known in the art. Liquid preparations for oral administration may take the form of, for example, solutions, syrups or suspensions, or they may be presented as a dry product for constitution with water or other suitable vehicle before use. Such liquid preparations may be prepared by conventional means with pharmaceutically acceptable additives such as suspending agents, emulsifying agents, non-aqueous vehicles and preservatives. The preparations may also contain buffer salts, flavouring, colouring and sweetening agents as appropriate.

Preparations for oral administration may be suitably formulated to give controlled release of the active compound.

For buccal administration the compositions may take the form of tablets or lozenges formulated in conventional manner.

The compounds for formula (1) may be formulated for parenteral administration by injection e.g. by bolus injection or infusion. Formulations for injection may be presented in unit dosage form, e.g. in glass ampoule or multi dose containers, e.g. glass vials. The compositions for injection may take such forms as suspensions, solutions or emulsions in oily or aqueous vehicles, and may contain formulatory agents such as suspending, stabilising, preserving and/or dispersing agents. Alternatively, the active ingredient may be in powder form for constitution with a suitable vehicle, e.g. sterile pyrogen-free water, before use.

In addition to the formulations described above, the compounds of formula (1) may also be formulated as a depot preparation. Such long acting formulations may be administered by implantation or by intramuscular injection.

For nasal administration or administration by inhalation, the compounds for use according to the present invention are conveniently delivered in the form of an aerosol spray presentation for pressurised packs or a nebuliser, with the use of suitable propellant, e.g. dichlorodifluoromethane, trichlorofluoromethane, dichlorotetrafluoroethane, carbon dioxide or other suitable gas or mixture of gases.

The compositions may, if desired, be presented in a pack or dispenser device which may contain one or more unit dosage forms containing the active ingredient. The pack or dispensing device may be accompanied by instructions for administration.

The quantity of a compound of the invention required for the prophylaxis or treatment of a particular inflammatory condition will vary depending on the compound chosen, and the condition of the patient to be treated. In general, however, daily dosages may range from around 100ng/kg to 100mg/kg, e.g. around 0.01mg/kg to 40mg/kg body weight for oral or buccal administration, from around 10ng/kg to 50mg/kg body weight for parenteral administration and around 0.05mg to around 1000mg e.g. around 0.5mg to around 1000mg for nasal administration or administration by inhalation or insufflation.

The compounds according to the invention may be prepared by the following processes. The symbols Y, $R^2$, $R^4$ and $R^5$, when used in the formulae below are to be understood to represent those groups described above in relation to formula (1) unless otherwise indicated. In the reactions described below it may be necessary to protect reactive functional groups, for example hydroxy, amino, thio, or carboxy groups, where these are desired in the final product, to avoid their unwanted participation in the reactions. Conventional protecting groups may be used in accordance with standard practice [see, for example, Green, T. W. in "Protective Groups in Organic Synthesis" John Wiley and Sons, 1981.] It may be that deprotection will form the last step in the synthesis of compounds of formula (1). Thus, in one example, compounds of formula (1) wherein $R^4$ and/or $R^5$ contains a carboxylic acid group may be prepared by deprotecting the corresponding compound wherein $R^4$ and/or $R^5$ contains a protected carboxyl group, such as an oxazolinyl group, e.g. 4,4-dimethyl-2-oxazolinyl, in the presence of a base, e.g. sodium hydroxide, in an acid solvent e.g. aqueous hydrochloric acid, at an elevated temperature, e.g. the reflux temperature.

Thus according to a further aspect of the invention, a compound of formula (1) may be prepared by hydrogenation of a compound of formula (3):

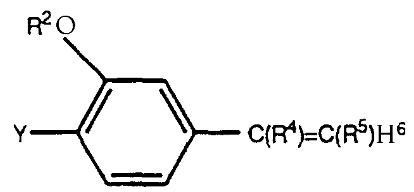

(3)

The hydrogenation may be performed using for example hydrogen in the presence of a catalyst. Suitable catalysts include metals such as platinium or palladium, optionally supported on an inert carrier such as carbon or calcium carbonate; nickel e.g. Raney nickel, or rhodium. The reaction may be performed in a suitable solvent, for example an alcohol such as methanol or ethanol, an ether such as tetrahydrofuran or dioxane or an ester such as ethyl acetate, optionally in the presence of a base, for example a tertiary organic base such as triethylamine, at for example ambient temperature.

Alternatively, the reaction may be accomplished by transfer hydrogenation using an organic hydrogen donor and a transfer agent. Suitable hydrogen donors include for example acids, such as formic acid, formates, e.g. ammonium formate, alcohols, such as benzyl alcohol or ethylene glycol, hydrazine, and cycloalkenes such as cyclohexene or cyclohexadiene. The transfer agent may be for example a transition metal, for example palladium or platinum, optionally supported on an inert carrier as discussed above, nickel e.g. Raney nickel, ruthenium, e.g. tris(triphenylphosphine)ruthenium chloride or copper. The reaction may generally be performed at an ambient or elevated temperature, optionally in the presence of a solvent, for example an alcohol such as ethanol or an acid such as acetic acid.

Intermediates of formula (3) may be prepared using a Horner-Wadsworth-Emmons approach by reaction of a ketone of formula (6) [described hereinafter] with a phosphonate $R^5CH_2PO(OAlk)_2$, where Alk is a $C_{1-4}$alkyl group such as a methyl group, in the presence of a base such as sodium hydride. The phosphonates for use in this reaction may be prepared by conventional methods, for example by reaction of a compound $R^5CH_2L$, where L is a leaving group such as a chlorine atom with a phosphine $P(OAlk)_3$.

In another process for the preparation of intermediates of formula (3), an alkene of formula (4):

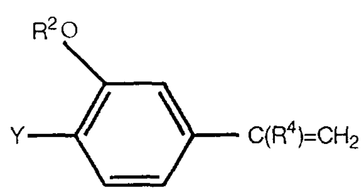

(4)

may be coupled in a Heck reaction with an organopalladium compound derived from a compound $R^5$Hal [where Hal is a halogen atom such as a bromine atom] and a palladium salt such as palladium acetate in the presence of a phosphine such as tri-o-tolyl phosphine and a base such as triethylamine at an elevated temperature and pressure.

Intermediate alkenes of formula (4) may be obtained by reaction of a corresponding ketone of formula (6) (described hereinafter) using a Wittig reaction employing a phosphonium salt such as methyltriphenylphosphonium bromide in the presence of a base such as n-butyllithium and an inert solvent such as tetrahydrofuran at, for example, 0°C to ambient temperature.

Intermediates of formula (3) may also be prepared by dehydration of an alcohol of formula (5):

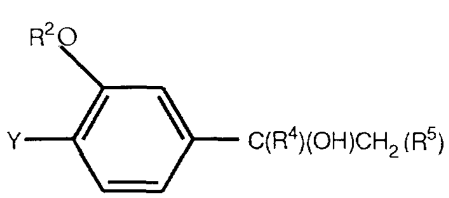

(5)

using an acid or base - catalysed elimination.

Suitable acids include for example phosphoric or sulphonic acids, e.g. 4-toluenesulphonic acid. The reaction may be performed in an inert organic solvent, for example a hydrocarbon such as toluene, at an elevated temperature, for example the reflux temperature. Base-catalysed elmination may be performed using for example trifluoroacetic anhydride in the presence of an organic base such as triethylamine at a low temperature e.g. from around 0°C to ambient temperature, in a solvent such as dichloromethane or tetrahydrofuran.

In certain instances, the reaction conditions used may also cleave the group $R^2$ in the starting material of formula (4) to yield an intermediate of formula (3) where $R^2$ is a hydrogen atom. Such compounds may be converted to the required compound of formula (3) by reaction with a halide $R^2Hal$ (where Hal is a halogen atom such as a bromine or chlorine atom) as described hereinafter for the preparation of compounds of formula (1) from the corresponding compounds where $R^2$ is a hydrogen atom.

The alcohols of formula (5) may be prepared by reaction of a ketone of formula (6):

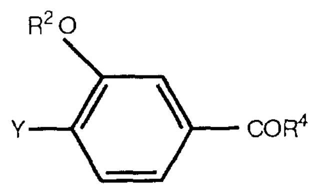

(6)

with an organometallic reagent $R^5CH_2Z$, where Z is a metal atom.

Metal atoms represented by Z include, for example, a lithium atom.

The reaction may be performed in a solvent such as an ether, e.g. a cyclic ether such as tetrahydrofuran, at a low temperature e.g. around -70°C to ambient temperature. This reaction is particularly suitable for the preparation of compounds of formula (1) wherein $R^5$ is an electron deficient group such as a 2- or 4-pyridyl group.

Reagents $R^5CH_2Z$ are either known compounds or may be prepared, preferably in situ during the above process, by reaction of a compound $AlkCH_2Z$ [where Alk is an alkyl group such as a n-propyl group] with a compound $R^5CH_3$ where necessary in the presence of a base such as an amine e.g. diisopropylamine using the above-mentioned conditions.

Ketones of formula (6) may be prepared by oxidation of a corresponding alcohol of formula (7):

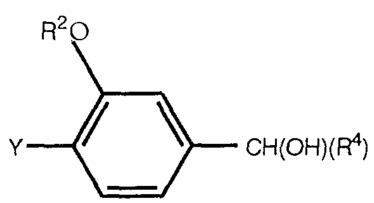

(7)

using an oxidising agent such as manganese dioxide in a solvent such as dichloromethane at ambient temperature.

Alternatively, ketones of formula (6) may be prepared by reaction of a halide of formula (8):

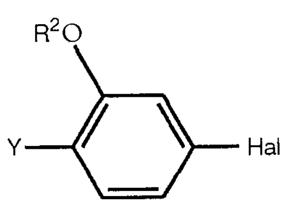

(8)

[where Hal is a halogen atom such as a bromine or chlorine atom] by halogen-metal exchange with a base such as n-butyllithium followed by reaction with a nitrile $R^4CN$, an acid chloride $R^4COCl$ or an ester $R^4CO_2Alk$ (where Alk is an alkyl group, e.g. a methyl group), in a solvent such as tetrahydrofuran at a low temperature, e.g. around -70°C, and

subsequent treatment with an acid such as hydrochloric acid at e.g. -20°C to ambient temperature.

Alcohols of formula (7) may be prepared by reaction of an aldehyde of formula (9):

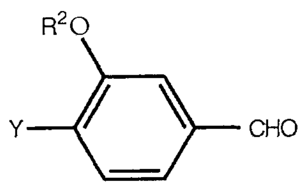

(9)

with an organometallic compound, such as an organolithium compound $R^4Li$, or a Grignard reagent $R^4MgBr$, in a solvent, such as tetrahydrofuran, at a low temperature, e.g. around -55°C to 0°C.

Aldehydes of formula (9) may be prepared by alkylation of a corresponding compound of formula (10):

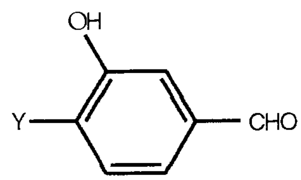

(10)

using a compound $R^2Hal$ [where Hal is as previously defined] using the reagents and conditions described hereinafter for the alkylation of intermediates of formula (18).

Intermediates of formula (10) are either known compounds or may be prepared from known starting materials by methods analogous to those used for the preparation of the known compounds.

Halides of formula (8) may be prepared by alkylation of a compound of formula (11):

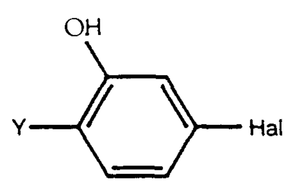

(11)

using the reagents and conditions discussed above in relation to the alkylation of aldehydes of formula (10).

Halides of formula (11) may be prepared by oxidation of an aldehyde of formula (12):

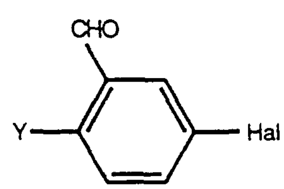

(12)

using an oxidising agent such as 3-chloroperoxybenzoic acid in a halogenated hydrocarbon such as chloroform at a temperature from around 0°C to room temperature.

In yet another process according to the invention, compounds of formula (1) may be prepared by decarboxylation of an acid of formula (13):

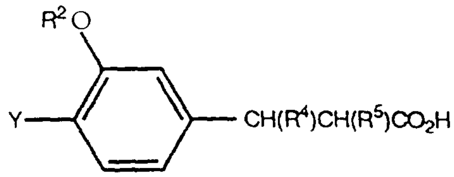

(13)

The reaction may be carried out by treatment of the compound of formula (13) with a base, for example an inorganic base such as a hydroxide, e.g. sodium hydroxide in a solvent such as an alcohol, e.g. ethanol, at an elevated temperature e.g. the reflux temperature, followed by acidification of the reaction mixture to a pH of around pH4 to around pH6 using an acid such as an inorganic acid, e.g. hydrochloric acid, at an elevated temperature, e.g. the reflux temperature.

If desired, the acid of formula (13) may be generated *in situ* from the corresponding ester or nitrile using the above reaction conditions, or by initial treatment with an acid.

Intermediates of formula (13) may be prepared by reacting a compound of formula (14)

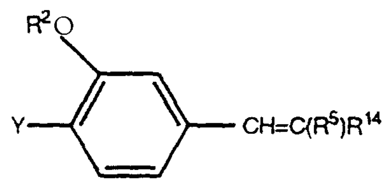

(14)

[where $R^{14}$ is an ester of an acid $-CO_2H$ (e.g. an alkyl ester such as an ethyl ester) or a group -CN], with a Grignard reagent $R^4MgBr$, in the presence of a complexing agent, e.g. a copper (I) bromide-dimethyl sulphide complex, or a copper (I) chloride, or with an organolithium compound, e.g. $R^4Li$, in a solvent, e.g. tetrahydrofuran, at low temperature, e.g. around -40°C, followed by treatment with a base or an acid to yield the acid of formula (13) where $R^{14}$ is $-CO_2H$. The Grignard and the lithium reagents are either known compounds or may be prepared in a manner similar to that used to synthesise the known compounds.

Compounds of formula (14) may be obtained by reacting an adehyde of formula (9) with an ester or nitrile $R^5CH_2R^{14}$ in an acid solvent, such as acetic acid, at an elevated temperature, for example the reflux temperature, in the presence of a base, such as ammonium acetate.

In a further process according to the invention a compound of formula (1) wherein $R^5$ is a heteroaryl group may be generally prepared by cyclisation of a compound of formula (15):

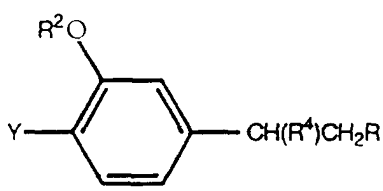

(15)

where R is a carboxylic acid $[-CO_2H]$ group or a reactive derivative thereof; or a nitrile [-CN] or an imine salt with a bifunctional reagent $W^1R^{5a}W^2$ and, where necessary, a compound $R^{5b}W^3$ [where $W^1$, $W^2$ and $W^3$, which may be the same or different, is each a reactive functional group or a protected derivative thereof; and $R^{5a}$ and $R^{5b}$ are components of the heteroaryl group $R^5$ such that when added together with $W^1$, $W^2$ and $W^3$ to the group R in compounds of formula (15) the resulting group $-RW^1 R^{5a}W^2$ or $-RW^1R^{5a}W^2R^{5b}W^3$ constitutes the heteroaryl group $R^5$].

Reactive derivatives of carboxylic acids for use in this reaction include acid halides, (e.g. acid chlorides), amides, including thioamides, or esters, including thioesters. Imine salts include for example salts of formula [e.g.-C (OAlk)=NH2+A-, where Alk is a $C_{1-4}$alkyl group and $A^-$ is a counterion e.g. a chloride ion].

In this general reaction the reactive functional groups represented by $W^1$, $W^2$ or $W^3$ may be any suitable

carbon, nitrogen, sulphur or oxygen nucleophiles. Particular examples include simple nucleophiles such as carbanions [e.g. generated by the coupling of an alkyl group with an organometallic compound], amino, thiol and hydroxyl groups.

In general, the cyclisation reaction will initially be performed in a solvent, for example an inert solvent such as a halocarbon, e.g. dichloromethane, an ether, e.g. a cyclic ether such as tetrahydrofuran, or a hydrocarbon, e.g. an aromatic hydrocarbon such as toluene, from a low temperature, e.g. around -70°C, to around the reflux temperature, where necessary in the presence of a base or a thiation reagent, e.g. Lawesson's reagent, followed if necessary by heating, to an elevated temperature, e.g. the reflux temperature.

Thus, in one particular example, compounds of formula (1) wherein $R^5$ is a benzothiazolyl, benzoxazolyl or benzimidazolyl group may be prepared by reaction of a compound of formula (15) where R is an acid halide, e.g. acid chloride, with a reagent $W^1R^{5a}W^2$ which is 2-aminothiophenol, 2-hydroxyphenol, or 1,2-diaminobenzene respectively in the presence of a base e.g. an organic amine such as pyridine, in a solvent e.g. a halocarbon such as dichloromethane, from around -70°C to the reflux temperature.

In another example of the general cyclisation process, a compound of formula (15) where R is an acid halide as described above may be reacted with a compound $W^1R^{5a}W^2$ which is a monoalkylmalonate, e.g. ethyl hydrogen malonate, followed by reaction with a compound $R^{5b}W^3$ which is hydrazine to give a compound of formula (1) wherein $R^5$ is a 5-hydroxypyrazolyl group.

In another variation of the cyclisation process, the halide of formula (15) may be reacted with a compound $W^1R^{5a}W^2$ which is $BrMg(CH_2)_3[-O(CH_2)_2O-]$ followed by reaction in an acid solution with a compound $R^{5b}W^3$ which is methylamine to yield a compound of formula (1) wherein $R^5$ is a N-methyl pyrrole group.

In a further example of the cyclisation process, the halide of formula (15) may be reacted with a compound $W^1R^{5a}W^2$ which is $H_2NNHCSNH_2$ in an aromatic hydrocarbon such as toluene, at an elevated temperature, e.g. around 150°C, followed by treatment with a base, e.g. an inorganic base such as sodium bicarbonate to give a compound of formula (1) wherein $R^5$ is a 1,2,4-triazolyl-5-thiolate group.

Intermediate compounds of formula (15) are particularly useful and form a further aspect of the invention. Active derivatives of the acids of formula (15) and other compounds of formula (15) where R is a nitrile or an imine salt may be prepared from the corresponding acids [where R is $-CO_2H$] using conventional procedures for converting carboxylic acids to such compounds, for example as described in the Examples hereinafter.

Acids of formula (15) [where R is $-CO_2H$] may be prepared by hydrolysing a diester of formula (16)

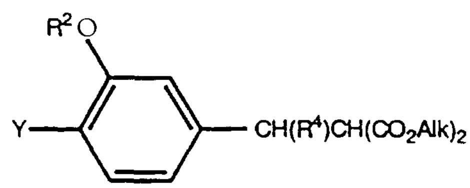

(16)

where Alk is a $C_{1-4}$alkyl group, e.g. an ethyl group, with a base, e.g. sodium hydroxide, in a solvent, e.g. dioxane, at an elevated temperature, e.g. the reflux temperature, followed by acidification at an elevated temperature.

Diesters of formula (16) may be prepared by reacting a diester of formula (17)

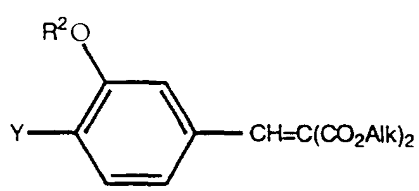

(17)

with an organometallic reagent, such as a Grignard reagent using the conditions described above for the preparation of alcohols of formula (1).

In another process according to the invention, a compound of formula (1) may be prepared by alkylation of a compound of formula (18):

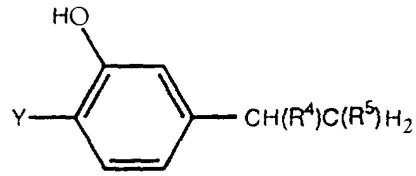

(18)

using a reagent $R^2L$, where L is a leaving group.

Leaving groups represented by L include halogen atoms such as iodine or chlorine or bromine atoms or sulphonyloxy groups such as arylsulphonyloxy groups, e.g. p-toluenesulphonyloxy.

The alkylation reaction may be carried out in the presence of a base, e.g. an inorganic base such as a carbonate, e.g. caesium or potassium carbonate, an alkoxide, e.g. potassium-t-butoxide, or a hydride, e.g. sodium hydride, in a dipolar aprotic solvent such as an amide, e.g. a substituted amide such as dimethylformamide or an ether, e.g. a cyclic ether such as tetrahydrofuran, at ambient temperature or above e.g. around 40°C to 50°C.

Intermediates of formula (18) may be obtained from the corresponding protected compound of formula (19):

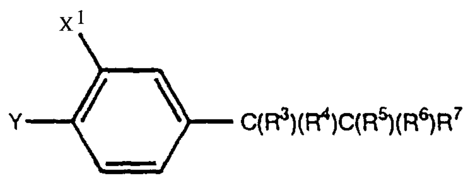

(19)

wherein $X^1$ is a protected hydroxy, group using conventional procedures [see Green, T. W. *ibid*]. Thus, for example, where $X^1$ is a t-butyldimethylsilyloxy group, the required hydroxyl group may be obtained by treatment of the protected intermediate with tetrabutylammonium fluoride. The protected intermediate of formula (18) may be prepared in an analogous manner to the compounds of formula (1) using the reactions described herein and appropriately protected intermediates.

Compounds of formula (17) may be prepared by condensing an aldehyde of formula (9) with a malonate, e.g. diethylmalonate, if necessary in the presence of catalysts, e.g. piperidine and acetic acid, in an inert solvent, e.g. toluene, at elevated temperature, e.g. the reflux temperature.

Compounds of formula (1) may also be prepared by interconversion of other compounds of formula (1). Thus, for example, a group represented by $R^4$ or $R^5$ in compounds of formula (1) may be substituted in the aryl or heteroaryl portions by any of the groups $R^{10}$ by an appropriate substitution reaction using the corresponding unsubstituted compound of formula (1) and a $R^{10}$ containing nucleophile or electrophile.

In another example of an interconversion process a compound of formula (1) wherein the aryl or heteroaryl group in $R^4$ and/or $R^5$ contains a $-CH_2NH_2$ substituent may be prepared by reduction of a corresponding compound wherein $R^4$ and/or $R^5$ contains a nitrile group, using for example a complex metal hydride such as lithium aluminium hydride in a solvent such as an ether e.g. diethylether.

In a further example, a compound of formula (1) wherein the aryl or heteroaryl group in $R^4$ and/or $R^5$ contains an alkanoylamino or alkanoylaminoalkyl substituent may be prepared by acylation of a corresponding compound wherein $R^4$ and/or $R^5$ contains a $-NH_2$ or alkylamino group by reaction with an acyl halide in the presence of a base, such as a tertiary amine e.g. triethylamine in a solvent such as dichloromethane.

In yet another example of an interconversion process, compounds of formula (1) wherein $R^4$ and/or $R^5$ is substituted by an ester [$CO_2Alk^2$], e.g. an ethanoate, may be prepared by esterification of a corresponding compound wherein $R^4$ and/or $R^5$ contains a carboxylic acid, using an acid halide, such as an acid chloride, e.g. acetyl chloride, in an alcohol, such as ethanol, at an elevated temperature, such as the reflux temperature.

Compounds of formula (1) wherein $R^4$ and/or $R^5$ is substituted by a carboxylic acid may be prepared from the corresponding compound wherein $R^4$ and/or $R^5$ contains a formyl group, by oxidation with an oxidising agent, e.g. potassium permanganate, in a solvent, such as an alcohol, e.g. tert-butanol, at ambient temperature.

In a further interconversion reaction, compounds of formula (1) wherein $R^4$ and/or $R^5$ is substituted by an aminoalkyl group, such as dimethylaminomethyl, may be prepared by reductive amination of a corresponding compound wherein $R^4$ and/or $R^5$ contains a formyl group, using an amine, e.g. dimethylamine, in the presence of a reducing agent, e.g. sodium cyanoborohydride, if necessary in the presence of a catalyst, e.g. ethanolic HCl, in a solvent, such

as an alcohol, e.g. methanol, at ambient temperature.

In another example of an interconversion reaction a compound of formula (1) wherein $R^4$ and/or $R^5$ is substituted by a formyl group, may be reduced to the corresponding alcohol, e.g. where $R^4$ and/or $R^5$ contains a hydroxymethyl group, using a reducing agent, e.g. sodium borohydride, in a solvent, such as an alcohol, e.g. ethanol, at a temperature from around 20°C to ambient temperature. The resulting alcohol may then be converted to the corresponding alkoxy derivative, e.g. methoxymethyl, by reaction with an alkyl halide or alkyl sulphonate using the methods and reagents described above for the alkylation of intermediates of formula (18).

In a further example of an interconversion process compounds of formula (1) wherein $R^4$ and/or $R^5$ contains a carboxamido (-CONHR$^{11}$) or an aminocarbonyl (-NHCOR$^{11}$) group may be prepared by reaction of the corresponding compound wherein $R^4$ and/or $R^5$ contains a -$CO_2H$ or a -$NH_2$ group respectively by reaction with a carbamate, such as isobutyl chloroformate or ethyl chloroformate, in the presence of a base, such as an amine, e.g. triethylamine or N-methylmorpholine, in a solvent, such as dichloromethane, or a mixture of solvents, e.g. tetrahydrofuran and dimethylformamide, at a temperature from around -20°C to room temperature.

In a still further interconversion reaction, compounds of formula (1) wherein $R^4$ and/or $R^5$ is substituted by a -NHCONHR$^{11}$ group may be prepared by reacting a corresponding compound wherein $R^4$ and/or $R^5$ is substituted by an amino (-$NH_2$) group, with an isocyanate, e.g. ethyl isocyanate, in a solvent, e.g. dichloromethane, at ambient temperature.

N-oxides of compounds of formula (1) may be prepared for example by oxidation of the corresponding nitrogen base using an oxidising agent such as hydrogen peroxide in the presence of an acid such as acetic acid, at an elevated temperature, for example around 70°C to 80°C, or alternatively by reaction with a peracid such as peracetic acid in a solvent, e.g. dichloromethane, at ambient temperature.

Salts of compounds of formula (1) may be prepared by reaction of a compound of formula (1) with an appropriate acid or base in a suitable solvent or mixture of solvents e.g. an organic solvent such as an ether e.g. diethylether, or an alcohol, e.g. ethanol using conventional procedures.

Where it is desired to obtain a particular enantiomer of a compound of formula (1) this may be produced from a corresponding mixture of enantiomers using any suitable conventional procedure for resolving enantiomers.

Thus for example diastereomeric derivatives, e.g. salts, may be produced by reaction of a mixture of enantiomers of formula (1) e.g. a racemate, and an appropriate chiral compound, e.g. a chiral acid or base. Suitable chiral acids include, for example, tartaric acid and other tartrates such as dibenzoyl tartrates and ditoluoyl tartrates, sulphonates such as camphor sulphonates, mandelic acid and other mandelates and phosphates such as 1,1'-binaphthalene-2,2'-diyl hydrogen phosphate. The diastereomers may then be separated by any convenient means, for example by crystallisation and the desired enantiomer recovered, e.g. by treatment with an acid or base in the instance where the diastereomer is a salt.

In another resolution process a racemate of formula (1) may be separated using chiral High Performance Liquid Chromatography, for example as described in the Examples hereinafter.

Alternatively, if desired a particular enantiomer may be obtained by using an appropriate chiral intermediate in one of the processes described above. Thus, for example, the procedure and chiral intermediates described herein for the preparation of (+)-2-[2-(3-cyclopentyloxy-4-methoxyphenyl)-2-phenylethyl]benz[d]oxazole (see Example 35) may be readily adapted to provide particular enantiomers of the invention.

The following examples illustrate the invention. The following abbreviations are used: DMF - dimethylformamide; THF - tetrahydrofuran; DME - dimethoxyethane; EtOAc - ethyl acetate; $Et_2O$ - diethylether; Et3N - triethylamine; BuLi - butyllithium; LDA - lithium diisopropylamide; EtOH - ethanol; RT - room temperature.

All $^1$Hnmr spectra were obtained at 300MHz unless specified otherwise.

## INTERMEDIATE 1

### 3-Cyclopentyloxy-4-methoxybenzaldehyde

$Cs_2CO_3$ (214g, 0.66mol) was added to a mixture of 3-hydroxy-4-methoxybenzaldehyde (100g, 0.66mol) and cyclopentyl bromide (98g, 0.66mol) in anhydrous DMF (500ml). The reaction mixture was stirred at RT for 16h then treated with a further portion of cyclopentyl bromide (98g, 0.66mol) and $Cs_2CO_3$ (214g, 0.66mol). After a further 6h at RT, the mixture was filtered and concentrated *in vacuo*. The residue was dissolved in $CH_2Cl_2$ (300ml) and washed with NaOH solution (10%; 2x150ml). The organic layer was dried ($MgSO_4$), concentrated *in vacuo*, and distilled (150°C, $10^{-2}$mbar) to afford the title compound (130g) as a viscous colourless oil. $\delta_H$ ($CDCl_3$) 1.5-2.0 (8H, br m, C$H_2$)$_4$), 3.87 (3H, s, OMe), 4.80 (1H, br m, OCHCH$_2$), 6.90 (1H, d, J 8.7Hz, ArH ortho to OMe), 7.30-7.45 (2H, m, 2xArH meta to OMe), and 9.77 (1H, s, ArCHO).

**INTERMEDIATE 2**

a) **(3-Cyclopentyloxy-4-methoxyphenyl)phenylketone**

Phenyllithium (1.5M in ether-cyclohexane, 33.5ml, 50mmol) was added dropwise to a solution of Intermediate 1 (10.0g, 45.4mmol) in THF (50ml) at about -55°C. The reaction mixture was allowed to warm to RT overnight then diluted with water (100ml) and extracted with $Et_2O$ (3x50ml). The organic extract was washed with aqueous HCl (1%, 70ml), brine (100ml), then dried ($MgSO_4$), and concentrated *in vacuo* to afford 1-(3-cyclopentyloxy-4-methoxyphenyl)-1-phenylmethanol (13.4g) as a white solid. m.p. 82.5-83°C; $\delta_H$ ($CDCl_3$) 1.5-2.0 (8H, br, m, $(CH_2)_4$), 2.30 (1H, br, s, OH), 3.77 (3H, s, OMe), 4.68 (1H, br, m, OCHCH$_2$), 5.77 (1H, s, CHOH), 6.75-6.85 (3H, m, ArH ortho to OMe + 2xArH meta to OMe), and 7.15-7.4 (5H, m, $C_6H_5$); m/z 298 ($M^+$ 20%), 230 (50), 151 (30), 125 (100), 124 (33), 105 (38), and 92 (22).

The alcohol (prepared above) (13.4g, 44.8mmol) was dissolved in $CH_2Cl_2$ (150ml) and treated with $MnO_2$ (22g). The reaction mixture was vigorously stirred at RT for 18h then treated with a further portion of $MnO_2$ (20g). More $MnO_2$ (20g) was added after 10h and the mixture stirred for 18h then filtered through Celite® and concentrated *in vacuo.* The residue was recrystallised from EtOH to afford the title compound (11.27g; two crops) as a white crystalline solid m.p. 59-75°C; $\delta_H$ ($CDCl_3$) 1.5-2.1 (8H, br, m, $(CH_2)_4$), 3.88 (3H, s, OMe), 4.80 (1H, br m, OCHCH$_2$), 6.83 (1H, d, J 8.5 Hz, ArH ortho to OMe), and 7.25-7.8 (7H, m, 2xArH meta to OMe + $C_6H_5$); m/z 296 ($M^+$ 11%), 229 (17), 228 (95), 152 (12), 151 (100), 105 (30), 77 (21), and 41 (10).

b) **(3-Cyclopentyloxy-4-methoxyphenyl)(2-methoxyphenyl)ketone**

From Intermediate 4 (1.35g, 5.0mmol) and 2-methoxybenzaldehyde (0.68g, 5.0mmol). Chromatography ($SiO_2$; EtOAc/hexane, 1:1) afforded the title compound (1.43g) as a white solid (Found: C, 73.53, H, 6.86. $C_{20}H_{22}O_4$ requires C, 73.60; H, 6.79%); m/z (EI) 326 ($M^+$, 28%), 258 (65), 241 (82), 151 (67), 138 (32), 135 (100), and 121 (45).

**INTERMEDIATE 3**

**5-Bromo-2-methoxyphenol**

A solution of 5-bromo-2-methoxybenzaldehyde (100g, 0.46mol) in $CHCl_3$ (250ml) was cooled with an ice bath and 3-chloroperoxybenzoic acid (50-60% purity) (146g, 0.51 mol) in $CHCl_3$ (1000ml) added. The reaction mixture was allowed to warm slowly to room temperature and stirred for 72h. The white solid was filtered off and the filtrate concentrated *in vacuo.* The residue was dissolved in $Et_2O$ (200ml) and washed with 1M sodium sulphite solution (2x200ml) then $NaHCO_3$ [half saturated] (3x200ml). The ether layer was washed with 10% aqueous NaOH (3x100ml) and the combined basic extract was acidified with concentrated hydrochloric acid and extracted with $Et_2O$ (3x100ml). The combined organic extract was dried ($MgSO_4$) and florisil (10g) filtered and the solvent removed under reduced pressure to give the title compound (90g) as a pale brown solid.

**INTERMEDIATE 4**

**4-Bromo-2-cyclopentyloxyanisole**

Intermediate 3 (90g)was dissolved in DMF (300ml), and treated with $Cs_2CO_3$ (158g, 490mmol), and cyclopentyl bromide (73g, 52.5ml, 490mmol). After stirring overnight, further $Cs_2CO_3$ (35g, 107mmol) and cyclopentylbromide (12ml, 16.7g, 112mmol) were added and stirring continued for 2h. Further portions of cyclopentylbromide (10ml) and $Cs_2CO_3$ were then added (14g). After stirring for 1h, the DMF was evaporated *in vacuo* and the residue diluted with water (200 ml) and extracted with $Et_2O$ (3x100ml). The combined organic extract was washed with NaOH solution (5%, 2x100ml), water (100ml), then dried ($MgSO_4$) and the solvent evaporated *in vacuo* to give a red oil which was distilled (140°C, 0.3mbar) to afford the title compound (101g) as a colourless oil (Found: C, 53.11; H, 5.53. $C_{12}H_{15}BrO_2$ requires C, 53.15; H, 5.58%).

**INTERMEDIATE 5**

a) **(3-Cyclopentyloxy-4-methoxyphenyl)(4-pyridyl)ketone**

n-BuLi (1.45M in hexanes; 19.6ml, 28.4mmol) was added dropwise at -70°C to a solution of Intermediate 4 (7.0g, 25.8mmol) in THF (50ml). After stirring for 0.25h, a solution of 4-cyanopyridine (3.08g, 29.7mmol) in THF (15ml)

was added and maintained at -70°C for 0.75h. The reaction mixture was then allowed to warm to -10°C and quenched with aqueous HCl (10%; 60ml). The mixture was stirred for 0.5h, basified with aqueous NaOH (10%, 70ml), and extracted with $Et_2O$ (3x70ml). The extract was washed with brine (100ml), dried ($MgSO_4$), and concentrated *in vacuo.* The residue was subjected to chromatography ($SiO_2$; EtOAc/hexane, 4:1) to afford the title compound (6.34g) as a white powder. $\delta_H$ ($CDCl_3$) 1.5-1.9 (8H, br m, ($CH_2)_4$), 3.90 (3H, s, OMe), 4.82 (1H, br m, $OCHCH_2$), 6.84 (1 H, d, J 8.4 Hz, ArH ortho to OMe) 7.29 (1 H, dd, J 8.4, 2.0 Hz, ArH para to cyclopentyloxy), 7.4-7.55 (3H, m, ArH ortho to cyclopentyloxy + pyridine $H_3$, $H_5$), and 8.73 (2H, dd, J 4.4 Hz, 1.5 Hz, pyridine $H_2$, $H_6$).

b) **(3-Cyclopentyloxy-4-methoxyphenyl)(4-methylphenyl)ketone**

From Intermediate 4 (2.71g, 10mmol) and 4-methylbenzonitrile (1.17g, 10mmol). Chromatography ($SiO_2$; $Et_2O$/hexane, 1:1) afforded the title compound (1.80g) as a white solid; $\delta_H$ (80MHz; $CDCl_3$) 1.5-2.1 (8H, br m, ($CH_2)_4$), 2.44 (3H, s, ArMe), 3.92 (3H, s, OMe), 4.83 (1H, br m, OCH), 6.87 (1H, d, J 8.3 Hz, ArH ortho to OMe), 7.27 (2H, d, J 8.0 Hz, ArH ortho to OMe), 7.36 (1H, dd, J 8.3, 2.0Hz, ArH para to cyclopentyloxy), 7.43 (1H, d, J 2.0Hz, ArH ortho cyclopentyloxy), and 7.68 (2H, ca d, J 8.0Hz, ArH meta to Me).

c) **(3-Cyclopentyloxy-4-methoxyphenyl)(4-methoxyphenyl)ketone**

From Intermediate 35 (2.17g, 10.0mmol) and 4-bromoanisole (1.82g, 10.0mmol). Chromatography ($SiO_2$; EtOAc/hexane, 1:1) afforded the title compound (2.88g) as a white solid; $\delta_H$ (80MHz; $CDCl_3$) 1.45-2.05 (8H, br m, ($CH_2)_4$), 3.90 (3H, s, OMe), 3.94 (3H, s, OMe). 4.83 (1H, br m, OCH), 6.90 (1H, d, J 8.5Hz, ArH meta to cyclopentyloxy), 6.94 (2H, ca. d, J ca. 8.3Hz, ArH meta to OMe), 7.35 (1H, dd, J 8.5, 2.0Hz, ArH para to OMe), 7.40 (1 H, d, J 2.0Hz, ArH ortho to cyclopentyloxy), and 7.80 (2H, ca. d, J 8.3 Hz, ArH ortho to OMe), m/z (EI) 326 ($M^+$, 35%), 259 (35), 258 (97), 227 (40), 151 (80), 135 (100), 77 (22), and 41 (28).

d) **(3-Cyclopentyloxy-4-methoxyphenyl)(3-methoxyphenyl)ketone**

From Intermediate 34 (2.17g, 10.0mmol) and 3-bromoanisole (1.82g, 10.0mmol). Chromatography ($SiO_2$; EtOAc/hexane, 1:1) afforded the title compound (2.87g) as a white solid (Found: C, 73.60; H, 6.73. $C_{20}H_{22}O_4$ requires C, 73.60; H, 6.79%); m/z (EI) 327 ($M^+$ + 1,15%), 326 ($M^+$ + 67), 259 (42), 258 (98), 241 (16), 227 (18), 152 (20), 151 (100), 135 (40), and 83 (17).

**INTERMEDIATE 6**

**(E) and (Z) isomers of 4-[1-(3-Hydroxy-4-methoxyphenyl)-2-(4-pyridyl) ethenyl]pyridine**

A solution of the alcohol of Example 2 (0.72g, 1.85mmol) in toluene (120ml) containing 4-toluenesulphonic acid (0.88g, 4.6mmol) was heated to reflux in a Dean-Stark apparatus for 18h. The cooled reaction mixture was treated with aqueous NaOH (10%) then taken to pH 7 with concentrated hydrochloric acid. The mixture was extracted with $CH_2Cl_2$ (3x40ml), the extract washed with saturated $NaHCO_3$ (100ml), and $Na_2CO_3$ (10%;2x60ml), then dried ($MgSO_4$), and concentrated *in vacuo* to afford the title compound (0.4g) as a yellow foam; $\delta_H$ ($CDCl_3$) (major isomer) 3.88 (3H, s, OMe), 6.6-6.9 (6H, m, ArH ortho to OMe + 2xArH meta to OMe + $C=C_H$ + pyridine $H_3$, $H_5$), 7.08 (2H, dd, J 4.6, 1.6 Hz, pyridine $H_3$, $H_5$), 8.30 (2H, dd, J 4.5, 1.6 Hz, pyridine $H_2$, $H_6$), and 8.51 (2H, dd, J 4.4, 1.6 Hz, pyridine $H_2$, $H_6$), [the minor isomer displays a signal at δ 3.90 (3H, s, OMe)].

**INTERMEDIATE 7**

a) **(E) and (Z) isomers of 4-[2-(3-Cyclopentyloxy-4-methoxyphenyl)-2-phenylethenyl] pyridine**

The alcohol of Example 1a (3.13g, 8.05mmol) was dissolved in toluene (70ml) containing 4-toluenesulphonic acid monohydrate (1.91g, 10.05mmol) and the mixture heated to reflux for 1h. The reaction mixture was poured into aqueous NaOH (10%; 100ml) and stirred for 5 min. The mixture was extracted with $Et_2O$ (3x70ml) and the organic extract washed with water (80ml), and brine (80ml), then dried ($MgSO_4$), and concentrated *in vacuo* to afford the title compound (3.0g) as a viscous pale yellow oil. $\delta_H$ ($CDCl_3$) 1.5-2.1 (8H, br m, ($CH_2)_4$), 3.82 (major) and 3.84 (minor) (3H, s, OMe), 4.8 (1H, br m, $OCHCH_2$), 6.6-7.4 (11H, m, ArH ortho to OMe + 2xArH meta to OMe + $C_6H_5$+ pyridine $H_3$, $H_5$), and 8.2 - 8.35 (2H, m, pyridine $H_2$, $H_6$); m/z 372 ($M^+$ + 1, 12%), 371 ($M^+$, 40), 304 (21), 303 (100), 302 (72) and 274 (22).

The following compounds were prepared using a similar procedure:

b) **(E) and (Z) isomers of 2-[2-(3-Cyclopentyloxy-4-methoxyphenyl)-2-phenylethenyl] pyrazine**

From the alcohol of Example 1b (570mg, 1.5mmol) and 4-toluene sulphonic acid (about 20mg). Upon completion, the reaction mixture was concentrated *in vacuo* then subjected to chromatography ($SiO_2$; $Et_2O$) to afford the title compound (520mg) as a colourless oil. $\delta_H$ ($CDCl_3$) 1.5-2.0 (8H, br m, $(CH_2)_4$), 3.84 and 3.86 (3H, s, OMe), 4.58 and 4.72 (1H, br m, OCH), 6.65-7.5 (9H, m, $C_6H_5$+C=CH+ArH ortho to OMe+2xArH meta to OMe), 7.90 and 8.04 (1H, d, J 1.5Hz, pyrazine $H_3$), 8.18 and 8.21 (1H, d, J 2.5Hz, pyrazine $H_6$), and 8.45 and 8.48 (1H, m, pyrazine $H_5$).

c) **(E) and (Z) isomers of 3-[2-(3-Cyclopentyloxy-4-methoxyphenyl)-2-phenylethenyl]-2-methoxypyrazine**

From the compound of Example 7a (2.94g, 7.0mmol) and 4-toluenesulphonic acid (about 20mg) as described for Intermediate 7b to afford the title compound (2.67g) as a yellow oil. $\delta_H$ ($CDCl_3$) 1.5-2.0 (8H, br m, $(CH_2)_4$), 3.80, 3.81, 3.83, 3.86 (2 x 3H, s, 2 x OMe), 4.50, 4.70 (1H, br m,

OCH), 6.60-7.5 (9H, m, $C_6H_5$ + C = CH + ArH ortho to OMe + 2 x ArH meta to OMe) and 7.7-7.95 (2H, m, pyrazine $H_5$, $H_6$).

d) (i) **(E) 4-[2-(3-Cyclopentyloxy-4-methoxyphenyl)-2-phenylethenyl]-3,5-dichloropyridine**

(ii) **(Z) 4-[2-(3-Cyclopentyloxy-4-methoxyphenyl)-2-phenylethenyl] -3,5-dichloropyridine**

From the compound of Example 1c (1.60g, 3.58mmol) and 4-toluenesulphonic acid (0.85g). Purification by column chromatography ($SiO_2$; $CH_2Cl_2$) afforded:

i) (E) title compound (960mg) as an off-white solid m.p. 138.5-140°C. $\delta_H$ ($CDCl_3$) 1.5-2.0 (8H, br m, $(CH_2)_4$), 3.88 (3H, s, OMe), 4.72 (1H, br m, OCH), 6.59 (1H, s, C=CH), 6.85 (1H, d, J 8.4Hz, ArH ortho to OMe), 6.90 (1H, d, J 2.0Hz, ArH ortho to cyclopentyloxy), 6.95 (1H, dd, J 8.4, 2.0Hz, ArH para to cyclopentyloxy), 7.0-7.1 (2H, m, $H_2$, $H_6$ of $C_6H_5$), 7.15-7.3 (3H, m, $H_3$, $H_4$, $H_5$ of $C_6H_5$), and 8.35 (2H, s, pyridine $H_2$, $H_6$).

and ii) (Z) title compound (240mg) as an off-white solid. m.p. 155-156.5°C. $\delta_H$ ($CDCl_3$) 1.4-1.8 (8H, br m $(CH_2)_4$), 3.80 (3H, s, OMe), 4.42 (1H, br m OCH), 6.52 (1H, d, J 2.0 OHz,m ArH ortho to cyclopentyloxy), 6.56 (1H, s, C=CH), 6.57 (1H, dd, J 8.4, 2.0 Hz, ArH para to cyclopentyloxy), 6.68 (1H, d, J 8.4 Hz, ArH ortho to OMe), 7.3-7.45 (5H,m, $C_6H_5$), and 8.37 (2H, s, pyridine $H_2$, $H_6$).

e) **(E) and(Z) isomers of 3-[2-(3-Cyclopentyloxy-4-methoxy phenyl)-2-phenylethenyl]pyridazine**

From the compound of Example 7b (4.0g). Purification by chromatography ($SiO_2$; $Et_2O$) afforded the title compound (2.07g) as a pale yellow solid (Found: C, 77.59; H, 6.49; N, 7.24. $C_{24}H_{24}N_2O_2$ requires C, 77.39; H, 6.50; N, 7.52%); $\delta_H$ ($CDCl_3$) 1.5-1.9 (8H, br m, $(CH_2)_4$), 3.88 ,3.90 (3H, s, OMe), 4.58, 4.70 (1H, br m, OCH), 6.6-7.5 (11H, m, $C_6H_5$+$C_6H_3$+C=CH + pyridazine $H_4$, $H_{5+}$ ), and 8.85-8.90 (1H, m, pyridazine $H_6$) ('Hnmr indicates a 3:2 E/Z ratio); m/z (ESI) 396 ($M^+$+1+Na, 57%), 395 ($M^+$+Na,100), 374 (66), 373 (78), and 305 (16).

f) **(E) and (Z) isomers of 2-[2-(3-Cyclopentyloxy-4-methoxy phenyl)-2-phenylethenyl]-4-methylpyridine**

From the compound of Example 7c (1.15g, 2.85mmol). Purification by chromatography ($SiO_2$; EtOAc) afforded the title compound (1.2g) as a pale yellow solid; $\delta_H$ ($CDCl_3$) 1.4-1.9 (8H, br m, $(CH_2)_4$), 2.04 (major), 2.09 (minor) (3H, pyridine Me), 3.85 (major), 3.88 (minor) (3H, s, OMe), 4.58 (minor, 4.72 (major) (1H, br m, OCH), 6.4-7.5 (11H, m, $C_6H_5$+$C_6H_3$+pyridine $H_3$, $H_{5+}$ C=CH), 8.5-8.55 (1H, m, pyridine $H_6$). 'Hn.m.r indicates a 2:1 E/Z ratio.

g) **(E) and (Z) isomers of 4-[2-(3-Cyclopentyloxy-4-methoxy phenyl)-2-phenylethenyl]pyrimidine**

From the compound of Example 7d (2.55g). Purification by chromatography ($SiO_2$; $Et_2O$) afforded the title compound (1.20g) as a pale yellow foam; $\delta_H$ ($CDCl_3$) 1.5-2.0 (8H, br m, $(CH_2)_4$), 3.88,3.90 (3H, s, OMe), 4.60, 4.70 (1H, br m, OCH), 6.44, 6.64 (1H, d, J 5.2Hz, pyrimidine $H_5$), 6.65-7.0 (3H, m, $C_6H_3$), 7.2-7.45 (6H, m, $C_6H_5$+C=CH), 8.26, 8.32 (1H, d, J 5.2Hz, pyrimidine $H_6$), and 9.10, 9.12 (1H, ca s, pyrimidine $H_2$).

h) **(E) and (Z) isomers of 4-[2-(3-Cyclopentyloxy-4-methoxy phenyl)-2-(3-methoxyphenyl)ethenyl]pyridine**

From the compound of Example 2b. Chromatography ($SiO_2$; EtOAc) afforded the title compound as a yellow oil (0.90g).

i) **(E) and (Z) isomers of 4-[2-(3-Cyclopentyloxy-4-methoxy phenyl)-2-(2-methoxyphenyl)ethenyl]pyridine**

From the compound of Example 2c. Chromatography ($SiO_2$; EtOAc) afforded the title comound as a yellow oil (0.75g).

**INTERMEDIATE 8**

**(E) and (Z) isomers of 4-[1-(3-Cyclopentyloxy-4-methoxyphenyl)-2-(4-pyridyl)ethenyl] pyridine**

A mixture of Intermediate 6 (0.48g, 1.58mmol), $Cs_2CO_3$ (0.56g,1.73mmol), and cyclopentyl bromide (0.26g, 1.743mmol) in DMF (20ml) was stirred at RT overnight. A further portion of $Cs_2CO_3$ (0.20g, 0.61 mmol) and cyclopentyl bromide (0.28g, 1.86mmol) was added, the mixture stirred for 1.5h then concentrated *in vacuo*. The residue was subjected to chromatography ($SiO_2$; EtOAc/$CH_3OH$/$Et_3N$, 100:1:0.4) to afford the title compound (0.42g) as a white solid. m.p. 136-138°C (cyclohexane); $\delta_H$ ($CDCl_3$) 1.5-2.0 (8H, br m ($CH_2$)$_4$), 3.84 (3H, s, OMe), 4.65 (1H, br m OCHC$H_2$), 6.7-6.9 (6H, m, ArH ortho to OMe+2xArH meta to OMe+C=CH + pyridine $H_3$,$H_5$), 7.08 (2H, dd, J 4.5, 1.5 Hz, pyridine pyridine $H_3$', $H_5$'), 8.32 (2H, dm, J 5.0 Hz pyridine $H_2$, $H_6$), and 8.55 (2H, dd, J 4.5, 1.5Hz, pyridine $H_2$', $H_{6'}$); m/z 372 ($M^+$28%), 305 (37), 304 (100), 303 (95), 275 (18), and 41 (18)

**INTERMEDIATE 9**

**1-(3-Cyclopentyloxy-4-methoxyphenyl)-1-phenylethene**

To a cold suspension (0°C) of methyl triphenylphosphonium bromide (53.6g; 0.15mol) in THF (500ml) under a nitrogen atmosphere was added n-BuLi (1.6M in hexanes; 94ml, 0.15mol) dropwise and the reaction mixture stirred at 0°C for 1h. A solution of Intermediate 2 (29.6g, 0.1 mol) in THF (100ml) was added dropwise and the stirred reaction mixture allowed to warm to RT over 3h. The mixture was poured into 10% $NH_4Cl$ solution (600ml) and extracted with $CH_2Cl_2$ (2x500ml). The combined organic layer was dried ($MgSO_4$), filtered and concentrated *in vacuo.* The residual slurry was triturated with hot hexane (500ml), the precipitated phosphine oxide filtered off and the filtrate evaporated *in vacuo* to yield the title compound (28.85g) as a yellow oil. $\delta_H$ ($CDCl_3$) 1.5-2.0 (8H, br m, ($CH_2$)$_4$), 3.85 (3H, s, OMe), 4.71 (1H, br m, OCH), 5.38 (2H, dd, J 10.5, 1.3Hz, C=$CH_2$), 6.75-6.9 (3H, m, $C_6H_3$), and 7.3-7.5 (5H, m, $C_6H_5$).

**INTERMEDIATE 10**

a) **(E) and (Z) isomers of 4-[2-(3-Cyclopentyloxy-4-methoxy phenyl)-2-phenylethenyl] phenol**

A mixture of Intermediate 9 (2.94g, 10mmol), 4-bromophenol (2.16g, 12.5mmol), $Et_3N$ (2.52g, 25mmol), tri-o-tolyl phosphine (0.06g, 0.2mmol) and palladium acetate (0.022g, 0.1mmol) was heated in a bomb at 140°C for 16h. Upon cooling, the reaction mixture was diluted with $NH_4Cl$ (10%; 50ml) and $CH_2Cl_2$ (50ml). The organic layer was separated and the aqueous layer extracted with $CH_2Cl_2$ (50ml). The combined organic layer was dried ($MgSO_4$), filtered and concentrated. Purification by column chromatography ($SiO_2$; hexane/$Et_2O$,1:1) yielded the title compound (1:1 mixture of isomers) (0.8g) as a yellow foam. $\delta_H$ ($CDCl_3$) 1.2-1.9 (8H, br m, ($CH_2$)$_4$), 3.81, 3.83 (3H, s, OMe), 4.59, 4.69 (1H, br m, OCH), 5.5, 5.63 (1H, br s, OH), 6.55-7.0 (8H, m, $C_6H_3$+$C_6H_4$+C=CH), and 7.15-7.35 (5H, m, $C_6H_5$) [N.B. 'Hn.m.r. indicates ca 1:1E/Z mixture of isomers); m/z (ESI) 410 ($M^+$+1+Na, 18%), 409 ($M^+$+Na, 100) 387 ($M^+$+1, 62), 319 (38), 318 (22), 301 (19), 236 (22), and 135 (20).

The following compounds were prepared using a similar procedure:

b) **(E) and (Z) isomers of 3-[2-(3-Cyclopentyloxy-4-methoxy phenyl)-2-phenylethenyl] benzoic acid**

From Intermediate 9 (2.94g, 10mmol) and 3-bromobenzoic acid (5.03g, 25mmol). Purification by column chromatography [$SiO_2$;10%, $CH_3OH$/ $CH_2Cl_2$] furnished the title compounds (2g) as a viscous yellow oil. $\delta_H$ ($CDCl_3$) 1.45-2.0 (8H, br m, ($CH_2$)$_4$), 3.86, 3.87 (3H, s, OMe), 4.55, 4.7 (1H, br m, OCH), 6.65-8.25 (13H, m, $C_6H_5$+$C_6H_4$+$C_6H_3$+C=CH), ($CO_2H$ not observed) [N.B. 'Hn.m.r. indicates ca 1:1E/Z mixture of isomers]; m/z (ESI) 437 ($M^+$+23, 60%), 301 (67), 281 (100), and 259 (52).

c) **(E) and (Z) isomers of 4-[2-(3-Cyclopentyloxy-4-methoxy phenyl)-2-phenylethenyl] anisole**

From Intermediate 9 (1.19g, 4.04mmol) and 4-bromoanisole (0.757g, 4.05mmol). Purification by column chromatography [$SiO_2$; hexane/$Et_2O$, 4:1] furnished the title compounds (0.78g) as a yellow oil. $\delta_H$ ($CDCl_3$) 1.5-2.0 (8H, br

m, ($CH_2)_4$), 3.72, 3.73 (3H, s, OMe), 3.82, 3.86 (3H, s, OMe), 4.58, 4.67 (1H, br m, OCH), 6.6-6.9 (6H, m, $C_6H_3$+2xArH ortho to OMe+C=CH), 6.93, 7.00 (2H, d, J 8.5Hz, 2xArH meta to OMe) and 7.15-7.35 (5H, m, $C_6H_5$) [N.B. 'Hn.m.r. indicates ca 1:1E/Z mixture of isomers]; m/z (ESI) 424 ($M^+$+1+Na, 20%), 423 ($M^+$+Na, 100%), 374 (12), 281 (20), 198 (12), 132 (12) and 86 (12).

d) **(E) and (Z) isomers of Methyl 4-[2-(3-Cyclopentyloxy-4-methoxyphenyl)-2-phenylethenyl]benzoate**

From Intermediate 9 (2.94g, 10mmol) and methyl 4-bromobenzoate (2.69g, 12.5mmol) to afford the title compounds (3.35g) as a yellow gum; $\delta_H$ ($CDCl_3$) 1.4-2.0 (8H, br m, $(CH_2)_4$), 3.86, 3.87 (6H, s, OMe+$CO_2Me$), 4.54, 4.67 (1H, br m, OCH), 6.6-7.4 (11H, m, $C_6H_5$+$C_6H_3$+C=CH+2xArH meta to $CO_2Me$), and 7.75-7.85 (2H, m, 2xArH ortho to $CO_2Me$) [N.B. 'Hn.m.r. indicates ca 1:1E/Z mixture of isomers]; m/z (ESI) 429 ($M^+$+1+Na, 28%), 362 (18), 361 (28), 330 (70), and 329 (68).

e) **(E) and (Z) isomers of 3-[2-(3-Cyclopentyloxy-4-methoxyphenyl)-2-phenylethenyl]pyridine**

From Intermediate 9 (1.00g, 3.4mmol) and 3-bromopyridine (1.28g, 8.1mmol). Purification by chromatorgaphy ($SiO_2$; $Et_2O$) afforded the title compound (0.50g) as a pale yellow gum; $\delta_H$ ($CDCl_3$) 1.45-2.0 (8H, br m, $(CH_2)_4$), 3.85 (major), 3.87 (minor) (3H, s, OMe), 4.55 (minor), 4.69 (major) (1H, br m, OCH), 6.65-7.5 (11H, m, $C_6H_5$+$C_6H_3$+pyridine $H_4$,$H_5$+ C=C), and 8.2-8.45 (2H, m, pyridine $H_2$,$H_6$).

**INTERMEDIATE 11**

**(E) and (Z) isomers of 4-[2-(3-Cyclopentyloxy-4-methoxyphenyl)-2-phenylethenyl] acetoxybenzene**

To a stirred solution of Intermediate 10a (0.2g, 0.52mmol) in $CH_2Cl_2$ (5ml), under a nitrogen atmosphere, was added $Et_3N$ (0.101g, 0.14ml, 1 mmol) followed by acetyl chloride (0.0785g, 0.071ml, 1mmol). The reaction mixture was stirred at RT for 4h then poured into saturated $NaHCO_3$ (10ml). The organic layer was separated and the aqueous layer extracted with $CH_2Cl_2$. The combined organic layer was dried ($MgSO_4$), filtered, and the solvent removed *in vacuo* to furnish the title compounds (0.222g) as a colourless oil. $\delta_H$ ($CDCl_3$) 1.5-1.9 (8H, br m, $(CH_2)_4$), 2.23, 2.24 (3H, s, OCOMe), 3.83, 3.86 (3H, s, OMe), 4.56, 4.67 (1H, br m, OCH), and 6.7-7.4 (13H, m, $C_6H_5$+$C_6H_4$+$C_6H_3$+C=CH) [N.B. 'Hn.m.r. indicates ca 1:1E/Z mixture of isomers]; m/z (ESI) ($M^+$+Na, 100% ), 319 (20), 281 (29), 191 (48), 127 (50) and 55 (54).

**INTERMEDIATE 12**

**(E) and (Z) isomers of Methyl 3-[2-(3-cyclopentyloxy-4-methoxy phenyl)-2-phenylethenyl]benzoate**

To a cold (0°C) solution of Intermediate 10b (0.25g, 0.6mmol) in $CH_3OH$ (20ml) was added $SOCl_2$ (0.357g, 0.22ml,3mmol) dropwise and the reaction mixture was stirred at RT for 3h. The solvent was evaporated *in vacuo*, the residue dissolved in $CH_2Cl_2$ (20ml) and washed with saturated $NaHCO_3$ (20ml). The organic phase was separated and the aqueous phase extracted with $CH_2Cl_2$ (20ml). The combined organic layer was dried ($MgSO_4$), filtered and the solvent evaporated *in vacuo* to yield the title compound (0.215g) as a yellow oil. $\delta_H$ ($CDCl_3$) 1.4-2.0 (8H, br m, $(CH_2)_4$), 3.82, 3.83, 3.84, 3.85 (6H, s, OMe + $CO_2Me$), 4.54, 4.69 (1H, br m, OCH), and 6.65-7.85 (13H, m, $C_6H_5$+$C_6H_4$+$C_6H_3$+C=CH) [N.B. 'Hn.m.r. indicates ca 1:1E/Z mixture of isomers]; m/z (ESI) 429 ($M^+$+1, 25%), 361 (22), 329 (100), 159(12), 102 (15), and 60 (75).

**INTERMEDIATE 13**

**Ethyl (E)-(3-Cyclopentyloxy-4-methoxyphenyl)-2-(4-pyridyl) propenoate**

A mixture of Intermediate 1 (26.62g, 0.12mol), ethyl-4-pyridylacetate (19.92g, 0.12mol, 1eq) and ammonium acetate (18.63g, 0.24g, 2eq) in glacial acetic acid (200ml) was stirred at 120°C under nitrogen for 20h. The solution was cooled to RT and the acid removed *in vacuo.* The orangy/brown residue was taken up in saturated $NaHCO_3$ solution to pH 8.5 and the aqueous layer extracted several times with EtOAc. The combined organic layer was washed (brine), dried ($MgSO_4$) and evaporated to dryness to give a yellow solid. Recrystallisation from toluene/hexane (1st crop) then toluene (2nd crop) followed by column chromatography (hexane-EtOAc/hexane, 7:3) gave the title compound as a white crystalline solid. m.p. 109-110°C. $\delta_H$ ($CDCl_3$) 1.27(3H, t, J 7.1Hz, $CH_2CH_3$), 1.45-1.8 (8H, br m, cyclopentyl H's), 3.81 (3H, s, OMe), 4.16 (1H, br m, OCH), 4.25 (2H, q, J 7.1 Hz, $CH_2CH_3$), 6.43 (1H, d J 2.0 Hz, ArH

ortho to cyclopentyloxy), 6.73 (1H, d, J 8.4 Hz, ArH ortho to OMe), 6.80 (1H, dd, J 2.0, 8.4 Hz, ArH para to cyclopentyloxy), 7.22 (2H, dd, J 1.6, 4.5Hz, pyridine $H_3$, $H_5$), 7.83 (1H, s, HC = C) and 8.64 (2H, dd, J 1.6, 4.5 Hz, pyridine $H_2$, $H_6$).

## INTERMEDIATE 14

### Ethyl 3-(3-Cyclopentyloxy-4-methoxyphenyl)-3-(4-fluorophenyl)-2-(4-pyridyl)propanoate

4-Fluorophenylmagnesium bromide (2M in $Et_2O$; 20.4ml, 40.8mmol) was added dropwise at -40°C over 20min to a suspension of copper (I) bromide-dimethyl sulphide complex (4.17g, 20.4mmol) in THF (50ml). The reaction mixture was warmed to -10°C over 15 min and a solution of Intermediate 13 (5g, 13.6mmol) in THF (25ml) was added dropwise over 15 min. The reaction mixture was allowed to warm slowly (about 2h) to RT, and quenched with saturated aqueous $NH_4Cl$ (30ml). The organic phase was extracted and evaporated. The concentrate was partitioned between EtOAc (150ml) and water (50ml) and filtered through Celite®. The organic extract was washed with 10% $NH_4OH$ (2 x 100ml) and brine (100ml), dried ($MgSO_4$) and evaporated to give a pale yellow gummy solid. Trituration with hot $Et_2O$ provided a white solid which was filtered off and washed with cold $Et_2O$. Purification by column chromatography ($SiO_2$; EtOAc/hexane, 1:1) furnished the title compound (2.2g) as a single isomer. $\delta_H$ ($CDCl_3$) 1.05 (3H, t, $COCH_2CH_3$), 1.6-2.0 (8H, br m, $(CH_2)_4$), 3.80 (3H, s, $OCH_3$), 4.0 (2H, m, $COCH_2$), 4.30 (1H, d, CHAr), 4.60 (1H, d, $CHCO_2Et$), 4.80 (1H, m, $OCHCH_2$), 6.75-7.0 (7H, m, Ar), 7.25 (2H, d, Ar), 8.45 (2H, d, Ar).

## INTERMEDIATE 15

### 3.5-Dichloro-4-methylpyridine

3,5-Dichloropyridine (2.04g, 13.5mmol) in THF (5ml) was added dropwise to a solution of LDA [prepared from diisopropylamine (1.9ml, 13.5mmol) and n-BuLi (1.6M; 8.4ml, 13.5mmol)] in THF (25ml) at -70°C. After stirring at this temperature for 5 min, iodomethane (0.85ml, 13.5 mmol) was added and the reaction mixture stirred for a further 1.5h at -70°C. Saturated $NaHCO_3$ (20ml) and $CH_2Cl_2$ (20ml) were added, the organic phase separated, dried ($MgSO_4$), and concentrated *in vacuo*. The residue was subjected to chromatography ($SiO_2$; $Et_2O$/hexane, 1:3) to afford the title compound (1.16g) as a pale yellow solid. $\delta_H$ ($CDCl_3$) 2.46 (3H, s, Me), and 8.36 (2H, s, pyridine $H_2$, $H_6$).

## INTERMEDIATE 16

### Ethyl 3-(3-Cyclopentyloxy-4-methoxyphenyl)-2-ethoxycarbonyl propenoate

A mixture of Intermediate 1 (109.8, 499.1 mmol). diethyl malonate (79.96, 499.1 mmol). piperidine (2.5ml) and $CH_3CO_2H$ (12ml) in toluene (700ml) was heated to reflux in a Dean-Stark apparatus for 20h. Further portions of diethyl malonate (9.6g, 59.9mmol), piperidine (2.5ml), and $CH_3CO_2H$ (12ml) were added and heating continued as before for 15h. The reaction mixture was concentrated *in vacuo* to afford the title compound (217g) as a brown oil. $\delta_H$ ($CDCl_3$) 1.33 (6H, t, J 7.1Hz, $2xCO_2CH_2Me$), 1.5-2.05 (8H, br m, $(CH_2)_4$), 3.88(3H, s, OMe), 4.30 (2H, q, J 7.1 Hz, $CO_2CH_2Me$), 4.36 (2H, q, J 7.1 Hz, $CO_2CH_2Me$), 4.73 (1H, br m, OCH), 6.85 (1H, d, J 8.1 Hz, ArH ortho to OMe), 7.0-7.1 (2H, m, 2xArH meta to OMe), and 7.63 (1H, s, $HC=CCO_2Et$).

## INTERMEDIATE 17

**Diethyl 2-[(3-Cyclopentyloxy-4-methoxyphenyl)phenylmethy]propan-1-3-dioate**

Phenylmagnesium bromide (1.0M in THF;340ml, 340mmol, 1.29eq) was added over 1.5h to a solution of Intermediate 16 (95.6g, 264mmol) in THF (200ml) at -60°C and stirred at this temperature for a further 5h. The reaction mixture was allowed to warm to -20°C, quenched with 10% aqueous $NH_4Cl$ (200ml), then extracted with EtOAc (3x100ml). The extract was dried ($MgSO_4$), concentrated *in vacuo*, the residual brown oil dissolved in EtOH and allowed to crystallise overnight to afford the title compound (74.9g) as a white solid. m.p. 97-98°C. $\delta_H$ ($CDCl_3$) 1.01 (6H, t, J 7.1Hz, $CO_2CH_2Me$), 1.05 (3H, t, J 7.1Hz, $CO_2CH_2Me$), 1.5-2.0 (8H, br m, $(CH_2)_4$), 3.77 (3H, s, OMe), 3.9-4.1 (4H, m, $2xCO_2CH_2Me$), 4.26 (1H, d, J 12.1Hz, $CHCHCO_2Et$), 4.67 (1H, d, J 12.1Hz, $CHCHCO_2Et$), 4.71 (1H. br m, OCH), 6.7-6.85 (3H, m, $C_6H_3$), and 7.15-7.35 (5H, m, $C_6H_5$).

**INTERMEDIATE 18**

**3-(3-Cyclopentyloxy-4-methoxyphenyl)-3-phenylpropanoic acid**

A mechanically stirred solution of Intermediate 17 (70.3g, 0.160mol) in NaOH solution (8M; 600ml) and dioxane (600ml) was heated to reflux for 7h. The reaction mixture was cooled, concentrated hydrochloric acid (about 400ml) added dropwise to pH4 and heating carried overnight to give a homogenous solution. The dioxane was removed *in vacuo* and the mixture partitioned between $CH_2Cl_2$ (500ml) and $H_2O$ (500ml). The organic layer was separated and combined with further $CH_2Cl_2$ extracts (3x150ml). The extract was dried ($MgSO_4$) and concentrated *in vacuo* to give the title compound (55g) as a yellow solid. $\delta_H$ ($CDCl_3$) 1.5-2.0 (8H, br m, $(CH_2)_4$), 3.04 (2H, d, J7.9Hz, $CHCH_2CO_2H$), 3.80 (3H, s, OMe), 4.45 (1H, t, J 7.9Hz $CHCH_2CO_2H$), 4.70 (1H, br m, OCH), 6.7-6.8 (3H, m, $C_6H_3$), and 7.15-7.35 (5H, m, $C_6H_5$) (N.B. $CO_2H$ not observed).

**INTERMEDIATE 19**

**3-(3-Cyclopentyloxy-4-methoxyphenyl)-3-phenylpropanoyl chloride**

$SOCl_2$ (14.8ml, 24.1g, 3eq) was added to a solution of Intermediate 18 (23.0g, 67.5mmol) in $CH_2Cl_2$ (250ml) and then heated to reflux for 6h. The reaction mixture was allowed to stir at RT overnight then concentrated *in vacuo* to afford the title compound (23.7g) as a dark brown oil. $\delta_H$ ($CDCl_3$) 1.5-2.0 (8H, br m, $(CH_2)_4$), 3.62 (2H, d, J 8.0Hz, $CHCH_2COCl$), 3.82 (3H, s, OMe), 4.56 (1H, t, J 8.0Hz, $CHCH_2COCl$), 4.73 (1H, br m, OCH), 6.7-6.85 (3H, m, $C_6H_3$), and 7.15-7.4 (5H, m, $C_6H_5$).

**INTERMEDIATE 20**

**5-(3-Cyclopentyloxy-4-methoxyphenyl)-1-[2-(1,3-dioxolanyl)]-5-phenyl-3-pentanone**

A solution of the Grignard reagent (1.0M in THF, 29ml, 29.0mmol, 1.2eq) [prepared from 2-(2-bromoethyl)-1,3-dioxolane (5.25g, 29.0mmol) and magnesium (10.8g, 33mmol)] was added dropwise at -70°C to a solution of Intermediate 19 (8.7g, 24.3mmol), in THF (200ml). The reaction mixture was stirred at -70°C for 0.5h, allowed to warm to RT over 1.75h then partitioned between $Et_2O$ (200ml) and aqueous NaOH (1M; 100ml). The organic layer was separated and combined with a further $Et_2O$ extract (150ml). The extract was washed with brine (50ml),dried ($MgSO_4$) and concentrated *in vacuo.* Purification by column chromatography ($SiO_2$; 20% EtOAC/hexane) furnished the title compound (3.95g) as an off-white waxy solid. m.p. 60-62°C. $\delta_H$ ($CDCl_3$) 1.5-2.0 (10H, br m, $(CH_2)_4$ + $CH_2CH_2CO$), 2.46 (2H, t, J 7.5Hz, $CH_2CH_2CO$), 3.13 (2H, d, J 7.6Hz, $CHCH_2CO$), 3.7-4.0 (4H, m, $O(CH_2)_2O$), 3.78 (3H, s, OMe), 4.53 (1H, t, J 7.6 Hz, $CHCH_2CO$), 4.68 (1H, m, ArOCH), 4.80 (1H, t, J 4.3Hz, OCHO), 6.65-6.8 (3H, m, $C_6H_3$), and 7.1-7.3 (5H, m, $C_6H_5$).

**INTERMEDIATE 21**

**6-(3-Cyclopentyloxy-4-methoxyphenyl)-4-oxo-6-phenyl-1-hexanal**

A solution of Intermediate 20 (800mg) in a mixture of aqueous HCl (2M; 5ml) and THF (15ml) was heated at about 45°C for 1.5h. The reaction mixture was concentrated to low volume (about 5ml) and partitioned between $Et_2O$ (50ml) and $H_2O$ (10ml). The organic layer was separated and combined with a further $Et_2O$ extract (30ml). The extract was washed with saturated $NaHCO_3$ (40ml), then brine (10ml), dried ($MgSO_4$) and concentrated *in vacuo.* The residual orange oil was subjected to chromatography($SiO_2$; $Et_2O$-hexane) to afford the title compound (450mg) as a pale yellow oil. $\delta_H$ ($CDCl_3$) 1.5-2.0 (8H, br m, $(CH_2)_4$), 2.6-2.7 (4H, m, $CH_2CH_2CHO$), 3.19 (2H, d, J 7.6Hz, $CHCH_2CO$), 3.79 (3H, s, OMe), 4.52 (1H, t, J 7.6Hz, $CHCH_2CO$), 4.70 (1H, br m, OCH), 6.7-6.8 (3H, m, ArH ortho to OMe + 2x ArH meta to OMe), 7.1-7.3 (5H, m, $C_6H_5$), and 9.71 (1H, s, $CH_2CHO$).

**INTERMEDIATE 22**

**Ethyl 5-(3-Cyclopentyloxy-4-methoxyphenyl)-3-oxo-5-phenylpentanoate**

n-BuLi(1.6M in hexanes; 29.3ml, 46.9mmol, 4.2eq) was added dropwise at -50°C to a solution of potassium ethyl malonate (2.95g, 22.3mmol. 2.1eq) in THF (60ml). The reaction mixture was allowed to warm to -10°C, stirred for 10 min, then recooled to -65°C and treated dropwise with a precooled solution of Intermediate 19 (4.0g, 11.1mmol)

in THF (20ml). The reaction mixture was stirred at -65°C for 20 min, then poured into a stirred mixture of $Et_2O$ (100ml) and aqueous HCl (1M; 150ml). After 0.5h, the organic phase was separated and combined with further $Et_2O$ extracts (2x75ml). The extract was dried ($MgSO_4$), concentrated *in vacuo*, and the residual oil subjected to chromatography ($SiO_2$; 40% $Et_2O$-hexane) to afford a colourless oil (3.4g) which crystallised on standing to give the title compound as a white solid. m.p. 56-58°C (EtOH). $\delta_H$ ($CDCl_3$) 1.24 (3H, t, J 7 Hz, $CO_2CH_2Me$), 1.5-1.9 (8H, br m, $(CH_2)_4$), 3.27 (2H. d J 7.5Hz, $CHCH_2CO$), 3.33 (2H, s, $CH_2CO_2Et$), 3.79 (3H, s, OMe), 4.14 (2H, q, J 7 Hz, $CO_2CH_2Me$), 4.52 (1H, t, J 7.5Hz, $CHCH_2CO$), 4.69 (1H, m, OCH), 6.7-6.8 (3H, m, $C_6H_3$), and 7.1-7.35 (5H, m, $C_6H_5$).

**INTERMEDIATE 23**

**(±)4-[2-(3-Hydroxy-4-methoxyphenyl)-2-phenylethyl]pyridine**

The compound of Example 3a (430mg) in dioxane/water (20ml:10ml) containing concentrated $H_2SO_4$ (10ml) was heated at 90°C for 1h. The reaction mixture was cooled, neutralised with aqueous $NaHCO_3$ then concentrated *in vacuo*. The residue was partitioned between EtOAc (25ml) and H20 (15ml), and the organic phase separated. The extract was washed with brine (25ml), dried ($MgSO_4$) and concentrated *in vacuo*. The residue was recrystallised (EtOH) to afford the title compound (240mg) as an off-white crystalline solid m.p. 195-197°C (Found: C, 78.66; H, 627; N, 4.59. $C_{20}H_{19}NO_2$ requires C, 78.64; H, 6.18; N, 4.42%); $\delta_H$ ($CDCl_3$) 3.30 (2H, d, J 8 Hz, $CHCH_2$), 3.86 (3H, s, OMe), 4.13 (1H, t, J 8Hz, $CHCH_2$), 5.7 (1H, br s, OH), 6.63 (1H, dd, J 8.3Hz, ArH para to OH), 6.71 (1H, d, J 8.3Hz, ArH ortho to OMe), 6.80 (1H, d, J 2.2Hz, ArH ortho to OH), 6.93 (2H, dd, J 4.5, 1.5Hz, pyridine $H_3$, $H_5$), 7.1-7.3 (5H, m, $C_6H_5$), and 8.37 (2H, dd, J 4.5 ,1.5Hz, pyridine $H_2$,$H_6$).

**INTERMEDIATE 24**

**(2S*,3S*) and (2S*,3R*) Ethyl 3-(3-Cyclopentyloxy)-4-methoxyphenyl)-3-[4-(1,3-dioxolanyl)phenyl]-2-(4-pyridyl) propanoate**

2-(4-bromophenyl)-1,3-dioxolane (3.25g, 14.2mml) in THF (10ml) was added dropwise to a stirred suspension of magnesium turnings (358mg,14.8mmol) in THF (5ml) at 40-45°C. The resulting green solution was allowed to cool to RT and copper (I) chloride (28mg, 0.28mmol) was added. The reaction mixture was cooled to -30°C, Intermediate 13 (4.34g, 11.8mmol) in THF (15ml) added at -25°C to -30°C, then stirred for 1h at -20°C and allowed to warm to RT over 2h. Saturated aqueous $NH_4Cl$ (20ml) was added, THF removed *in vacuo* and the concentrate partitioned between $Et_2O$ (50ml) and water (50ml). The organic layer was separated, washed with brine (20ml), dried ($MgSO_4$), and concentrated *in vacuo*. The residue was subjected to chromatography ($SiO_2$; $Et_2O$ to $Et_2O$-EtOAc, 1:1) to afford

i) (2S*, 3R*)- title compound (1.45g) as a colourless gum; $\delta_H$ ($CDCl_3$) 1.02 (3H, t, J 7.1 Hz, $CO_2CH_2Me$), 1.5-2.0 (8H, br m, $(CH_2)_4$), 3.70 (3H, s, OMe), 3.8-4.2 (6H, complex m, $O(CH_2)O$ + $CO_2CH_2Me$), 4.35 (1H, d, J 8.0 Hz, $CHCHCO_2Et$), 4.55 (1H, br m, OCH), 4.60 (1H, d, J 8.0Hz, $CHCHCO_2Et$), 5.78 (1H, s, OCHO), 6.5-6.65 (3H, m, $C_6H_3$), 7.22 (2H, d, J 6.0Hz, pyridine $H_3$, $H_5$), 7.35-7.5 (4H, m, $C_6H_4$), and 8.45 (2H, d, J 6.0Hz, pyridine $H_2$,$H_6$) and
ii) (2S*, 3S*) - title compound (1.45g) as a white solid; $\delta_H$ ($CDCl_3$) 1.03 (3H, t, J 7.1Hz, $CO_2CH_2Me$), 1.5-2.0 (8H, br m, $(CH_2)_4$), 3.80 (3H, s, OMe), 3.9-4.1 (6H, complex m, $O(CH_2)O$ + $CO_2CH_2Me$), 4.36 (1H, d, J 8.0 Hz, $CHCHCO_2Et$), 4.60 (1H, d, J 8.0Hz, $CHCHCO_2Et$), 4.78 (1H, br m, OCH), 5.66 (1H, s, OCHO), 6.78 (1H, d, J 8.2Hz, ArH of $C_6H_3$), 6.85-6.95 (2H, m, 2xArH of $C_6H_3$), 7.08 (2H, d, J 6.0Hz, 2xArH of $C_6H_4$), 7.15-7.3 (4H, m, 2xArH of $C_6H_4$+pyridine $H_3$, $H_5$), and 8.42 (2H, ca. d, J 6.0Hz, pyridine $H_2$,$H_6$).

**INTERMEDIATE 25**

**(S*,R*) and (S*,S*) Ethyl 3-(3-Cyclopentyloxy-4-methoxyphenyl)-3-(4-trifluoromethylphenyl)-2-(4-pyridyl) propanoate**

4-Bromo(trifluoromethyl)benzene (3.43ml, 24.5mmol) was added dropwise to a suspension of magnesium turnings (614mg,25.3mmol) in $Et_2O$ (15ml). A crystal of iodine was added and the mixture gently warmed to initiate the reaction. The dark brown solution was then added dropwise *via* a syringe to a suspension of copper bromide-dimethyl sulphide complex (2.48g, 12.24mmol) in THF (30ml) at -40°C. The red-brown suspension was allowed to warm to -20°C over 0.5h then re-cooled to -40°C and treated with a solution of Intermediate 13 (3.00g, 8.16mmol) in THF (15ml) over 5 min. The reaction mixture was allowed to warm to RT over 2h, stirred overnight at RT, then heated at 40°C for 3h. The reaction mixture was quenched with $NH_4Cl$ solution (20ml), concentrated *in vacuo*, and the residue partitioned between EtOAc (50ml) and water (25ml). The mixture was filtered through Celite® and the organic layer

was separated, washed with aqueous $NH_4OH$ (10%; 25ml), and brine (25ml), dried ($MgSO_4$), and cconcentrated *in vacuo* to give a red-brown oily residue which was subjected to chromatography ($SiO_2$; $Et_2O$-hexane) to afford the title compound (ca 1:1) (2.05g) as a pale yellow gum; $\delta_H$ ($CDCl_3$) 1.10-1.15 (3H, m, $CO_2CH_2Me$), 1.5-2.0 (8H, br m, $(CH_2)_4$), 3.73, 3.84 (3H, s, OMe), 3.9-4.15 (2H, m, $CO_2CH_2Me$), 4.40 (1H, d, J 8.0 Hz, $CHCHCO_2Et$), 4.58, 4.80 (1H, br m, OCH), 4.6-4.75 (1H, m, $CHCHCO_2Et$), 6.5-6.7, 6.8-7.05 (3H, m, $C_6H_3$), 7.1-7.7 (6H, m, $C_6H_4$+ pyridine $H_3$, $H_5$), and 8.48 (2H, br s, pyridine $H_2$,$H_6$).

## INTERMEDIATE 26

### (E) and (Z) isomers of 4-[2-(4-Aminophenyl)-2-(3-cyclopentyloxy-4-methoxyphenyl)ethenyl]pyridine

Water (15ml) and trifluoroacetic acid (10ml) were added to Intermediate 13 (6.1g) in $CH_2Cl_2$ (15ml) at O°C and the mixture allowed to warm to RT. After 6h, the reaction mixture was concentrated *in vacuo* and the residue partitioned between 10% hydrochloric acid (50ml) and EtOAc (50ml). The aqueous layer was separated, basified to pH 14 with 20% sodium hydroxide solution, and extracted with $CH_2Cl_2$ (3x50ml). The extract was dried ($MgSO_4$) and concentrated *in vacuo* to give the crude title compound (4.2g). A portion (0.40g) was subjected to chromatography ($SiO_2$); EtOAc) to afford the title compound (0.29g); $\delta_H$ ($CDCl_3$) 1.45-2.0 (8H, br m, $(CH_2)_4$), 3.80 (2H, br s, $NH_2$), 3.87, 3.90 (3H, s, OMe), 4.58, 4.70 (1H, br m, OCH), 6.6-7.2 (10H, $C_6H_4$+ $C_6H_3$+pyridine $H_3$, $H_5$+C=CH), and 8.3-8.4 (2H, m, pyridine $H_2$,$H_6$); m/z (ESI) 388 ($M^+$+1, 100%).

## INTERMEDIATE 27

### (4-Bromophenyl)(3-cyclopentyloxy-4-methoxyphenyl)ketone

A solution of Intermediate 4 (8.00g, 29.5mmol) in THF (50ml) at -70°C was treated with n-BuLi (19.4ml, 31.0mmol, 1.6M solution in hexanes). The slightly yellow solution was stirred at -70°C for 0.5h then a solution of 4-bromobenzaldehyde (5.46g, 29.5mmol) in THF (50ml) was added *via* cannula. The reaction was allowed to warm to RT over 2h then quenched with water (25ml) and extracted with $Et_2O$ (2x50ml). The extract was dried ($MgSO_4$) and concentrated *in vacuo* to give a pale yellow oil which was dissolved in $CH_2Cl_2$ (150ml) and treated with manganese dioxide (19.24g, 0.22mol). The mixture was stirred vigorously for 20h at RT then filtered through Celite® and the residue washed with $CH_2Cl_2$ (5x50ml). The filtrate was concentrated *in vacuo* to give an off-white solid which was triturated with hexane to give the title compound (7.50g) as a white solid; $\delta_H$ ($CDCl_3$) 1.55-2.05 (8H, m, $(CH_2)_4$), 3.92 (3H, s, OMe), 4.83 (1H, m, OCH), 6.89 (1H, d, J 8.4Hz, ArH ortho to OMe), 7.33 (1H, dd, J 8.4, 2.0Hz, ArH para to OMe), 7.42 (1H, d, J 2.0Hz, ArH ortho to cyclopentyloxy), and 7.55-7.7 (4H, m, $C_6H_4$); $\nu_{max.}$ ($CDCl_3$) 2248, 1652, 1590, and 1270 $cm^{-1}$; m/z (ESI) 399 ($M^+$ +2+Na, 100%), 397 ($M^+$+Na, 90), 296 (16), and 236 (10).

## INTERMEDIATE 28

### a) (E) and (Z) isomers of 4-[2-(4-Bromophenyl)-2-(3-cyclopentyloxy-4-methoxyphenyl)ethenyl]pyridine

A solution of the compound of Example 1d (7.52g, 16.0mmol) and triethylamine (4.05g, 5.60ml, 40.0mmol) in $CH_2Cl_2$ (100ml) was cooled to 0°C and trifluoroacetic anhydride (3.70g, 2.50ml, 17.6mmol) was added dropwise. The orange-red solution was allowed to warm to RT over 20h then water (25ml) was added. The mixture was extracted with $CH_2Cl_2$ and the extract was dried ($MgSO_4$), concentrated *in vacuo* and subjected to chromatography to give the title compound (4.73g) as a white amorphous powder. (Found: C, 66.66; H, 5.27; N, 2.99. $C_{25}H_{24}BrNO_2$ requires C, 66.67; H, 5.37; N, 3.11%); $\delta_H$ ($CDCl_3$) 1.45-1.95 (8H, br, m, $(CH_2)_4$), 3.86, 3.88 (3H, s, OMe), 4.55, 4.70 (1H, br m, OCH), 6.6-6.95 (6H, m, $C_6H_3$, + pyridine $H_3$, $H_5$) + C=CH), 7.06, 7.21 (2H, d, J 8.4Hz, ArH of $C_6H_4$), 7.4-7.5 (2H, m, ArH of $C_6H_4$), and 8.36 (2H, ca. d, J 6.0Hz, pyridine $H_2$, $H_6$) ('H n.m.r. indicates a 1:1 E/Z mixture); $\nu_{max}$ ($CDCl_3$) 1597, 1514, and 1251 $cm^{-1}$; m/z (ESI) 452 ($M^+$ + 2 + Na, 100%), 450 ($M^+$ +Na, 88), 384 (30) and 382 (28).

The following intermediates were prepared in a manner similar to Intermediate 28a.

### b) (E) and (Z) isomers of 4-{2-(3-Cyclopentyloxy-4-methoxyphenyl)-2-[4-(4,4-dimethyl-2-oxazolinyl)phenyl] ethenyl}pyridine

From Intermediate 40 (4.75g, 9.8mmol), trifluoroacetic anhydride (2.47g, 1.66ml, 11.8mmol) and triethylamine (0.99g, 1.36ml, 11.8mmol). A portion of the residue (100mg) was subjected to chromatography ($SiO_2$; EtOAc) to give the title compound (68mg) as a yellow foam. $\delta_H$ ($CDCl_3$) 1.39, 1.41 (6H, s, $CMe_2$), 1.5-1.95 (8H, m, $(CH_2)_4$), 3.85, 3.88 (3H, s, OMe), 4.11, 4.14 (2H, s, oxazoline $CH_2$), 4.55, 4.69 (1H, m, OCH), 6.6-6.7 (1H, m, ArH), 6.8-6.85 (3H, m, ArH),

6.91 (1H, d, J 6.2Hz, pyridine H$_3$, H$_5$), 7.23, 7.38 (2H, d, J 8.2Hz, ArH), 7.9-8.0 (2H, m, ArH), and 8.3-8.45 (2H, m, pyridine H$_2$, H$_6$); $\nu_{max}$ ($CDCl_3$) 1735, 1646, 1597 and 1318 cm$^{-1}$; m/z (ESI) 469 ($M^+$, 100%).

c) **(Z)-4-[2-(3-Cyclopentyloxy-4-methoxyphenyl)-2-(2-furyl) ethenyl]pyridine**

From the compound of Example 34 (1.0g, 2.64mmol) in $CH_2Cl_2$ (30ml), triethylamine (0.4g, 0.55ml, 3.96mmol) and trifluoroacetic anhydride (0.61g, 0.41ml, 2.91mmol). Work up [includes treatment with 10% NaOH solution (25ml)] and chromatography ($SiO_2$; EtOAc/hexane, 7:3) afforded the title compound (0.78g) as a pale pink solid m.p. 122-123°C; (Found: C, 76.37; H, 6.46; N, 3.85. $C_{23}H_{43}NO_3$ requires C, 76.43; H, 6.41; N, 3.88%) ;$\delta_H$ ($CDCl_3$) 1.45-1.9 (8H, br m, $(CH_2)_4$), 3.90 (3H, s, OMe), 4.65 (1H, br m, OCH), 6.07 (1H, d, J 3.3Hz, furan H$_3$), 6.41 (1H, dd, J 3.3, 1.8Hz, furan H$_4$), 6.75-6.9 (5H, m, $C_6H_3$ + pyridine H$_3$, H$_5$), 7.03 (1H, s, C=CH), 7.49 (1H, d, J 1.6Hz, furan H$_5$), and 8.33 (2H, ca. d, J 4.6Hz, pyridine H$_2$, H$_6$); m/z (ESI) 362 ($M^+$ +1, 100%), 294 (45).

**INTERMEDIATE 29**

**[4-(4,4-dimethyl-2-oxazolinyl)phenyl-3'-cyclopentyloxy-4'-methoxyphenyl)ketone**

A solution of 2-(4-bromophenyl)-4,4-dimethyloxazoline (A. J. Meyers, D. L. Temple, D. Haidukewych and E. D. Milhelich J. Org. Chem, 39, 2787, 1974) (53.25g, 0.21mol) in THF (200ml) was added dropwise to magnesium turnings (6.0g, 0.25g atoms). The reaction was stirred for 2h at RT, then a solution of Intermediate 1 (46.0g, 0.21 mol) in THF (200ml) was added dropwise. The reaction was stirred for 16h then heated to reflux for 1 h, cooled to RT and quenched with $NH_4Cl$ solution (200ml). The layers were separated and the aqueous layer extracted with EtOAc (2x250ml). The organic layer was washed with brine (250ml), dried ($MgSO_4$), then concentrated *in vacuo* to give an orange oil. The crude oil was dissolved in $CH_2Cl_2$ (350ml) and treated with manganese dioxide (137g, 1.58mol) then stirred vigorously for 72h. The mixture was filtered through Celite® and the residue washed with $CH_2Cl_2$ (300ml). The filtrate was concentrated *in vacuo* and the residue triturated with $Et_2O$ to give the title compound (59.4g) as an off white amorphous powder m.p. 159°C. $\delta_H$ ($CDCl_3$) 1.41 (6H, s, CMe$_2$), 1.5-2.1 (8H, m, $(CH_2)_4$), 3.92 (3H, s, OMe), 4.15 (2H, s, oxazoline CH$_2$), 4.84 (1H, m, OCH), 6.89 (1H, d, J 8.4HZ, ArH ortho to OMe), 7.35 (1 H, dd, J 2.0, 8.4 Hz, ArH para to OMe), 7.43 (1 H, d, J 2.0 Hz, ArH ortho to cyclopentyloxy), 7.78 (2H, d, J 8.5Hz, ArH), and 8.03 (2H, d, J 8.5Hz, ArH); $\nu_{max}$ ($CDCl_3$)1648 and 1271 cm$^{-1}$; m/z (ESI) 394($M^+$+1, 100%).

**INTERMEDIATE 30**

**(E) and (Z) isomers of 4-[-1-(3-Cyclopentyloxy-4-methoxyphenyl)-2-(4-pyridyl)ethenyl]benzoic acid hydrochloride**

A solution of intermediate 28b (4.25gm 8.8mmol) in 10% aqueous HCl (15ml) was heated to reflux for 20 min. Aqueous NaOH solution (5M; 20ml) and EtOH (15ml) were then added and heating continued for a further 2h. The reaction was cooled to RT and acidified to pH 1 with 10% aqueous HCl. The mixture was extracted with $CHCl_3$ (10x100ml), the organic extract was dried ($MgSO_4$) and concentrated *in vacuo* to give the title compound (2.83g) as a yellow solid; $\delta_H$ ($d_4$-MeOH) 1.45-1.8 (8H, m, $(CH_2)_4$), 3.86, 3.88 (3H, s, OMe), 4.66, 4.74 (1H, br m, OCH), 6.65-7.65 (8H, m, C=CH + $C_6H_3$+ pyridine H$_3$, H$_5$+ ArH meta to $CO_2H$), 8.05, 8.13 (2H, d, J ca. 8Hz, ArH ortho to $CO_2H$), and 8.46, 8.55 (2H, d, J ca. 6Hz, pyridine H$_2$, H$_6$) (N.B. $CO_2H$ and HCl not observed); $\nu_{max}$ (Nujol) 1710, and 1633 cm$^{-1}$; m/z (ESI) 416 ($M^+$ + 1, 100%).

**INTERMEDIATE 31**

**Ethyl 3-(3-Cyclopentyloxy-4-methoxyphenyl)-3-phenyl-2-(4-pyridyl)**

**propanoate**

Phenyl magnesium bromide (3M in THF) (2.3ml, 6.8mmol) was added to a slurry of copper (I) chloride (54mg, 0.55mmol) in THF (20ml) at -70°C. The yellow turbid solution was stirred at -78°C for 0.25h, then Intermediate 13 (1.00g, 2.7mmol) in THF (10ml) was added via cannula. The reaction was stirred for 2h whilst allowing to warm to RT. The mixture was quenched with saturated $NH_4Cl$ solution (5ml) and water (20ml) then extracted with EtOAc (2x20ml). The extract was dried ($MgSO_4$), and concentrated *in vacuo* to give a yellow oil which was subjected to chromatography ($SiO_2$; EtOAc/hexane, 1:1) to give the title compound (0.29g) as a white solid m.p. 165-166°C. (Found: C, 75.48; H, 7.01; N, 3.14. $C_{28}H_{31}NO_4$ requires C, 75.76; H, 7.00; N, 3.14%); $\delta_H$ ($CDCl_3$) 1.03 (3H, t, J 7.1Hz, COCH$_2$Me), 1.5-2.0

(8H, br m, ($CH_2$)$_4$), 3.80 (3H, s, OMe), 3.9-4.0 (2H, complex m, $COCH_2CH_3$), 4.35 (1H, d, J 12.3Hz, CHCH), 4.57 (1H, d, J 12.3Hz, CHCH), 4.78 (1H, m, OCH), 6.75-7.1 (8H, m, aromatic $C_6H_5$ + $C_6H_3$), 7.22 (2H, dd, J 4.6, 1.6Hz pyridine $H_3$, $H_5$), and 8.41 (2H, dd, J 4.6, 1.6Hz, pyridine $H_2$, $H_6$). $\nu_{max}$ ($CDCl_3$) 1734, 1602, and 1516 $cm^{-1}$; m/z (ESI) 468 ($M^+$ + Na, 20%), 446 ($M^+$ +1, 20), 282 (22), 281 (100), and 213 (12).

## INTERMEDIATE 32

### (E) and (Z) isomers of -t-Butyl N-{4-[1-(3-cyclopentyloxy-4-methoxyphenyl)-2-(4-pyridyl)ethenyl]phenyl} carbamate

A mixture of diphenyl phosphoryl azide (0.61g, 0.48ml, 2.2mmol), Intermediate 29 (1.00g, 2.2mmol), triethylamine (0.49, 0.68ml, 4.9mmol) and t-butanol (25ml) was heated to reflux for 20h. The mixture was concentrated *in vacuo* and the resulting brown oil partitioned between $CH_2Cl_2$ (30ml) and 5% citric acid solution (30ml). The organic layer was separated, washed with water (20ml), $NaHCO_3$ solution (20ml), and brine (20ml), then dried ($MgSO_4$) and concentrated *in vacuo* to give a red oil; which was subjected to chromatography ($SiO_2$; 5% MeOH/$CH_2Cl_2$) to give the title compound (0.60g) as a yellow foamy solid. $\delta_H$ ($CDCl_3$) 1.52, 1.54 (9H, s, $CMe_3$), 1.65-1.9 (8H, br m, ($CH_2$)$_4$), 3.86, 3.89 (3H, s, OMe), 4.56, 4.70 (1H, m, OCH), 6.6-7.4 (11H, m, ArH + C=CH+NCO), and 8.34 (2H, d, J 5.2Hz, pyridine $H_2$, $H_6$). ('H n.m.r. indicates ca. 1:1 mixture of isomers); $\nu_{max}$ ($CHCl_3$) 3441, 1730, 1596, 1518 and 1157 $cm^{-1}$; m/z (ESI) 487 ($M^+$ +1, 75%), 472 (12), and 431 (100).

## INTERMEDIATE 33

### (S*,R*) and (S*,S*) Ethyl 3-(3-Cyclopentyloxy-4-methoxyphenyl)-2-(4-pyridyl)-3-(thienyl)propanate

A solution of 2-bromothiophene (0.49g, 3.0mmol) in $Et_2O$ (5ml) was added to Mg (0.08g, 3.3mmol) in $Et_2O$ (2ml) at RT. The mixture was stirred at RT for 0.3h then heated to reflux for 0.25h before adding a solution of Intermediate 13 (1.0g, 2.72mmol) in $Et_2O$-toluene (2:1; 15ml) dropwise over 10min at RT. The reaction mixture was stirred at RT for 18h then quenched with 10% $NH_4Cl$ solution (60ml) and extracted with EtOAc (3x50ml). The extract was washed with brine (80ml), dried ($MgSO_4$), and concentrated *in vacuo.* The residual brown oil was subjected to chromatography ($SiO_2$; $Et_2O$/hexane, 9:1 to $Et_2O$) to afford

(i) Intermediate 13 (205mg) and
(ii) title compound (132mg) after recrystallisation from $Et_2O$-hexane, 1:1) as a white solid m.p. 124-126°C; $\delta_H$ ($CDCl_3$) 0.99 (3H, t, J 7.1 Hz, $OCH_2Me$), 1.55-2.0 (8H, br m, ($CH_2$)$_4$), 3.82 (3H, s, OMe), 3.85-4.05 (2H, m, $OCH_2Me$), 4.26 (1H, d, J 11.9Hz, $CHCHCO_2Et$), 4.81 (1H, d, J 11.9Hz, $CHCHCO_2Et$), 4,85 (1H, br m, OCH), 6.51 (1H, d, J 3.5Hz, thiophene $H_3$), 6.69 (1H, dd, J 5.1, 3.5Hz, thiophene $H_4$), 6.81 (1H, d, J 8.8Hz, ArH ortho to OMe), 6.95-7.0 (3H, m, thiophene $H_5$ + 2xArH meta to OMe), 7.30 (2H, ca d, J 4.6Hz, pyridine $H_3$, $H_5$), and 8.48 (2H, ca. d, J 4.6Hz, pyridine $H_2$, $H_6$); m/z (ESI) 474 ($M^+$ +Na, 28%), 452 ($M^+$+1, 12), 368 (25), 289 (12), 288 (38), 287 (100), and 219 (22).

## INTERMEDIATE 34

a) **(E) and (Z) isomers of 2-Cyclopentyloxy-4-[2-(4-fluorophenyl)-1-phenylethenyl]anisole**

Dimethyl 4-fluorobenzylphosphonate (432mg, 2.0ml) was aded to a mixture of sodium hydride (80mg, 2.2mmol) and Intermediate 2 (592mg, 2.0mmol) in THF (5ml) at 0°C. The reaction mixture was stirred overnight at RT then quenched with $NaHCO_3$ solution (5ml) and extracted with $Et_2O$ (25ml). The extract was washed with brine (10ml), dried ($MgSO_4$), and concentrated *in vacuo* to afford the title compound (115mg) as a colourless gum; $\delta_H$ ($CDCl_3$) 1.5-1.9 (8H, br m, ($CH_2$)$_4$), 3.84, 3.86 (3H, s, OMe), 4.56,4.67 (1H, br m, OCH), and 6.65-7.4 (13H, m, $C_6H_3$ + $C_6H_4$ + $C_6H_5$ + C=CH); m/z (ESI) 390 ($M^+$ + 2, 23%), 389 ($M^+$+1, 92), 253 (37), and 235 (100).

b) **(E) and (Z) isomers of 4-[2-(4-chlorophenyl)-1-phenylethenyl]-2-cyclopentyloxyanisole**

From Intermediate 2 and diethyl 4-chlorobenzylphosphonate to afford the title compound as a clear oil. $\delta_H$ ($CDCl_3$) 1.5-2.0 (8H, br m, ($CH_2$)$_4$), 3.86, 3.89 (3H, s, OMe), 4.57,4.70 (1H, br m, OCH), and 6.65-7.4 (13H, m, $C_6H_3$ + $C_6H_4$ + $C_6H_5$ + C=CH) ('Hn.m.r. indicates ca 1:1 E:Z ratio); m/z (ESI) 429 ($M^+$ + 2 + Na, 15%), 427 ($M^+$+Na, 45), 387 (30), 386 (100), 301 (25), and 60 (20).

## INTERMEDIATE 35

### 3-Cyclopentyloxy-4-methoxybenzonitrile

Intermediate 1 (46.5h, 0.211mol) and hydroxylamine hydrochloride (17.60g, 0.25mol) was stirred in pyridine (250ml) overnight at RT. The reaction mixture was concentrated *in vacuo*, the residue dissolved in formic acid (100ml) and heated to reflux for 0.75h. The cooled reaction mixture was carefully poured into cold 10% NaOH solution, extracted with $Et_2O$ (1x500ml, 2x100ml), the extract washed with hydrochloric acid (10%. 2x150ml), sodium hydroxide (10%; 2x150ml), and brine (100ml), then dried ($Na_2SO_4$), and concentrated *in vacuo.* Chromatography ($SiO_2$; $Et_2O$/hexane, 1:2) afforded the title compound (39.55g) as a white solid (Found: C, 71.85; H, 7.02; N, 6.42. $C_{13}H_{15}NO_2$ requires C, 71.87; H, 6.96; N, 6.45%); $\delta_H$ ($CDCl_3$) 1.5-2.1 (8H, br m, ($CH_2$)$_4$), 3.85 (3H, s, OMe), 4.73 (1H, br m, OCHCH$_2$), 6.83 (1H, d, J 8.4Hz, ArH ortho to OMe), 7.03 (1H, d, J 1.6Hz, ArH ortho to cyclopentyloxy) and 7.19 (1H, dd, J 8.4, 1.6Hz, ArH para to cyclopentyloxy); m/z 217 ($M^+$, 14%), 150 (60), 149 (100), 134 (86), 106 (24), 77 (11), 69 (28), and 41 (69).

## INTERMEDIATE 36

### (3-Cyclopentyloxy-4-methoxyhenyl)(3-methoxyphenyl)ketone

n-BuLi (1.6M solution in hexanes; 6.7ml, 10.7mmol) was added dropwise to Intermediate 4 (2.71g, 10mmol) in THF (50ml) at -70°C. After 0.5h, 3-methylbenzonitrile (1.17g, 10mmol) in THF (25ml) was added dropwise and the mixture allowed to warm to RT. The reaction mixture was poured into saturated $NaHCO_3$ solution (25ml) extracted with $CH_2Cl_2$ (2x25ml), the extract dried ($Na_2SO_4$), concentrated *in vacuo* and the residue dissolved in dioxane (30ml) and 10% hydrochloric acid (20ml) then heated to reflux for 3h. The cooled reaction mixture was poured into brine (25ml) and extracted with $CH_2Cl_2$ (2x 25ml). The extract was dried ($Na_2SO_4$), concentrated *in vacuo*, and the residue subjected to chromatography ($SiO_2$; $Et_2O$/hexane, 1:1) to afford the title compound (2.47g); $\delta_H$ (80MHz; $CDCl_3$) 1.45-2.05 (8H, br m, ($CH_2$)$_4$), 2.42 (3H, s, ArMe), 3.70 (3H, s, OMe), 4.83 (1H, br m, OCH), 6.89 (1H, d, J 8.3Hz, ArH ortho to OMe), 7.3-7.4 (2H, m, ArH meta and para to C=O of $C_6H_3$), 7.37 (1H, dd, J 8.3, 2.0Hz, ArH para to OMe), 7.45 (1H, d, J 2.0Hz, ArH ortho to cyclopentyloxy), and 7.5-7.6 (2H, m, ArH ortho to C=O of $C_6H_4$).

## INTERMEDIATE 37

### (-)-(E)-(4S)-3-[3-(3-Cyclopentyloxy-4-methoxyphenyl)propenoyl]-4-phenyl-2-oxazolone

A mixture of Intermediate 1 (10.0g, 45.4mmol) and malonic acid (9.45g, 90.8mmol, 2equiv) in pyridine (20ml) was stirred at 50°C until a clear solution was obtained. Piperidine (0.68ml) was added and the mixture gradually heated to 80°C over 0.5h ($CO_2$ evolution commences) then at ca. 80°C over 1.5h and finally heated to reflux for 0.75h. The cooled reaction mixture was poured into cold water (250ml) and acidified, with stirring and cooling, with concentrated HCl (30ml). The precipitate was collected by filtration, washed with water (6x10ml), and dried *in vacuo* to afford (E)-3-(3-cyclopentyloxy-4-methoxyphenyl)propenoic acid (11.3g) as a white solid; $\delta_H$ ($CDCl_3$) 1.6-2.1 (8H, br m, ($CH_2$)$_4$), 3.88 (3H, s, OMe), 4.80 (1H, br m, OCH), 6.30 (1H, d, J 15.9Hz, HC=CHCO$_2$H), 6.87 (1H, d, J 8.5Hz, ArH ortho to OMe), 7.05-7.2 (2H, m, 2xArH meta to OMe), and 7.72 (1H, d, J 15.9Hz, HC=CHCO$_2$H) (N.B. CO$_2$H not observed).

A mixture of the acid (8.03g, 30.6mmol) in thionyl chloride (10.9g, 6.7ml, 91.9mmol, 3.0equiv) and $CH_2Cl_2$ (30ml) was heated to reflux for 1h. The reaction mixture was concentrated *in vacuo* and the residue diluted with toluene (30ml) and concentrated *in vacuo* again to afford (E)-3-(3-cyclopentyloxy-4-methoxyphenyl)propenoyl chloride (8.6g) as a dark crystalline solid; $\delta_H$ ($CDCl_3$) 1.5-2.1 (8H, br m, ($CH_2$)$_4$), 3.90 (3H, s, OMe), 4.81 (1H, br m, OCH), 6.47 (1H, d, J 15.4Hz, HC=CHCOCl), 6.88 (1H, d, J 8.5Hz, ArH ortho to OMe), 7.06 (1H, d, J 2.0Hz, ArH ortho to cyclopentyloxy), 7.14 (1H, dd, J 8.5, 2.0Hz, ArH para to cyclopentyloxy), and 7.76 (1 H, d, J 15.4Hz, HC=CHCOCl).

n-Butyllithium (1.6M solution in hexanes; 17.4ml, 27.8mmol) was added dropwise to a stirred soution of 4S-phenyloxazolidin-2-one (4.54g, 27.8mmol) in THF (140ml) at -70°C. After 0.25h at -70°C, a cold solution of the acid chloride (8.60g, 3.06mmol, 1.1equiv) in THF (40ml) was added to the resulting white slurry. The reaction mixture was stirred at -70°C for 0.5h then at 0°C for 1.5h. Saturated $NaHCO_3$ solution (100ml) and water (50ml) was added and the mixture extracted with EtOAc (100ml, 2x75ml). The extract was washed with saturated $NaHCO_3$ solution (50ml), brine (50ml), then dried ($Na_2SO_4$), and concentrated *in vacuo.* The residual solid (11.9g) was triturated with hot diisopropyl ether (100ml), cooled, and filtered to afford the title compound (10.9g) as white needles m.p. 107-109°C (from EtOAc/hexane, 1:1) (Found: C, 70.71; H, 6.09; N, 3.41. $C_{24}H_{25}NO_5$ requires C, 70.75; H, 6.19; N, 3.44%); $\delta_H$ ($CDCl_3$) 1.5-2.0 (8H, br m, ($CH_2$)$_4$), 3.86 (3H, s, OMe), 4.29 (1H, dd, J 8.7, 3.8Hz, OCHH'), 4.71 (1H, apparent t, J 8.7Hz, OCHH'), 4.81 (1H, m, ArOCH), 5.55 (1H, dd, J 8.7, 3.8Hz, CHN), 6.84 (1H, d, J 8Hz, ArH ortho to OMe), 7.1-7.5 (2H, m, ArH meta to OMe), 7.25-7.45 (5H, m, $C_6H_5$), 7.70 (1H, d, J 15.6Hz, HC=CH), and 7.80 (1H, d, J 15.6Hz, HC=CH,

$[\alpha]^{22}$ = -42° (0.150g/100ml EtOH).

**INTERMEDIATE 38**

**3-(3-Cyclopentyloxy-4-methoxyphenyl)-3-phenyl-1-propanol**

To a stirred solution of Intermediate 18 (9.0g, 2.65mmol) in THF (30ml) cooled to 0°C was added borane-THF complex (1.0M in THF; 3.41g, 162ml) and the reaction temperature kept below 20°C for 3h. The reaction mixture was warmed to RT, water (60ml) was added carefully, then 10% aqueous NaOH (100ml) and the stirring maintained for 0.5h. $Et_2O$ (50ml) was added, the organic layer separated, washed with water (1x30ml) and brine (1x30ml) then dried ($Na_2SO_4$). Concentration *in vacuo* afforded the title compound (9.16g) as a pale yellow oil.

**INTERMEDIATE 39**

**3-(3-Cyclopentyloxy-4-methoxyphenyl)-3-phenyl-1-propanal**

To Intermediate 38 (0.100g, 0.31mmol) in $CH_2Cl_2$ (10ml) was added 4Å molecular sieves (1 spatula) and the mixture degassed before adding pyridinium chlorochromate (0.099g, 0.47mmol). Stirring was maintained for 4h at RT and the mixture cooled to 0°C before adding $Et_2O$ (30ml). Filtration through a pad of florisil followed by concentration *in vacuo* afforded the title compound (0.98g) as a brown oil.

Examples 1, 2, 7 and 34 below are included for reference purposes

**EXAMPLE 1**

a) **(±)-4-[2-(3-Cyclopentyloxy-4-methoxyphenyl)-2-hydroxy-2-phenylethyl] pyridine**

n-Buli (1.4M in hexanes; 2.7ml, 3.7mmol) was added dropwise at -70°C to a solution of 4-methylpyridine (0.35g, 3.72mmol) in THF (20ml). After 0.5h, a solution of Intermediate 2 (1.00g, 3.38mmol) in THF (4ml) was added over 5 min at -70°C, the mixture stirred for 1h at this temperature then allowed to warm to RT over 2h. The reaction mixture was partitioned between $Et_2O$ (50ml) and water (50ml) and the organic layer was separated. The aqueous layer was further extracted with $Et_2O$ (2x40ml) and the combined organic extract was dried ($MgSO_4$) and concentrated *in vacuo.* The residue was subjected to chromatography ($SiO_2$; EtOAc-hexane) to afford, first, Intermediate 2 (300mg) then the title compound (738mg) as a white solid. m.p. 148-149°C (toluene-hexane) (Found : C, 77.32; H, 7.04; N, 3.50. $C_{25}H_{27}O_3$ requires C, 77.09; H, 6.99; N, 3.60%); $\delta_H$ ($CDCl_3$) 1.4-1.9 (8H, br, m, $(CH_2)_4$), 2.3 (1H, v.br.s, OH exchanges with $D_2O$), 3.51 (2H, s, $CH_2$ pyridine), 3.78 (3H, s, OMe), 4.60 (1H, br, m, $OCHCH_2$), 6.65-6.9 (5H, m) and 7.15-7.4 (5H, m) (ArH ortho to OMe + 2xArH meta to OMe + $C_6H_5$+ pyridine $H_3$, $H_5$), and 8.22 (2H, dm, J 4.5Hz, pyridine $H_2$, $H_6$); m/z 389 ($M^+$ 3%), 298 (15), 297 (69), 229 (27), 228 (37), 151 (43), 105 (100), 93 (52), 77 (24), and 41 (14).

The following compounds were prepared in a manner similar to the compound of Example 1a.

b) **(±)-2-[2-(3-Cyclopentyloxy-4-methoxyphenyl)-2-hydroxy-2-phenylethyl]pyrazine**

From 2-methylpyrazine (1.0ml, 110mmol) and Intermediate 2 (3.24g, 11.0mmol). Trituration with $Et_2O$ gave the title compound (0.885g) as a white solid. $\delta_H$ ($CDCl_3$) 1.45-1.9 (8H, br, m, $(CH_2)_4$), 3.73 (2H, s, $CH_2$ pyrazine), 3.80 (3H, s, OMe), 4.68 (1H, br, m, OCH), 6.22 (1H, br s, OH), 6.73 (1H, d, J 8.4 Hz, ArH ortho to OMe), 6.89 (1H, dd, J 8.4, 2.0Hz, ArH para to cyclopentyloxy), 7.0 (1H, d, J 2.0Hz, ArH ortho to cyclopentyloxy, 7.1-7.5 (5H, m, $C_6H_5$), and 8.37 (3H, s, pyrazine $H_3$, $H_5$ $H_6$).

c) **(±)-4-[2-(3-Cyclopentyloxy-4-methoxyphenyl)-2-hydroxyphenylethyl]-3,5-dichloropyridine**

From Intermediate 15 (2.0g, 12.3mmol) and Intermediate 2 (3.65g, 12.3mmol). Purification by column chromatography ($SiO_2$;0-2% MeOH/ $CH_2Cl_2$) afforded the title compound (1.74g) as a white solid. m.p. 129-130°C. $\delta_H$ ($CDCl_3$) 1.5-1.9 (8H, br, m, $(CH_2)_4$), 2.65 (1H, br s, OH), 3.85 (3H, s, OMe), 3.92 (1H, d, J 14Hz, $CH_AH_B$ pyridine), 3.98 (1H, d, J 14 Hz, $CH_AH_B$ pyridine), 4.57 (1H, br, m, OCH), 6.7-6.9 (3H, m, ArH ortho + 2x ArH meta to OMe), 7.2-7.4 (5H, m, $C_6H_5$), and 8.36 (2H, s, pyridine $H_2$, $H_6$).

d) **4-[2-(4-Bromophenyl)-2-(3-cyclopentyloxy-4-methoxyphenyl)-2-hydroxyethyl]pyridine**

From 4-picoline (2.0ml, 1.90g, 20.4mmol) and Intermediate 26 (7.30g, 19.5mmol). Purification by column

chromatography ($SiO_2$; gradient elution 50-75%, EtOAc/hexane) gave the title compound (7.77g) as a pale yellow foamy solid. Found: C, 63.82; H, 5.58; N, 2.96. $C_{25}H_{26}BrNO_3$ requires C, 64.11; H, 5.60; N, 2.99%. $\delta_H$ ($CDCl_3$) 1.5-1.9 (8H, br, m, $(CH_2)_4$), 2.7 (1H, br s, OH), 3.46 (1H, d, J 13.1Hz, $CH_AH_B$ pyridine), 3.54 (1H, d, J 13.1 Hz, $CH_AH_B$ pyridine), 3.82 (3H, s, OMe), 4.64 (1H, br m, OCH), 6.75-6.9 (5H, m, $C_6H_3$ + pyridine $H_3$, $H_5$), 7.21 (2H, ca. d, J 8.7Hz, ArH of $C_6H_4$), and 8.29 (2H, ca. d, J 6.0Hz, pyridine $H_2$, $H_6$); $\nu_{max.}$ ($CDCl_3$) 3604, 1605, 1513, and 1256 $cm^{-1}$; m/z (ESI) 470 ($M^+$ +2, 20%), 468 ($M^+$, 18), 377 (52), 375 (55), 95 (13), and 94 (100).

**INTERMEDIATE 40**

**(±)-4-{2-(3-Cyclopentyloxy-4-methoxyphenyl)-2-[4-(4,4-dimethyl-2-oxazolinyl)phenyl]-2-hydroxyethyl}pyridine**

From 4-methylpyridine (1.45g, 1.52ml), 15.6mmol) and Intermediate 29 (5.82g, 14.9mmol). Trituration with $Et_2O$ gave the title comound (6.61g) as an off-white solid. $\delta_H$ ($CDCl_3$) 1.37 (6H, s, CMe), 1.55-1.8 (8H, m, $(CH_2)_4$), 2.7 (1H, br s, OH), 3.56 (2H, br s, $CH_2$ pyridine), 3.82 (3H, s, OMe), 4.10 (2H, s, oxazoline $CH_2$), 4.63 (1H, m, OCH), 6.75-6.9 (5H, m, ArH), 7.37 (2H, d, J 8.6Hz, pyridine $H_3$, $H_5$), 7.85 (2H, d, J 7.3Hz ArH ortho to oxazoline) and 8.29 (2H, br s, pyridine $H_2$, $H_6$); $\nu_{max.}$ ($CDCl_3$) 3603, 1649, 1512, and 1257 $cm^{-1}$; m/z (ESI) 487 ($M^+$ +1, 100%), and 394 (61).

**EXAMPLE 2**

a) **(±)-4-[1-(3-Cyclopentyloxy-4-methoxyphenyl)-1-hydroxy-2-(4-pyridyl)ethyl]pyridine**

n-BuLi (1.45M in hexanes; 5.1ml,7.41mmol) was added dropwise at -70°C to a solution of 4-methylpyridine (0.69g, 7.41mmol) in THF (20ml). After 0.5h a solution of Intermediate 5 (2.0g, 6.73mmol) in THF (10ml) was added dropwise over 5 min. The reaction mixture was stirred for 0.5h at -70°C then at RT for 0.5h. Water (50ml) was added and the mixture extracted with EtOAc (3x60ml). The extract was washed with brine (80ml), dried ($MgSO_4$), and concentrated *in vacuo*. The residue was subjected to chromatography ($SiO_2$; EtOAc to EtOAc/$CH_3OH$, 9:1) to afford the title compound (2.33g) as a white amorphous solid m.p. 99-103°C; $\delta_H$ ($CDCl_3$) 1.5-2.0 (9H, br, m, $(CH_2)_4$ + OH), 3.49 (2H, d, J 2.3 Hz, $CH_2$ COH), 4.65 (1H, br m, $OCHCH_2$), 6.7-6.9 (5H, m, ArH ortho to OMe+ 2xArH meta to OMe + pyridine $H_3$, $H_5$), 7.20 (2H, dd, J 4.6, 1.6 Hz, pyridine $H_3$, $H_5$), 8.22 (2H, dd, J 4.6, 1.6 Hz, pyridine $H_2$, $H_6$), and 8.40 (2H, dd, J 4.6, 1.6 Hz, pyridine $H_2$, $H_6$); m/z 390 (M+ 3%), 298 (21), 297 (14), 230 (21), 229 (91), 151 (100), 106 (22), 93 (27), 78 (12), and 41 (23).

The following compounds were prepared in a manner similar to the compound of Example 2a.

b) **(±)-4-[2-(3-Cyclopentyloxy-4-methoxyphenyl)-2-hydroxy-2-(3-methoxyphenyl)ethyl]pyridine**

From Intermediate 5d (1.95g, 6.0mmol) and 4-methypyridine (0.58ml, 6.0mmol). Chromatography ($Si_2O$; EtOAc) afforded the title compound as a yellow oil (2.16g). $\delta_H$ ($CDCl_3$) 1.45-1.9 (8H, br, m, $(CH_2)_4$), 2.4 (1H, br s, OH), 3.53 (2H, s, $CH_2$ pyridine), 3.74 (3H, s, OMe), 3.82 (3H, s, OMe), 4.64 (1H, br m, OCH), 6.7-7.0 (8H, m, $C_6H_3$ + pyridine $H_3$, $H_5$ + 3xArH of $C_6H_4$), 7.22 (1H, ca. t, J ca. 7.6Hz, ArH of $C_6H_4$), and 8.31 (2H, dd, J 4.4, 1.6 Hz, pyridine $H_2$, $H_6$).

c) **(±)-4-[2-(3-Cyclopentyloxy-4-methoxyphenyl)-2(hydroxy-2-(2-methoxyphenyl)ethyl]pyridine**

From Intermediate 2c (2.44g, 7.5mmol) and 4-methylpyridine (0.78ml, 8.0mmol). Chromatography ($SiO_2$; EtOAc) afforded the title compound (2.5g).

d) **(±)-4-[2-(3-Cyclopentyloxy-4-methoxyphenyl)-2-(hydroxy-2-(4-methylphenyl)ethyl]pyridine**

From Intermediate 5b (1.41g, 4.54mmol) and 4-methylpyridine (0.49ml, 5.0mmol). Chromatography ($SiO_2$; $Et_2O$) afforded the title compound as a gum (1.45g); $\delta_H$ ($CDCl_3$) 1.5-1.9 (8H, br, m, $(CH_2)_4$), 2.25 (1H, br s, OH), 2.33 (3H, S, ArMe), 3.53 (2H, s, $CH_2$ pyridine), 3.81 (3H, s, OMe), 4.63 (1H, br m, OCH), 6.7-6.85 (5H, m, $C_6H_3$ + pyridine $H_3$, $H_5$), 7.11 (2H, d, J 8.1Hz, ArH of $C_6H_4$), 7.24 (2H, d, J 8.1Hz, ArH of $C_6H_4$), and 8.32 (2H, ca. d, J 4.6 Hz, pyridine $H_2$, $H_6$).

e) **(±)-4-[2-(3-Cyclopentyloxy-4-methoxyphenyl)-2-hydroxy-(4-methoxyphenyl)ethyl]pyridine**

From Intermediate 5c (2.46, 7.55mmol) and 4-methylpyridine (0.81ml, 8.3mmol). Chromatography ($SiO_2$; EtOAc) afforded the title compound as a gum (2.21g).

f) **(±)-4-[2-(3-Cyclopentyloxy-4-methoxyphenyl)-2-hydroxy-2-(3-methylphenyl)ethyl]pyridine**

From Intermediate 2f (2.12g, 6.85mmol) and 4-methylpyridine (0.68ml, 7.0mmol). Chromatography ($SiO_2$; $Et_2O$) afforded the title compound (2.08g) gum; $\delta_H$ ($CDCl_3$) 1.5-1.9 (8H, br, m, $(CH_2)_4$), 2.31 (3H, s, ArMe), 3.53 (2H, s, $CH_2$ pyridine), 3.82 (3H, s, OMe), 4,64 (1H, br m, OCH), 6.75-6.9 (5H, m, $C_6H_3$ + pyridine $H_3$, $H_5$), 7.0-7.25 (4H, m, $C_6H_4$), and (2H, dd, J 4.5, 1.6Hz, pyridine $H_2$, $H_6$). (N.B. OH no observed).

**EXAMPLE 3**

a) **(±)-4-[2-(3-Cyclopentyloxy-4-methoxyphenyl)-2-phenylethyl] pyridine**

Intermediate 7a (3.0g, 8.09mmol) in THF (50ml) was treated with 10% Pd/C (about 500mg) and hydrogenated over 38h at RT. The reaction mixture was filtered through Celite® and the filtrate concentrated *in vacuo.* The residue was subjected to chromatography ($SiO_2$; EtOAc/hexane 1:1) to afford the title compound (1.87g) as a clear oil which slowly crystallised on standing (Found : C, 79.87; H, 7.26; N, 3.69. $C_{25}H_{27}NO_2$ requires C, 80.40; H, 7.29; N, 3.75%); $\delta_H$ ($CDCl_3$) 1.5-2.1 (8H, br, m, $(CH_2)_4$), 3.27 (2H, d, J 8.0Hz, $CH_2$ pyridine), 3.75 (3H, s, OMe), 4.12 (1H, t, J 8.0 Hz, $PhCHCH_2$), 4.61 (1H, br m, $OCHCH_2$), 6.5-6.7 (3H, m, ArH ortho to OMe + 2xArH meta to OMe), 6.87 (2H, dm, J 4.5Hz, pyridine $H_3$, $H_5$), 7.05-7.2 (5H, m, $C_6H_5$) and 8.32 (2H, dm, J 4.5Hz, pyridine $H_2$, $H_6$); m/z 373 ($M^+$ 7%), 281 (38), 214 (16), 213 (100), 181 (10), and 152 (11).
Treatment of the free base (1.08g, 2.90mmol) in $Et_2O$ (10ml) with ethereal HCl gave, after decantation, the title compound hydrochloride (1.182g) as a white solid. $\delta_H$ ($CDCl_3$) 1.5-1.7 (2H, br s, cyclopentyl H), 1.75-1.95 (6H, br s, cyclopentyl H),3.58 (2H, d, J 7.8Hz, $CH_2$ pyridine), 3.80 (3H, s, OMe), 4.18 (1H, t, J 7.8 Hz, $CHCH_2$ pyridine), 4.67 (1H, br m, OCH), 6.67 (2H, br m, ArH), 6.76 (1H, m, ArH), 7.1-7.35 (5H, m, $C_6H_5$), 7.45 (2H, d, J 6.5 Hz, pyridine $H_3$, $H_5$) and 8.50 (2H, d, J 6.5 Hz, pyridine $H_2$, $H_6$).

The following compounds were prepared in a similar manner to the compound of Example 3a.

b) **(±)-4-[2-(3-Cyclopentyloxy-4-methoxyphenyl)-2-phenylethyl] phenol**

From Intermediate 10a (0.46g, 1.19mmol) in $CH_3OH$ (40ml). Removal of the solvent *in vacuo* gave the title compound (0.45g) as a yellow oil; $\delta_H$ ($CDCl_3$) 1.4-1.9 (8H, br, m, $(CH_2)_4$), 3.20-3.23 (2H, m, $PhCHCH_2$), 3.70 (3H, s, OMe), 4.07 (1H, t, J 8.0 Hz, $PhCHCH_2$), 4.64 (1H, br m, OCH), 5.88 (1 H, br s, OH), 6.59 (2H, ca d, J 8.6 Hz, ArH ortho to OH), 6.65-6.75 (3H, m, $C_6H_3$), 6.81 (2H, ca d. J 8.6Hz, ArH meta to OH), and 7.1-7.25 (5H, m, $C_6H_5$); m/z (ESI) 411 ($M^+$ + Na, 100%), 215 (15), and 197 (50).

c) **(±)-4-[2-(3-Cyclopentyloxy-4-methoxyphenyl)-2-phenylethyl] anisole**

From Intermediate 10c (0.47g, 1.18mmol) in $CH_3OH$/dioxane (1:1, 50ml). Removal of the solvent *in vacuo* gave the title compound (0.45g) as a colourless oil; $\delta_H$ ($CDCl_3$) 1.5-1.9 (8H, br, m, $(CH_2)_4$), 3.24 (2H, ca d, J ca 8.0Hz, $PhCHCH_2$), 3.68 (3H, s, OMe), 3.74 (3H, s, OMe),4.09 (1H, t, J 8.0 Hz, $PhCHCH_2$), 4.63 (1H, br m, OCH), 6.65-6.75 (5H, m, $C_6H_3$+ 2xArH ortho to OMe), 6.89 (2H, ca d, J 8.5Hz,2xArH meta to OMe), and 7.1-7.25 (5H, m, $C_6H_5$); m/z (ESI) 426 ($M^+$ + 1+Na, 25%), 425 ($M^+$ +Na, 100), 279 (24), 236 (48), 211 (30), 183 (25), 151 (36), 119 (48), 87 (78), and 65 (25).

d) **(±)-4-[2-(3-Cyclopentyloxy-4-methoxyphenyl)-2-phenylethyl] acetoxybenzene**

From Intermediate 11 (0.14g, 0.33mmol) in $CH_3OH$/dioxane (1:1, 40ml). Removal of the solvent *in vacuo* gave the title compound (0.13g) as a colourless oil; $\delta_H$ ($CDCl_3$) 1.5-1.9 (8H, br, m, $(CH_2)_4$), 2.24 (3H, s, COMe), 3.30 (2H, d, J 7.7Hz, $PhCHCH_2$), 3.78 (3H, s, OMe), 4.11(1 H, t, J 7.7 Hz, $PhCHCH_2$), 4.65 (1H, br m, OCH), 6.65-6.8 (3H, m, $C_6H_3$), 6.88 (2H, d, J 8.5Hz, 2xArH of $C_6H_4$), 6.98 (2H, d, J 8.5Hz, 2xArH of $C_6H_4$), and 7.1-7.3 (5H, m, $C_6H_5$); m/z (ESI) 453 ($M^+$ + Na, 100%).

e) **(±)-2-[2-(3-Cyclopentyloxy-4-methoxyphenyl)-2-phenylethyl] pyrazine**

From Intermediate 7b (520mg) in THF/EtOH (12ml, 1:5). Purification by column chromatography ($SiO_2$; $Et_2O$) gave the title compound (114mg) as a white solid. m.p. 71.5-72°C. $\delta_H$ ($CDCl_3$) 1.4-1.9 (8H, br, m, $(CH_2)_4$), 3.50 (2H, d, J 8.0 Hz, $CH_2CH$), 3.78 (3H, s, OMe), 4.51 (1H, t, J 8.0Hz, $CHCH_2$), 4.66 (1H, br m, OCH), 6.7-6.75 (3H, m, ArH ortho to OMe + 2xArH meta to OMe ), 7.15-7.3 (5H, m, $C_6H_5$), 8.17 (1H, d, J 1.5Hz, pyrazine $H_2$), 8.31 (1H, d, J 2.5Hz, pyrazine $H_5$), and 8.47 (1H, m, pyrazine $H_6$).

f) **(±)-3-[2-(3-Cyclopentyloxy-4-methoxyphenyl)-2-phenylethyl]-2-methoxypyrazine**

From Intermediate 7c (2.67g, 6.6mmol) in THF/EtOH (21ml, 1:20). Purification by column chromatography ($SiO_2$; $CH_2Cl_2$) furnished the title compound (2.55g) as a colourless oil; $\delta_H$ ($CDCl_3$) 1.5-1.9 (8H, br m ($CH_2)_4$), 3.42-3.60 (2H, m, $CHCH_2$), 3.77 (3H, s, OMe), 3.89 (3H, s, OMe), 4.67 (1H, t, J 8.0 Hz, $CHCH_2$), 4.67 (1H, br m, OCH), 6.7-6.8 (3H, m, ArH ortho to OMe + 2 x ArH meta to OMe), 7.1-7.3 (5H, m, $C_6H_5$), 7.85 (1H, d, J 2.8Hz, pyrazine H), and 7.96 (1 H, d, J 2.8Hz, pyrazine H).

g) **(±) Methyl 4-[2-(3-Cyclopentyloxy-4-methoxyphenyl)-2-phenyl ethyl]benzoate**

From Intermediate 10 d (3.00g, 7.0mmol) in $CH_3OH$/THF (1:1, 100ml) to afford the title compound (2.87g) as a colourless gum; $\delta_H$ ($CDCl_3$) 1.5-1.9 (8H, br m ($CH_2)_4$), 3.34-3.37 (2H, m, $PhCHCH_2$), 3.78 (3H, s, OMe), 3.87 (3H, s, OMe), 4.15 (1H, t, J 8.0 Hz, $PhCHCH_2$), 4.63 (1H, br m, OCH), 6.65 (1H, dd, J 7.8, 2.0Hz, ArH para to cyclopentyloxy), 6.69 (1H, d, J 2.0Hz, ArH ortho to cyclopentyloxy), 6.73 (1H, d, J 7.8Hz, ArH ortho to OMe), 7.05 (2H, ca. d, J 8.5Hz, 2xArH meta to $CO_2Me$), 7.15-7.3 (5H, m, $C_6H_5$), and 7.83 (2H, ca. d, J 8.5Hz 2xArH ortho to $CO_2Me$); m/z (ESI) 454 ($M^+$+1+Na, 40%), 453 ($M^+$+Na,100), 301 (12), 239 (10), and 213 (17).

h) **(±) Methyl 3-[2-(3-Cyclopentyloxy-4-methoxyphenyl)-2-phenylethyl]benzoate**

From Intermediate 12 (140mg, 0.33mmol) in $CH_3OH$/THF (1:1, 20ml) to afford the title compound (137mg) as a colourless gum. $\delta_H$ ($CDCl_3$) 1.5-1.9 (8H, br m ($CH_2)_4$), 3.34-3.37 (2H, m, $PhCHCH_2$), 3.78 (3H, s, OMe), 3.88 (3H, s, OMe), 4.17 (1H, t, J 8.0 Hz, $PhCHCH_2$), 4.64 (1H, br m, OCH), 6.65-6.75 (3H, m, $C_6H_3$), 7.1-7.3 (7H, m, $C_6H_5$+2xArH meta and para to $CO_2Me$), and 7.75-7.85 (2H, m, 2xArH ortho to $CO_2Me$); m/z (ESI) 453 ($M^+$+Na, 100%).

i) **(±) -3-[2-(3-Cyclopentyloxy-4-methoxyphenyl)-2-phenylethyl] pyridazine**

From Intermediate 7e (1.87g). Purification by chromatography ($SiO_2$; $Et_2O$-EtOAc) afforded the title compound (0.91g) as a pale yellow oil; $\delta_H$ ($CDCl_3$) 1.5-1.9 (8H, br m, ($CH_2)_4$), 3.6-3.7 (2H, m, $CHCH_2$), 3.80 (3H, s, OMe), 4.55 (1H, t, J 8.0Hz, $CHCH_2$), 4.65 (1H, br m, OCH), 6.7-6.8 (3H, m, $C_6H_3$), 6.93 (1H, dd, J 8.5, 0.8Hz, pyridazine $H_4$), 7.1-7.3 (6H, m, $C_6H_5$+, pyridazine $H_5$), and 8.97 (1 H, dd, J 5.5, 0.8Hz, pyridazine $H_6$); m/z (ESI) 397 ($M^+$+23, 70%), 375 ($M^+$+1, 72), and 281 (100).

(j) **(±)-3-[2-(3-Cyclopentyloxy-4-methoxyphenyl)-2-phenylethyl] benzoic acid**

From Intermediate 10b (1.75g, 4.23mmol) in $CH_3OH$-THF (75ml, 2:1) to afford the title compound (1.56g) as a pale orange gum; $\delta_H$ ($CDCl_3$) 1.5-2.0 (8H, br m, ($CH_2)_4$), 3.2-3.6 (1H, v.br.s), 3.38 (2H, d, J 8.0Hz, $PhCHCH_2$), 3.79 (3H, s, OMe), 4.18 (1H, t, J 8.0Hz, $PhCHCH_2$), 4.65 (1H, br m, OCH), and 6.6-8.2 (12H, m, $C_6H_5$+$C_6H_4$+$C_6H_3$).

k) **(±)-2-[2-(3-Cyclopentyloxy-4-methoxyphenyl)-2-phenylethyl]-4-methylpyridine**

From Intermediate 7f (1.03g). Purification by chromatography ($SiO_2$; $Et_2O$) afforded the title compound (354mg) as a colourless oil; $\delta_H$ ($CDCl_3$) 1.5-1.9 (8H, br m, ($CH_2)_4$), 2.19 (3H, s, pyridine Me). 3.43 (2H, dd, J 8.2, 1.6Hz, $PhCHCH_2$), 3.78 (3H, s, OMe), 4.55 (1H, t, J 8.2Hz, $PhCHCH_2$), 4.65 (1H, br m, OCH), 6.7-6.75 (4H, m, $C_6H_3$+ pyridine $H_3$), 6.85-6.9 (1H, m, pyridine $H_5$), 7.1-7.3 (5H, m, $C_6H_5$), and 8.38 (1H, ca d, J 5.1Hz, pyridine $H_6$).

l) **(±)-4-[2-(3-Cyclopentyloxy-4-methoxyphenyl)-2-phenylethyl] pyrimidine**

From Intermediate 7g (1.10g). Purification by chromatography ($SiO_2$; $Et_2O$) afforded the title compound (299mg) as a colourless oil which slowly crystallised on standing (Found: C, 76.82; H, 6.85; N, 7.35. $C_{24}H_{26}N_2O_2$ requires C, 76.98; H, 7.00; N, 7.48%); $\delta_H$ ($CDCl_3$) 1.5-2.0 (8H, br m, ($CH_2)_4$), 3.45 (2H, d, J 8.0Hz, $CHCH_2$), 3.78 (3H, s, OMe), 4.52 (1H, t, J 8.0Hz, $CHCH_2$), 4.65 (1H, br m, OCH), 6.7-6.8 (3H, m, $C_6H_3$), 6.89(1H, dd, J 5.1, 1.2Hz, pyrimidine $H_5$), 7.15-7.4 (5H, m, $C_6H_5$), 8.44 (1H, d, J 5.1Hz, pyrimidine $H_6$), and 9.11 (1H, d, J 1.2Hz, pyrimidine $H_2$).

m) **2-Cyclopentyloxy-4-[2-(4-fluorophenyl)-1-phenylethyl]anisole**

From Intermediate 34a (65mg). Filtration through Celite® and concentration *in vacuo* afforded a colourless gum (62mg) which slowly solidified to give the title compound as a white solid; $\delta_H$ ($CDCl_3$) 1.5-1.95 (8H, br m, ($CH_2)_4$), 3.27 (2H, d, J 8.2Hz, $CHCH_2$ pyridine), 3.78 (3H, s, OMe), 4.08 (1H, t, J 8.2Hz, $CHCH_2$ pyridine), 4.64 (1H, br m, OCH),

6.6-6.75 (3H, m, $C_6H_3$), 6.8-7.0 (4H, m, $C_6H_4$), and 7.1-7.3 (5H, m, $C_6H_5$).

n) **(±)-4-[2-(4-Chlorophenyl)-1-phenylethyl]-2-cyclopentyloxyanisole**

From Intermediate 34b (200mg, 0.49mmol). Trituration with hexane afforded the title compound (45mg) as a white solid m.p. 63°C. $\delta_H$ ($CDCl_3$) 1.5-2.0 (8H, br m, $(CH_2)_4$), 3.2-3.3 (2H, m, $PhCHCH_2$), 3.78 (3H, s, OMe),4.08 (1H, t, J 7.8Hz, $PhCHCH_2$), 4.63 (1H, br m, OCH), 6.6-6.7 (2H, m, ArH meta to OMe), 6.73 (1H, d, J 8.2Hz, ArH ortho to OMe), 6.90 (2H, d, J 8.3Hz, 2xArH of $C_6H_4$), and 7.0-7.3 (7H, m, $C_6H_5$ + 2xArH of $C_6H_4$) (N.B.) ('Hn.m.r. indicates the presence of ca 10% of the des-chloro compound); m/z 431 ($M^+$+2 + Na, 40%),430 ($M^+$ + 1 + Na, 38), 429 ($M^+$+Na, 100), 396 (22), 395 (92), 301 (15), 236 (15), 213 (15), and 60 (27).

o) **(±)-3-[2-(3-Cyclopentyloxy-4-methoxyphenyl)-2-phenylethyl]-pyridine**

From Intermediate 10e (390mg, 1.05mmol). Chromatography ($SiO_2$; $Et_2O$) afforded the title compound (200mg) as a colourless oil; $\delta_H$ ($CDCl_3$) 1.5-2.0 (8H, br m, $(CH_2)_4$), 3.31 (2H, d, J 8.0Hz,$CHCH_2$ pyridine), 3.78 (3H, s, OMe), 4.10 (1H, t, J 8.0Hz $CHCH_2$ pyridine), 4.65 (1H, br m, OCH), 6.6-6.8 (3H, m, $C_6H_3$), 7.0-7.5 (7H, m, $C_6H_5$ + pyridine $H_4$, $H_5$), 9.30 (1H, d, J 1.0Hz, pyridine $H_2$), and 9.48 (1H, dd, J 3.0, 2.0Hz, pyridine $H_6$), m/z (ESI) 374 ($M^+$+1, 100%), 321 (20), and 306 (80).

## EXAMPLE 4

**(±)-4-[1-(3-Cyclopentyloxy-4-methoxyphenyl)-2-(4-pyridyl)ethyl] pyridine**

A solution of Intermediate 8 (0.20g, 0.54mmol) in EtOH (10ml) containing $Et_3N$ (0.5ml) was hydrogenated over 10% Pd/C (54mg) for 18h. The reaction mixture was filtered through Celite® and concentrated *in vacuo.* The residue was subjected to chromatography ($SiO_2$; EtOAc/$CH_3OH$, 19:1) to afford the title compound (170mg) as a colourless oil. $\delta_H$ ($CDCl_3$) 1.5-1.9 (8H, br, m, $(CH_2)_4$), 3.27 (2H, d, J 8.0 Hz, $CH_2$ pyridine), 3.77 (3H, s, OMe), 4.10 (1H, t, J 8.0 Hz, $CH_2CH$ pyridine), 4.62, (1H, br m, $OCHCH_2$), 6.5-6.8 (3H, m, ArH ortho to OMe+ 2xArH meta to OMe), 6.88 (2H, dd, J 4.5, 1.5 Hz, pyridine $H_3$, $H_5$), 7.06 (2H, dd, J 4.5, 1.5 Hz, pyridine $H_3$, $H_5$), 8.35 (2H, dd, J 4.5, 1.5 Hz, pyridine $H_2$, $H_6$), and 8.43 (2H, dd, J 4.5, 1.5 Hz, pyridine $H_2$, $H_6$); m/z 374 ($M^+$ 17%), 306 (32), 282 (12), 215 (16), 214 (100), 154 (11), 129 (14), 93 (12), 57 (15), and 41 (18).

Treatment of the title compound with ethereal solution furnished the title compound dihydrochloride. m.p. 230-233°C (dec).

## EXAMPLE 5

a) **(±)-4-[2-(3-Cyclopentyloxy-4-methoxyphenyl)-2-(4-fluorophenylethyl] pyridine hydrochloride**

To Intermediate 14 (2.19g, 4.72mmol) in EtOH(50ml) was added NaOH (1.0g, 25mmol) in water (20ml). The reaction mixture was heated to reflux until complete hydrolysis (about 1h) and the pH adjusted to pH 6 with concentrated hydrochloric acid (about 2ml). The reaction mixture was then heated to reflux until complete decarboxylation occurred (about 7h). Upon cooling, the yellow solution was half-concentrated and partitioned between 0.5N NaOH (100ml) and $Et_2O$ (100ml). The organic layer was washed with brine, dried ($MgSO_4$) and evaporated. The residual yellow tinged gum (1.81g) was taken up in $Et_2O$ (50ml) and 2.5M hydrochloric acid in EtOH (about 2ml) was added to pH2. The solvent was evaporated and the yellow foam obtained redissolved in EtOH (20ml). $Et_2O$ was added until the solution became slightly cloudy and the mixture cooled to 0°C to give an off-white solid. The mother liquor was decanted off, the solid washed with $Et_2O$ and dried *in vacuo* to give the title compound (1.85g) as an off-white solid. m.p. 147-150°C. $\delta_H$ ($CD_3OD$) 1.50-1.90 (8H, m, $(CH_2)_4$), 3.70 (2H, d, $CH_2Ar$), 3.75 (3H, s, $OCH_3$), 4.45 (1H, t, CHAr), 4.75, (1H, m, $OCHCH_2$), 6.80 (3H, m, Ar), 7.05 (2H, m, Ar), 7.35 (2H, m, Ar), 7.90 (2H, d, Ar), 8.65 (2H, d, Ar); m/z (ESI) 393 ($M^+$ +2, 12%), 392 ($M^+$ +1, 38), 300 (29), and 299 (100).

The following compound was prepared in a similar manner to the compound of Example 5a.

b) **(±)-4-[2-(3-Cyclopentyloxy-4-methoxyphenyl)-2-(4-trifluoromethylphenyl)ethyl]pyridine hydrochloride**

From intermediate 25 (2.05g, 3.99 mmol) and NaOH (0.80g, 20mmol) to afford the free base (1.70g) as a pale yellow gum; $\delta_H$ ($CDCl_3$) 1.5-1.9 (8H, br m, $(CH_2)_4$), 3.36 (2H, d, J 7.6, 0.8Hz, $CHCH_2$ pyridine), 3.80 (3H, s, OMe), 4.23 (1H, t, J 7.6Hz, $CHCH_2$ pyridine), 4.67 (1H, br m, OCH), 6.65 (1H, d, J 2.0Hz, ArH ortho to cyclopentyloxy), 6.70 (1H, dd, J 7.8, 2.0Hz, ArH para to OMe), 6.79 (1H, d, J 7.8Hz, ArH ortho to OMe), 6.94 (2H, d, J 5.2Hz, pyridine $H_3$,

$\underline{H}_5$), 7.30 (2H, d, J 8.3Hz, 2xArH meta to $CF_3$), 7.55 (2H, d, J 8.3Hz, 2xAr$\underline{H}$ ortho to $CF_3$), and 8.42 (2H, d, J 5.2Hz, pyridine $\underline{H}_2$,$\underline{H}_6$); m/z (ESI) 443 ($M^+$+2, 24%) 442 ($M^+$+1, 87), 350 (22), 349 (100), 281 (40), and 250 (30).

Treatment of the free base (1.65g) in $Et_2O$(50ml) with ethanolic HCl (2.5M), concentration *in vacuo* and recrystallisation ($EtOH$-$Et_2O$) afforded the title compound (1.66g) as an off-white solid m.p. 149-152°C; $\delta_H$ ($d_4$-MeOH) 1.55-1.95 (8H, br m, $(C\underline{H}_2)_4$), 3.77 (3H, s, O$\underline{Me}$), 3.78 (2H, d, J 7.8Hz, CHC$\underline{H}_2$ pyridine), 4.60 (1H, t, J 7.8Hz, C$\underline{H}CH_2$ pyridine), 4.75 (1H, br m, OC$\underline{H}$), 6.8-6.9 (3H, m, $C_6\underline{H}_3$), 7.5-7.65 (4H, m, $C_6\underline{H}_4$), 7.91 (2H, d, J 5.2Hz pyridine $\underline{H}_3$, $\underline{H}_5$), and 8.68 (2H, d, J 5.2Hz, pyridine $\underline{H}_2$,$\underline{H}_6$). (N.B. $\underline{H}$Cl not observed).

c) **(±)-4-[2-(3-Cyclopentyloxy-4-methoxyphenyl)-2-thienylethyl] pyridine hydrochloride**

From Intermediate 33 (566mg, 1.25mmol). Chromatography ($SiO_2$; EtOAc/hexane, 4:1) afforded the title compound tree base (350mg) as a colourless oil; $\delta_H$ ($CDCl_3$) 1.5-2.0 (8H, br m, $(C\underline{H}_2)_4$), 3.25 (1H, dd, J 13.5, ca. 8Hz, $CHC\underline{H}_AH_B$), 3.41 (1H, dd, J 13.5, ca. 7Hz, $CHCH_A\underline{H}_B$), 3.80 (3H, s, O$\underline{Me}$), 4.36 (1H, t, J ca. 8, ca. 7 Hz, C$\underline{H}CH_A$ $H_B$), 4.65 (1H, br m, OC$\underline{H}$), 6.65-6.85 (4H, m , $C_6\underline{H}_3$ + thiophene $\underline{H}_3$), 6.90 (1H, dd, J 5.1, 3.5Hz, thiophene $\underline{H}_4$), 6.94 (2H, dd, J 4.4, 1.6 Hz, pyridine $\underline{H}_3$, $\underline{H}_5$), 7.16, (1H, dd, J 5.1, 1.2Hz, thiophene $\underline{H}_5$), and 8.40 (2H, dd, J 4.4, 1.6Hz, pyridine $\underline{H}_2$,$\underline{H}_6$); m/z (ESI) 381 ($M^+$+2, 13%) 381 ($M^+$+1, 65), 288 (2), and 287 (100).

Treatment of the free base (270mg) in $Et_2O$ (15ml) with ethanolic HCl (2.5M). afforded the title compound (226 mg) as a pale yellow solid. $\delta_H$ ($CDCl_3$) 1.5-1.9 (8H, br m, $(C\underline{H}_2)_4$), 3.51 (1H, dd, J 13.5, 8.6Hz, $CHC\underline{H}_AH_B$), 3.64 (1H, dd, J 13.5, 7.2Hz, $CHCH_A\underline{H}_B$), 3.81 (3H, s, O$\underline{Me}$), 4.41 (1H, ca. t, J ca. 7.8Hz, C$\underline{H}CH_A$ $H_B$), 4.72 (1 H, br m, OC$\underline{H}$), 6.64 (1 H, dd, J 8.2, 2.0Hz, Ar$\underline{H}$ para to OMe), 6.7-6.8 (3H, m, 2x Ar$\underline{H}$ of $C_6\underline{H}_3$ + thiophene $\underline{H}_3$), 6.91 (1H, dd, J 5.1, 3.5Hz, thiophene $\underline{H}_4$), 7.19 (1H, dd, J 5.1, 1.0Hz, thiophene $\underline{H}_5$), 7.49 (2H, d, J 6.3Hz, pyridine $\underline{H}_3$, $\underline{H}_5$), and 8.55 (2H, d, J 6.3Hz, pyridine $\underline{H}_2$,$\underline{H}_6$).

**EXAMPLE 6**

**(+)-4-[2-(3-Cyclopentyloxy-4-methoxy)-2-phenylethyl]pyridine-N-oxide**

A solution of a compound of Example 16 (i) (264mg) in peracetic acid (0.5ml) and $CH_2Cl_2$ (50ml) was stirred at RT for 3h. Additional peracetic acid (0.5ml) was added and the mixture stirred overnight then treated with saturated aqueous sodium sulphite for 5 min. The organic phase was separated and combined with further $CH_2Cl_2$ extracts (2 x 30ml). The extract was washed with aqueous HCl (10%; 30ml), aqueous $NaHCO_3$ (2 x 30ml), brine (30ml), then dried ($MgSO_4$), and concentrated *in vacuo*. Purification by column chromatography ($SiO_2$; 1-5% $CH_3OH/CH_2Cl_2$) gave a colourless oil which was triturated with $Et_2O$-hexane to afford the title compound (260mg) as a white solid. m.p. 114-116°C. $\delta_H$ ($CDCl_3$) 1.5-1.9 (8H, br, m, $(C\underline{H}_2)_4$), 3.29 (2H, d, J 8 Hz, CHC$\underline{H}_2$), 3.80 (3H, s, O$\underline{Me}$), 4.06 (1H, t, J 8 Hz, C$\underline{H}CH_2$), 4.67, (1H, br m, OC$\underline{H}$), 6.65-6.8 (3H, m, Ar$\underline{H}$ ortho to OMe + 2xAr$\underline{H}$ meta to OMe ), 6.84 (2H, d, J 7 Hz, pyridine $\underline{H}_3$, $\underline{H}_5$), 7.1-7.35 (5H, m, $C_6\underline{H}_5$), and 8.00 (2H, d, J 7Hz, pyridine $\underline{H}_2$, $\underline{H}_6$). (Optical rotation at 0.153g/100ml of EtOH $[\alpha]_D^{22}$ = +43°).

**EXAMPLE 7**

a) **(±)-3-[2-(3-Cyclopentyloxy-4-methoxyphenyl)-2-hydroxy-2-phenylethyl]-2-methoxypyrazine**

n-BuLi (1.6M in hexanes; 6ml, 12mmol) was added dropwise at 4°C to a solution of N, N-diisopropylamine (1.85ml, 13mmol) in THF (40ml). After 0.5h, 2-methoxy-3-methylpyrazine (1.28ml, 11mmol) was added dropwise at -70°C and the mixture stirred for 2h at this temperature. A solution of Intermediate 2 (3.26g, 11mmol) in THF (20ml) was added over 10 min at -70°C and the mixture stirred for a further 1h and then allowed to warm to RT. The reaction mixture was partitioned between $CH_2Cl_2$ (75ml) and saturated $NaHCO_3$ (100ml). The organic layer was separated, combined with further $CH_2Cl_2$ extracts (2x75ml), dried ($MgSO_4$) and concentrated *in vacuo*. The residue was subjected to chromatography ($SiO_2$; $CH_2Cl_2$) to afford the title compound (2.94g) as a white foam. $\delta_H$ ($CDCl_3$) 1.5-2.0 (8H, br m, $(C\underline{H}_2)_4$), 3.63 (1H, d, J 14 Hz, C$\underline{H}$H pyrazine), 3.77 (1H, d, J 14Hz, CH$\underline{H}$ pyrazine), 3.79 (3H, s, O$\underline{Me}$ ortho to cyclopentyloxy), 3.97 (3H, s, pyrazine O$\underline{Me}$), 4.67 (1H, br m, OC$\underline{H}$), 6.72 (1H, dd, J 8.4Hz, Ar$\underline{H}$ ortho to OMe), 6.77 (1H, s, O$\underline{H}$), 6.91 (1H, dd, J 8.4Hz, 2.0Hz, Ar$\underline{H}$ para to cyclopentyloxy), 7.00 (1H, d, J 2.0Hz, Ar$\underline{H}$ ortho to cyclopentyloxy), 7.1-7.5 (5H, m, $C_6\underline{H}_5$), and 7.85-7.95 (2H, m, pyrazine $\underline{H}_5$, $\underline{H}_6$).

The following compounds were prepared in a manner similar to the compound of Example 7a.

b) **(±)-3-[2-(3-Cyclopentyloxy-4-methoxyphenyl)-2-hydroxy-2-phenylethyl]pyridazine**

From 3-methylpyridazine (1.0ml) and Intermediate 2 (3.98g). Purification by chromatography ($SiO_2$; EtOH-

$CH_2Cl_2$) afforded the title compound (4.02g) as an off-white solid.

c) **(±)-2[-2-(3-Cyclopentyloxy-4-methoxyphenyl)-2-hydroxy-2-phenylethyl]-4-methylpyridine**

From 2,4-dimethylpyridine (1.7ml, 14.5mmol) and Intermediate 2 (4.30g, 14.5mmol). Purification by chromatography ($SiO_2$; $CH_2Cl_2$) afforded the title compound (1.23g) as a colourless oil (Found: C, 77.07; H, 7.10; N, 3.25. $C_{26}H_{29}NO_3$ requires C, 77.39; H, 7.24; N, 3.47%); $\delta_H$ ($CDCl_3$) 1.4-1.9 (8H, br m, $(CH_2)_4$), 2.25 (3H, s, pyridine Me), 3.60 (2H, s, $CH_2$ pyridine), 3.77 (3H, s, OMe), 4.68 (1H, br m, OCH), 6.72 (1H, d, J 8.5Hz, ArH ortho to OMe), 6.8-6.95 (3H, m, ArH para to cyclopentyloxy + pyridine $H_3$, $H_5$), 7.02 (1H, d, J 2.2Hz, ArH ortho to cyclopentyloxy), 7.1-7.3 (3H, m, meta and para ArH of $C_6H_5$), 7.46 (2H, ca d, J 8.5Hz, ortho ArH of $C_6H_5$), and 8.23 (1H, ca d, J 6 Hz, pyridine $H_6$); m/z (ESI) 404 ($M^+$+1, 72%), 387 (13), and 386 (100).

d) **(±)-4-[2-(3-Cyclopentyloxy-4-methoxyphenyl)-2-hydroxy-2-phenylethyl]pyrimidine**

From 4-methylpyrimidine (1.0ml) and Intermediate 2 (3.98g). Purification by chromatography ($SiO_2$;$CH_2Cl_2$) afforded the title compound (2.56g) as a white solid; $\delta_H$ ($CDCl_3$) 1.5-2.0 (8H, br m, $(CH_2)_4$), 3.66 (2H, s, $CH_2$ pyrimidine), 3.77 (3H, s, OMe), 4.65 (1H, br m, OCH), 6.58 (1H, s, OH), 6.72 (1H, d, J 8.4Hz, ArH ortho to OMe), 6.85 (1H, dd, J 8.4, 2.2Hz, ArH para to cyclopentyloxy), 6.98 (1 H, d, J 2.2Hz, ArH ortho to cyclopentyloxy), 7.07 (1H, d, J 5.2Hz, pyrimidine $H_5$), 7.15-7.45 (5H, m, $C_6H_5$), 8.53 (1H, d, J 5.2Hz, pyrimidine $H_6$), and 8.99 (1H, s, pyrimidine $H_2$).

## EXAMPLE 8

**(±)-2-[2-(3-Cyclopentyloxy-4-methoxyphenyl)-2-phenylethyl]-1-methylpyrrole**

$CH_3NH_2$ (generated from a concentrated aqueous solution of $CH_3NH_2$.HCl and KOH) was bubbled into a stirred solution of Intermediate 21 (400mg) in toluene (20ml) containing a catalytic amount of $CH_3NH_2$.HCl at RT for 0.5h. $Et_3N$ (2 drops) was added and the reaction mixture concentrated *in vacuo*. The residue was subjected to chromatography ($SiO_2$; 20% $Et_2O$/hexane) to afford the title compound (290mg) as a colourless oil. $\delta_H$ ($CDCl_3$) 1.5-1.9 (8H, br m, $(CH_2)_4$), 3.23 (2H, d, J 7.5Hz, $CHCH_2CO$), 3.28 (3H, s, NMe), 3.79 (3H, s, OMe), 4.19 (1H, t, J 7.5Hz, $CHCH_2CO$), 4.66 (1H, m OCH), 5.74 (1H, m, pyrrole H), 5.97 (1H, app. t, J 3.2Hz, pyrrole H), 6.44 (1H, app. t, J 2.2Hz, pyrrole H), 6.67 (1H, d, J 1.8Hz, ArH ortho to cyclopentyloxy), 6.70 (1H, dd, J 8.1Hz, ArH para to cyclopentyloxy), 6.76 (1H, d, J 8 Hz, ArH ortho to OMe), and 7.13-7.30 (5H, m, $C_6H_5$).

## EXAMPLE 9

**(±)-3-[2-(3-Cyclopentyloxy-4-methoxyphenyl)-2-phenylethyl]-5-hydroxy-[1H]-pyrazole**

A solution of Intermediate 22 (503mg,1.2mmol) and hydrazine monohydrate (73mg, 1.5mmol) in EtOH (10ml) was heated to reflux for 1.5h then cooled in an ice-bath. The crystalline product was filtered off, washed with cold EtOH and dried *in vacuo* to afford the title compound (3.5mg) as a white solid. m.p. 189-190°C. $\delta_H$ ($CDCl_3$) (indicates mixture of enol:keto forms; 2:1) 1.5-1.9 (8H, br m, $(CH_2)_4$), 2.85 (2/3H, s, $CH_2CO$; keto), 3.13 (4/3H, d, J 8Hz, $PhCHCH_2$; Keto), 3.25 (2/3H, d, J 8Hz, $PhCHCH_2$; enol), 3.80 (3H, s, OMe), 4.12 (2/3H, t, J 8Hz, $PhCHCH_2$; enol), 4.19 (1/3H, t, J 8Hz, $PhCHCH_2$; keto), 4.68 (1H, m, OCH), 5.35 (2/3H, s, HC=COH; enol), 6.65-6.8 (3H, m, $C_6H_3$), and 7.15-7.35 (5H, m, $C_6H_5$). (N.B. 2/3H for HC=COH; enol and NH for keto and enol forms not observed).

## EXAMPLE 10

**(±)-2-[2-(3-Cyclopentyloxy-4-methoxyphenyl)-2-phenylethyl]thiophene**

A mixture of Intermediate 19 (4.75mg) and Lawesson's Reagent (2,4-bis (4-methoxyphenyl)-1,3-dithia-2,4-diphosphetane-2,4-disulphide)(760mg) in toluene (10ml) was stirred at 85°C for 1.5h. The reaction mixture was cooled and filtered. The filtrate was concentrated *in vacuo* and the residual oil subjected to chromatography ($SiO_2$; 10% $Et_2O$/hexane) to afford the title compound (380mg) as a colourless oil (Found: C, 76.12; H, 6.88. $C_{24}H_{26}O_2S$ requires C, 76.15; H, 6.92%); $\delta_H$ ($CDCl_3$) 1.5-2.0 (8H, br m, $(CH_2)_4$), 3.56 (2H, d, J 7.6Hz, $PhCHCH_2$), 3.81 (3H, s, OMe), 4.21 (1H, t, J 7.6Hz, $PhCHCH_2$), 4.71 (1H, br m, OCH), 6.63 (1H, dd, J 3.4, 0.9Hz, thiophene $H_3$), 6.75-6.80 (3H, m, $C_6H_3$), 6.82 (1H, dd, J 5.1, 3.4Hz, thiophene $H_4$), 7.05 (1H, dd, J 5.1, 1.2Hz, thiophene $H_5$), and 7.15-7.35 (5H, m, $C_6H_5$).

**EXAMPLE 11**

**(±)-4-[2-(3-Cyclopentyloxy-4-methoxyphenyl)-2-phenylethyl] benzoic acid monohydrate**

Aqueous NaOH (10%; 50ml) was added to the compound of Example 3g (2.7g, 6.28mmol) in $CH_3OH$ (50ml) and the mixture heated to reflux for 3h. $CH_3OH$ was removed *in vacuo*, the remaining aqueous phase adjusted to pH7 with concentrated hydrochloric acid then extracted with $CH_2Cl_2$ (2x100ml). The extract was dried ($Na_2SO_4$) and concentrated *in vacuo* to afford the title compound (2.41g) as a white solid. m.p. 187-188.5°C. (Found: C, 74.44; H, 6.40. $C_{27}H_{28}O_4$. $H_2O$ requires C, 74.62; H, 6.96%), $\delta_H$ ($CDCl_3$) 1.2-2.0 (~10H, br m, $(CH_2)_4$+$H_2O$), 3.3-3.45 (2H, m, PhCHC$H_2$), 3.78 (3H, s, OMe), 4.16 (1H, t, J 8.0Hz, PhCHCH$_2$), 4.63 (1H, br m, OCH), 6.62 (1H, d, J 2.0Hz, ArH ortho to cyclopentyloxy), 6.69 (1H, dd, J 8.0, 2.0Hz, ArH para to cyclopentyloxy), 6.74 (1H, d, J 8.0Hz, ArH ortho to OMe), 7.09 (2H, d, J 8.2Hz, 2 x ArH meta to $CO_2H$), 7.15-7.3 (5H, m, $C_6H_5$), and 7.90 (2H, d, J 8.2Hz, 2xArH ortho to $CO_2H$); m/z (ESI) 439 ($M^+$+Na, 100%), 415 (20), 331 (25), 302 (28), 301 (35), and 213 (70).

**EXAMPLE 12**

**(±)-4-[2-(3-Cyclopentyloxy-4-methoxyphenyl)-2-phenylethyl] benzamide**

To the compound of Example 11 (210mg, 0.52mmol) in $CH_2Cl_2$ (10ml) was added $Et_3N$ (58mg, 0.58mmol) followed by isobutyl chloroformate (79mg, 0.58mmol) at RT and stirred for 0.5h. Ammonia was bubbled into the mixture for 10min and stirring continued for a further 0.5h. The reaction mixture was poured into aqueous $NaHCO_3$ (20ml) and extracted with $CH_2Cl_2$ (2x20ml). The extract was dried ($MgSO_4$), concentrated *in vacuo* and the residue subjected to chromatography ($SiO_2$; $Et_2O$)) to afford the title compound (150mg) as an off-white solid m.p. 73-75°C. $\delta_H$ ($CDCl_3$) 1.5-1.9 (8H, br m, $(CH_2)_4$), 3.35-3.79 (2H, m, PhCHC$H_2$), 3.79 (3H, s, OMe), 4.14 (1H, t, J 8.0Hz, PhCHCH$_2$), 4.65 (1H, br m, OCH), 5.5-6.0 (2H, v.br. s, CON$H_2$), 6.65 (1H, d, J 2.0Hz, ArH ortho to cyclopentyloxy), 6.68 (1H, dd, J 8.1, 2.0Hz, ArH para to cyclopentyloxy), 6.74 (1H, d, J 8.1Hz ArH ortho to OMe), 7.07 (2H, d, J 8.3Hz, 2xArH meta to $CONH_2$), 7.15-7.3 (5H, m, $C_6H_5$), and 7.62 (2H, d, J 8.3Hz, 2xArH ortho to $CONH_2$); m/z (ESI) 439 ($M^+$+1+Na, 25%), and 438 ($M^+$+Na, 100).

**EXAMPLE 13**

a) **(±)-tert.Butyl N-{4-[2-(3-Cyclopentyloxy-4-methoxyphenyl)-2-phenylethyl] phenyl}carbamate**

To the compound of Example 11 (1.5g, 3.6mmol) in 2-methylpropan-2-ol (50ml) was added $Et_3N$ (360mg, 3.6mmol) followed by diphenylphosphoryl azide (990mg, 3.6mmol) and the mixture heated to reflux for 3h. The cooled reaction mixture was poured into aqueous $NaHCO_3$ (100ml) and extracted with $CH_2Cl_2$ (2x100ml). The extract was dried ($MgSO_4$), concentrated *in vacuo* and the residue subjected to chromatography ($SiO_2$; hexane/$Et_2O$, 2:1) to afford the title compound (510mg) as a white solid m.p. 123-125°C; $\delta_H$ ($CDCl_3$) 1.49, 1.50 (9H, s, $CMe_3$), 1.5-1.95 (8H, br m, $(CH_2)_4$+$H_2O$), 3.25 (2H, d, J 7.5Hz, PhCHC$H_2$), 3.782, 3.790 (3H, s, OMe), 4.10 (1H, t, J 7.5Hz, PhCHCH$_2$), 4.65 (1H, br m, OCH), 6.33 (1H, br s, NH), 6.65-6.75 (3H, m, $C_6H_3$), 6.91 (2H, ~d, J 8.4Hz, 2xArH ortho to $NHCOCMe_3$), and 7.1-7.45 (7H, m, $C_6H_5$+2xArH meta to $NHCO_2CMe_3$), [N.B. CONH conformers observed by 'Hn.m.r.].

The following compound was prepared in a manner similar to the compound of Example 13a.

b) **(±)-tert.Butyl N-{3-[2-(3-cyclopentyloxy-4-methoxyphenyl)-2-phenylethyl]phenyl}carbamate**

From a compound of Example 3 j (1.44g, 3.46mmol), in 2-methylpropan-2-ol (50ml), $Et_3N$ (0.35g, 3.46mmol) and diphenylphosphoryl azide (0.95g, 3.46mmol). Purification by chromatography ($SiO_2$; hexane-EtOAc, 4:1) to afford the title compound (0.64g) as a colourless gum; $\delta_H$ ($CDCl_3$) 1.4-1.9 (8H, br m, $(CH_2)_4$), 1.50 (9H, s, $CMe_3$), 3.28 (2H, ca. d, J 8.0Hz, PhCHC$H_2$), 3.77 (3H, s, OMe), 4.16 (1H, t, J 8.0Hz, PhCHCH$_2$), 4.65 (1H, br m OCH), 6.39 (1H, br s, NH), and 6.6-7.4 (12H, m, $C_6H_5$+$C_6H_4$+$C_6H_3$).

**EXAMPLE 14**

**(±)-4-[2-(3-Cyclopentyloxy-4-methoxyphenyl)-2-phenylethyl]phenyl-N-ethylcarbamate**

Ethyl isocyanate (71mg, 1.0mmol) and a catalytic amount of $Et_3N$ (10µl) was added to a compound of Example 3b (300mg, 0.8mmol) in toluene (20ml) and the mixture heated at 60°C for 4h. The reaction mixture was poured into aqueous $NaHCO_3$ (50ml) and extracted with $CH_2Cl_2$ (2x50ml). The extract was dried ($MgSO_4$), concentrated *in vacuo,*

and the residue subjected to chromatography ($SiO_2$; $Et_2O$/hexane, 1:1) to afford the title compound (140mg) as a colourless gum; $\delta_H$ ($CDCl_3$) 1.18 (3H, t, J 7.2Hz, $NHCH_2Me$), 1.5-2.0 (8H, br m, $(CH_2)_4$), 3.2-3.35 (2H, m, $NHCH_2Me$), 3.29 (2H, d, J 7.8Hz, $PhCHCH_2$), 3.78 (3H, s, OMe), 4.11 (1H, t, J 7.8Hz, $PhCHCH_2$), 4.65 (1H, br m, OCH), 4.98 (1H, br s, NH), and 6.6-7.3 (12H, m, $C_6H_5+C_6H_4+C_6H_3$); m/z (ESI) 483 ($M^+$+1+Na, 38%), 482 (100), and 186 (23).

**EXAMPLE 15**

(i) **(+)-4-[1-(3-Cyclopentyloxy-4-methoxyphenyl)-2-(4-pyridyl)ethyl] pyridine**

(ii) **(-)-4-[1-(3-Cyclopentyloxy-4-methoxyphenyl)-2-(4-pyridyl)ethyl] pyridine**

A 60mg $ml^{-1}$ solution of the compound of Example 4 in EtOH was made up and prefiltered through a 45μ filter. The sample soution was injected onto a preparative chiracel OJ preparative column (mobile phase: 90:10, hexane/EtOH; flow rate 6ml.$min^{-1}$) in 0.5ml aliquots (a column loading of 30 mg). The two enantiomeric peaks were collected with a typical retention time of 50 to 63 min for the first peak and 70 to 105 min for the second peak.

**EXAMPLE 16**

(i) **(+)-4-[2-(3-Cyclopentyloxy-4-methoxyphenyl)-2-phenylethyl] pyridine**

(ii) **(-)-4-[2-(3-Cyclopentyloxy-4-methoxyphenyl)-2-phenylethyl] pyridine**

The compound of Example 3a (500mg) was made up to a 100mg $ml^{-1}$ solution in EtOH, filtered through a 45μ filtron. The sample solution was injected onto a preparative chiracel OJ preparative column (mobile phase; 80:20, hexane/EtOH; flow rate 6ml.$min^{-1}$) in 0.9ml aliquots. The two enantiomeric peaks were collected with a typical retention time of 22 to 32 min for the first peak corresponding to title enantiomer (i) (optical rotation at 0.151g/100ml of EtOH $[\alpha]_D^{22}$ = +37°) and 42 to 80 min for the second peak, corresponding to title enantiomer (ii) (optical rotation at 0.151g/100ml of EtOH $[\alpha]_D^{22}$ = +36°).

**CHIRACEL SEPARATION OF OTHER ENANTIOMERS OF THE INVENTION:**

The procedures described in Examples 15 and 16 were repeated [flow rate of 0.75ml.$min^{-1}$] with the following compounds, to obtain each enantiomer with the retention time shown:

| Compounds of | Mobile Phase (hexane-ethanol) | Peak A (min) | Peak B (min) |
|---|---|---|---|
| Example 1b | 80:20 | 22.21 | 30.96 |
| Example 5a | 80:20 | 10.76 | 13.22 |
| Example 5b | 80:20 | 7.31 | 7.93 |
| Example 1c | 80:20 | 13.96 | 17.44 |
| Example 3e | 80:20 | 17.87 | 30.34 |
| Example 3l | 80:20 | 17.73 | 26.54 |
| Exmple 3i | 80:20 | 17.33 | 25.50 |
| Example 3f | 90:10 | 11.77 | 13.25 |
| Example 3g | 80:20 | 19.30 | 40.32 |
| Example 8 | 80:20 | 13.52 | 15.42 |
| Example 27 | 70:30 | 31.54 | 50.03 |
| Example 11 | 80:20 | 20.00 | 42.00 |
| Example 3k | 80:20 | 6.25 | 7.10 |
| Example 21 | 80:20 | 15.67 | 20.64 |
| Example 25 | 80:20 | 15.47 | 17.90 |
| Example 22 | 80:20 | 8.30 | 11.00 |
| Example 33 | 80:20 | 13.82 | 15.15 |
| Example 31 | 80:20 | 21.87 | 28.84 |
| Example 10 | 80:20 | 10.96 | 11.81 |
| Example 24 | 85:15 | 36.81 | 39.07 |

(continued)

| Compounds of | Mobile Phase (hexane-ethanol) | Peak A (min) | Peak B (min) |
|---|---|---|---|
| Example 5c | 80:20 | 18.96 | 54.27 |

**EXAMPLE 18**

**(±)-2-[2[(3-Cyclopentyloxy-4-methoxyphenyl)-2-phenylethyl]benzo[d] thiazole**

Intermediate 19 (1.26g, 3.5mmol) in $CH_2Cl_2$ (6ml) was added to a stirred solution of 2-aminothiophenol (0.44g, 3.51 mmol) in $CH_2Cl_2$ (8ml) and pyridine (2ml) at -70°C. The reaction mixture was stirred at -70°C for 20h, warmed to RT, concentrated *in vacuo*, and the residual brown oil subjected to chromatography ($SiO_2$; EtOAc-hexane, 1:1) to afford the title compound (826mg) as a pale green oil (Found: C, 75.15; H, 6.31; N, 3.30. $C_{27}H_{27}NO_2S$ requires C, 75.49; H, 6.34; N, 3.26%); $\delta_H$ ($CDCl_3$) 1.5-1.9 (8H, br m, $(CH_2)_4$), 3.78 (3H, s, OMe), 3.83 (2H, ca d, J ca 8Hz, $PhCHCH_2$), 4.60 (1H, t, J 8.0Hz, $PhCHCH_2$), 4.63 (1H, br m, OCH), 6.7-6.85 (3H, m, $C_6H_3$), 7.1-7.45 (7H, m, $C_6H_5$+ benzothiazole $H_5$, $H_6$), 7.74 (1H, ca d, J 8Hz, benzothiazole $H_4$ or $H_7$), and 7.95 (1H, ca d, J ca 8Hz, benzothiazole $H_4$ or $H_7$).

**EXAMPLE 19**

**(±)-4-[1-(3-Cyclopentyloxy-4-methoxyphenyl)-2-(4-pyridyl)ethyl] benzaldehyde**

NaOH (800mg, 20mmol) in water (20ml) was added to a solution of Intermediate 24 (2.46g, 4.87mmol) in EtOH (50ml) and the mixture heated to reflux for 1.5h. Concentrated hydrochloric acid was added to pH 4.5 and the mixture heated to reflux for 18h to complete the decarboxylation. The reaction mixture was concentrated to half-volume and partitioned between NaOH solution (0.5M; 100ml) and $Et_2O$ (100ml). The organic layer was separated, washed with brine (25ml), dried ($MgSO_4$), and concentrated *in vacuo* to afford the title compound (1.80g) as a pale orange gum; $\delta_H$ ($CDCl_3$) 1.5-2.0 (8H, br m, $(CH_2)_4$), 3.35 (2H, ca d, J 7.8Hz, $CHCH_2$ pyridine), 3.80 (3H, s, OMe), 4.25 (1H, t, J 7.8Hz, $CHCH_2$ pyridine), 4.65 (1H, br m, OCH), 6.63 (1H, d, J 1.8Hz, ArH ortho to cyclopentyloxy), 6.70 (1H, dd, J 7.8, 1.8Hz, ArH para to OMe), 6.78(1H, d, J 7.8Hz, ArH ortho to OMe), 6.92 (2H, ca d, J 6.7Hz, pyridine $H_3$, $H_5$), 7.35 (2H, d, J 8.3Hz, 2xArH meta to CHO), 7.79 (2H, d, J 8.3Hz, 2xArH ortho to CHO), 8.40 (2H, ca d, J 6.7Hz, pyridine $H_2$,$H_6$) and 9.97 (1H, s, CHO); m/z (ESI) 402 ($M^+$ +1,38%), 310 (22), and 309 (100). Treatment of the title compound (400mg) in $Et_2O$ (40ml) with ethanolic HCl (2.5M) and concentration *in vacuo* afforded the title compound hydrochloride (420mg) as a yellow solid.

**EXAMPLE 20**

**(±)-4-[2-(3-Cyclopentyloxy-4-methoxyphenyl)-2-(4-hydroxymethyl phenyl)ethyl]pyridine**

Sodium borohydride (235mg, 6.21 mmol) was added portionwise to the compound of Example 19 (1.11g, 2.85mmol) in EtOH (35ml) at -20°C. The suspension was allowed to warm to RT and stirred for 18h then treat ed dropwise with glacial acetic acid. The reaction mixture was concentrated *in vacuo* and the residue partitioned between $Et_2O$ (50ml) and NaOH solution (1M; 50ml). The organic phase was separated, dried ($MgSO_4$) and concentrated *in vacuo.* to afford the title compound (1.03g) as a colourless gum m.p. 179-182°C; $\delta_H$ ($CDCl_3$) 1.5-2.0 (8H, br m, $(CH_2)_4$), 2.4 (1H, v.br.s, $CH_2OH$), 3.31 (2H, d, J 7.9Hz, $CHCH_2$ pyridine), 3.80 (3H, s, OMe), 4.15 (1H, t, J 7.9Hz, $CHCH_2$ pyridine), 4.65 (3H, sl.br.s, OCH+$CH_2OH$), 6.6-6.8 (3H, m, $C_6H_3$), 6.92 (2H, ca d, J 6.5Hz, pyridine $H_3$, $H_5$), 7.19 (2H, d, J 8.1Hz, 2xArH of $C_6H_4$), 7.26 (2H, d, J 8.1Hz, 2xArH of $C_6H_4$), and 8.35 (2H, ca d, J 6.5Hz, pyridine $H_2$,$H_6$); m/z (ESI) 404 ($M^+$ +1,35%), 312 (30), and 311 (100).

Treatment of the title compound (600mg) in $Et_2O$ (50ml) with ethanolic HCl (2.5M), concentration *in vacuo* followed by recrystallisation ($EtOH-Et_2O$) afforded the title compound hydrochloride (602mg) as a white solid; $\delta_H$ ($d_4$-MeOH) 1.5-2.0 (8H, br m, $(CH_2)_4$), 3.72 (2H, d, J 8.1Hz, $CHCH_2$ pyridine), 3.76 (3H, s, OMe), 4.44 (1H, t, J 8.1Hz, $CHCH_2$ pyridine), 4.55 (2H, s, $CH_2OH$), 4.74 (1H, br m, OCH), 6.8-6.85 (3H, m, $C_6H_3$), 7.25-7.35 (4H, m, $C_6H_4$), 7.87 (2H, ca d, J 6.8Hz, pyridine $H_3$, $H_5$), and 8.62 (2H, ca d, J 6.8Hz, pyridine $H_2$,$H_6$) (N.B. $CH_2OH$ and HCl not observed).

**EXAMPLE 21**

**(±)-4-[2-(3-Cyclopentyloxy-4-methoxyphenyl)-2-(4-methoxymethylphenyl)ethyl]pyridine**

The compound of Example 20 (400mg, 1.02mmol) in THF (10ml) was added to a suspension of NaH (60% dispersion in oil) (124mg, 3.09mmol) in THF (10ml) at O°C then allowed to warm at RT over 0.5h. The mixture was cooled to -20°C, treated with a solution of methyl iodide (98.4µl, 1.58mmol) in THF (5ml) and allowed to warm to RT. A further portion of methyl iodide (300µL, 4.8mmol) was added and the mixture allowed to stir at RT overnight then concentrated *in vacuo*. The residue was subjected to chromatography ($SiO_2$; EtOAc-hexane) to afford the title compound (125mg) as a pale yellow gum; $\delta_H$ ($CDCl_3$) 1.5-2.0 (8H, br m, $(CH_2)_4$), 3.29 (2H, d, J 8.3Hz, $CHCH_2$ pyridine), 3.38 (3H, s, $CH_2OMe$), 3.80 (3H, s, OMe), 4.14 (1H, t, J 8.3Hz, $CHCH_2$ pyridine), 4.40 (2H, s, $CH_2OMe$), 4.63 (1H, br m, OCH), 6.6-6.8 (3H, m, $C_6H_3$), 6.92 (2H, ca d, J 6.5Hz, pyridine $H_3$, $H_5$), 7.18 (2H, d, J 8.2Hz, 2xArH of $C_6H_4$), 7.25 (2H, d, J 8.2Hz, 2xArH of $C_6H_4$), and 8.39 (2H, ca d, J 6.5Hz, pyridine $H_2$,$H_6$); m/z (ESI) 419 ($M^+$ +2, 15%), 418 ($M^+$ +1, 45), 326 (33), and 325 (100).

Treatment of the title compound (100mg) in $Et_2O$ (25ml) with ethanolic HCl (2.5M) then concentration *in vacuo* and recrystallisation (EtOH-$Et_2O$) afforded the title compound hydrochloride (102mg) as an off-white solid m.p. 182-185°C; $\delta_H$ ($d_4$-MeOH) 1.5-1.9 (8H, br m, $(CH_2)_4$), 3.34 (3H, s, $CH_2OMe$), 3.71 (2H, d, J 8.3Hz, $CHCH_2$ pyridine), 3.75 (3H, s, OMe), 4.39 (2H, s, $CH_2OMe$), 4.43 (1H, t, J 8.3Hz, $CHCH_2$ pyridine), 4.73 (1H, br m, OCH), 6.75-6.85 (3H, m, $C_6H_3$), 7.25 (2H, d, J 8.3Hz, 2xArH of $C_6H_4$), 7.32 (2H, d, J 8.3Hz, 2xArH of $C_6H_4$), 7.84 (2H, ca d, J 6.7Hz, pyridine $H_3$, $H_5$), and 8.61 (2H, ca d, J 6.7Hz, pyridine $H_2$,$H_6$).

**EXAMPLE 22**

**(±)-4-[2-(3-Cyclopentyloxy-4-methoxyphenyl)-2-(4-dimethylaminomethylphenyl)ethyl]pyridine**

Ethanolic HCl (2.5M) was added dropwise to dimethylamine (3.6ml of a 14% w/v solution in $CH_3OH$, 11.1mmol, 7.6eq) followed by the compound of Example 19 (570mg, 1.46mmol) in $CH_3OH$ (5ml) and sodium cyanoborohydride (92mg, 1.46mmol) in one portion. The reaction mixture was stirred at RT for 24h then concentrated *in vacuo* and partitioned between EtOAc (25ml) and NaOH solution (2M). The organic layer was separated, dried ($K_2CO_3$), and concentrated *in vacuo* to give a pale brown gum which was subjected to chromatography ($SiO_2$; $CH_3OH$-$CH_2Cl_2$, 1: 19) to afford the title compound (310mg) as a pale yellow gum; $\delta_H$ ($CDCl_3$; 250MHz) 1.5-2.0 (8H, br m, $(CH_2)_4$), 2.21 (6H, s, $NM_2$), 3.29 (2H, d, J 7.9Hz, $CHCH_2$ pyridine), 3.37 (2H, s, $CH_2NMe_2$), 3.79 (3H, s, OMe), 4.13 (1H, t, J 7.9Hz, $CHCH_2$ pyridine), 4.64 (1H, br m, OCH), 6.63 (1H, d, J 1.9Hz, ArH ortho to cyclopentyloxy), 6.68 (1H, dd, J 8.2, 1.9Hz, ArH para to OMe), 6.74 (1H, d, J 8.2Hz, ArH ortho to OMe), 6.92 (2H, ca d, J 6.0Hz, pyridine $H_3$, $H_5$), 7.13 (2H, d, J 8.2Hz, 2xArH of $C_6H_4$), 7.24 (2H, d, J 8.2Hz, 2xArH of $C_6H_4$), and 8.37 (2H, ca d, J 6.0Hz, pyridine $H_2$,$H_6$); m/z (ESI) 432 ($M^+$+2, 30%), 431 ($M^+$ +1, 100), 338 (31), 294 (16), 226 (16), and 136 (9).

Treatment of the title compound (310mg) in $Et_2O$ (25ml) with ethanolic HCl (2.5M) and concentration *in vacuo* afforded the title compound dihydrochloride (360mg) as a pale yellow solid; $\delta_H$ ($d_4$-MeOH) 1.5-2.0 (8H, br m, $(CH_2)_4$), 2.82 (6H,s, $CH_2NMe_2$), 3.75 (5H, sl.br.s, OMe + $CHCH_2$ pyridine), 4.27 (2H, s, $CH_2NMe_2$), 4.52 (1H, t, J ca 8.0Hz, $CHCH_2$ pyridine), 4.78 (1H, br m, OCH), 6.8-6.9 (3H, m, $C_6H_3$), 7.4-7.6 (4H, m, $C_6H_4$), 7.88 (2H, ca d, J 6.7Hz, pyridine $H_3$, $H_5$), 8.63 (2H, ca d, J 6.7Hz, pyridine $H_2$,$H_6$) (N.B. HCl not observed).

**EXAMPLE 23**

**(±)-4-[1-(3-Cyclopentyloxy-4-methoxyphenyl)-2-(4-pyridyl)ethyl]benzoic acid**

Aqueous sodium dihydrogen phosphate (5%; 15ml), then $KMnO_4$ (2.0g, 12.7mmol) in water (20ml), were added to a solution of the compound of Example 19 (1.50g, 3.85mmol) in t-butanol (25ml) at RT. After 0.25h, aqueous sodium sulphite solution (20ml) was added, the reaction mixture filtered through Celite®, the filter pad washed well with NaOH solution (0.5M), and the filtrate concentrated *in vacuo.* The residue was partitioned between $Et_2O$ (50ml) and water (50ml), the aqueous phase separated and acidified to pH 4 with concentrated hydrochloic acid. The mixture was cooled overnight at about 4°C, the precipitate filtered off and washed with water then $Et_2O$ and dried *in vacuo* to afford the title compound (950mg, 61%) as a white solid m.p. 161-163°C; $\delta_H$ ($d_4$-MeOH) 1.5-1.9 (8H, br m, $(CH_2)_4$), 3.42 (2H, d, J 8.0Hz, $CHCH_2$ pyridine), 3.75 (3H, s, OMe), 4.35 (1H, t, J 8.0Hz, $CHCH_2$ pyridine), 4.70 (1H, br m, OCH), 6.7-6.85 (3H, m, $C_6H_3$), 7.18 (2H, d, J 6.7Hz, pyridine $H_3$, $H_5$), 7.38 (2H, d, J 8.3Hz, 2xArH meta to $CO_2H$), 7.93 (2H, d, J 8.3Hz, 2xArH ortho to $CO_2H$), and 8.30 (2H, d, J 6.7Hz, pyridine $H_2$,$H_6$) (N.B. $CO_2H$ not observed); m/z (ESI) 419 ($M^+$+2, 12%), 418 ($M^+$+1, 40), 326(23) and 325 (100).

Treatment of the title compound (235mg) in $Et_2O$ (25ml) with ethanolic HCl (2.5M), concentration *in vacuo*

and recrystallisation (EtOH-$Et_2O$) afforded the title compound hydrochloride (224mg) as a white solid; $\delta_H$ ($d_4$-MeOH) 1.5-1.9 (8H, br m, $(CH_2)_4$), 3.75(2H, d, J 8.0Hz, $CHCH_2$ pyridine), 3.75 (3H, s, OMe), 4.52 (1H, t, J 8.0Hz, $CHCH_2$ pyridine), 4.74 (1H, br m, OCH), 6.8-6.9 (3H, m, $C_6H_3$), 7.43 (2H, d, J 8.3Hz, 2xArH meta to $CO_2H$), 7.80 (2H, d, J 8.3Hz, 2xArH ortho to $CO_2H$), 7.88 (2H, d, J 6.7Hz, pyridine $H_3$, $H_5$), and 8.62 (2H, d, J 6.7Hz, pyridine $H_2$,$H_6$) (N.B. $CO_2H$ and HCl not observed).

## EXAMPLE 24

### (±)-4-[1-(3-Cyclopentyloxy-4-methoxyphenyl)-2-(4-pyridyl)ethyl] benzamide

N-Methylmorpholine (163µL, 1.48mmol, 1.5eq) then isobutyl chloroformate (142µL, 1.09mmol, 1.1 eq) were added to the compound of Example 23 (400mg, 1.00mmol) in THF-DMF (20ml; 3:1) at -20°C. Concentrated aqueous ammonia solution (1.0ml) was added, the mixture allowed to warm to RT overnight then concentrated *in vacuo*. The residue was partitioned between EtOAc (25ml) and NaOH solution (1M; 20ml). The organic layer was separated, washed with phosphate buffer (pH 7), dried ($MgSO_4$), and concentrated *in vacuo*. The residue was subjected to chromatography ($SiO_2$; $CH_3OH$-$CH_2Cl_2$, 1:19) to afford the title compound (245mg) as a pale yellow gum m.p. 180-182°C; $\delta_H$ ($CDCl_3$; 250MHz) 1.5-2.0 (8H, br m, $(CH_2)_4$), 3.32(2H, d, J 7.9Hz, $CHCH_2$ pyridine), 3.80 (3H, s, OMe), 4.20 (1H, t, J 7.9Hz, $CHCH_2$ pyridine), 4.64 (1H, br m, OCH), 5.6 (1H, v.br.s. CONH), 6.0 (1H, v.br.s. CONH), 6.63 (1H, d, J 2Hz, ArH ortho to cyclopentyloxy), 6.68 (1H, dd, J 8.2, 2.0Hz, ArH para to OMe), 6.76 (1H, d, J 8.2Hz, ArH ortho to OMe), 6.92 (2H, ca d, J 6.0Hz, pyridine $H_3$, $H_5$), 7.26 (2H, ca d, J 8.3Hz, 2xArH meta to $CONH_2$), 7.71 (2H, ca d J 8.3Hz, 2xArH ortho to $CONH_2$) and 8.40 (2H, ca d. J 6.0Hz, pyridine $H_2$,$H_6$); m/z (ESI) 418 ($M^+$+2, 15%), 417 ($M^+$+1, 48), 325 (22), and 324 (100).

Treatment of the title compound (240mg) in $Et_2O$ (25ml) with ethanolic HCl (2.5M), concentration *in vacuo,* and recrystallisation (EtOH-$Et_2O$) afforded the title compound hydrochloride (245mg) as a white solid; $\delta_H$ ($d_4$-MeOH) 1.5-2.0 (8H, br m, $(CH_2)_4$), 3.75 (2H, d, J 8.2Hz, $CHCH_2$ pyridine), 3.75 (3H, s, OMe), 4.54 (1H, t, J 8.2Hz, $CHCH_2$ pyridine), 4.76 (1H, br m, OCH), 6.8-6.9 (3H, m, $C_6H_3$), 7.44 (2H, d, J 8.4Hz, 2xArH meta to $CONH_2$), 7.88 (2H, d, J 6.7Hz, pyridine $H_3$, $H_5$), 7.94 (2H, d, J 8.4Hz, 2xArH ortho to $CONH_2$), and 8.63 (2H, d, J 6.7Hz, pyridine $H_2$,$H_6$) (N.B. $CONH_2$ and HCl not observed).

## EXAMPLE 25

### (±)-Ethyl 4-[1-(3-Cyclopentyloxy-4-methoxyphenyl)-2-(4-pyridyl)ethyl] benzoate

Acetyl chloride (500µL) was added to EtOH (10ml) followed by the compound of Example 23 (385mg, 0.95mmol) and the resulting solution heated to reflux for 18h. The reaction mixture was concentrated *in vacuo* and the residue partitioned between aqueous sodium carbonate solution (2M; 10ml) and $Et_2O$ (25ml). The organic layer was separated, dried ($MgSO_4$), concentrated *in vacuo* and the residue subjected to chromatography ($SiO_2$; EtOAc-hexane, 1:1 to 3:2) to afford the title compound (300mg) as a pale yellow gum m.p. 170-173°C; $\delta_H$ ($CDCl_3$) 1.39 (3H, t, J 7.5Hz, $COCH_2Me$), 1.5-2.0 (8H, br m, $(CH_2)_4$), 3.32 (2H, d, J 8.0Hz, $CHCH_2$ pyridine), 3.80 (3H, s, OMe), 4.20 (1H, t, J 8.0Hz, $CHCH_2$ pyridine), 4.30 (2H, q, J 7.5HZ, $COCH_2Me$), 4.62 (1H, br m, OCH), 6.65 (1H, d, J 2.0Hz, ArH ortho to cyclopentyloxy), 6.68 (1H, dd, J 7.8, 2.0Hz, ArH para to OMe), 6.78 (1H, d, J 7.8Hz, ArH ortho to OMe), 6.92 (2H, dd, J 5.2, 0.8Hz, pyridine $H_3$, $H_5$), 7.25 (2H, d, J 8.5Hz, 2xArH meta to $CO_2Et$), 7.94 (2H, d, J 8.5Hz, 2xArH ortho to $CO_2Et$), and 8.40 (2H, dd. J 5.2, 0.8Hz, pyridine $H_2$,$H_6$); m/z (ESI) 447 ($M^+$+2, 20%), 446 ($M^+$+1, 63), 354 (27), 353 (100), and 285 (35).

Treatment of the title compound (295mg) in $Et_2O$ (25ml) with ethanolic HCl (2.5M), concentration *in vacuo* and recrystallisation (EtOH-$Et_2O$) afforded the title compound hydrochloride (300mg) as an off-white solid; $\delta_H$ ($d_4$-MeOH) 1.36 (3H, t, J 7.2Hz, $COCH_2Me$), 1.5-1.9 (8H, br m, $(CH_2)_4$), 3.73 (2H, d, J 8.2Hz, $CHCH_2$ pyridine), 3.76 (3H, s, OMe), 4.33 (2H, q, J 7.2Hz, $COCH_2Me$), 4.53 (1H, t, J 8.2Hz, $CHCH_2$ pyridine), 4.75 (1H, br m, OCH), 6.8-6.9 (3H, m, $C_6H_3$), 7.45 (2H, d, J 8.4Hz, 2xArH meta to $CO_2Me$), 7.84 (2H, d, J 6.5Hz, pyridine $H_3$, $H_5$), 7.94 (2H, d, J 8.4Hz, pyridine $H_2$,$H_6$), and 8.61 (2H, d, J 6.5Hz, pyridine $H_2$,$H_6$).

## EXAMPLE 26

### (±)-2-Chloro-4-[2-(3-cyclopentyloxy-4-methoxyphenyl)-2-phenylethyl] pyridine

A mixture of the compound of Example 6 (2.39g, 6.16mmol) and phosphorus oxychloride (25ml) was heated to reflux overnight. The reaction mixture was cooled to RT then carefully added to saturated potassium carbonate solution (250ml). Potassium hydroxide (2M) was added to pH 7.5 and the yellow-orange mixture extracted with EtOAc

(3x50ml). The extract was washed with brine (30ml), dried ($MgSO_4$), and concentrated *in vacuo* to give a red-brown gum which was subjected to chromatography ($SiO_2$; $Et_2O$-hexane, 1:1) to afford the title compound (1.16g, 46%) as a pale yellow gum; $\delta_H$ ($CDCl_3$) 1.5-2.0 (8H, br m, $(CH_2)_4$), 3.30 (2H, d, J 8.0Hz, $PhCHCH_2$), 3.80 (3H, s, OMe), 4.14 (1H, t, J 8.0Hz, $PhCHCH_2$), 4.66 (1H, br m, OCH), 6.68 (1H, d, J 2.0Hz, ArH ortho to cyclopentyloxy), 6.69 (1H, dd, J 8.0, 2.0Hz, ArH para to OMe), 6.76 (1H, d, J 8.0Hz, ArH ortho to OMe), 6.84 (1H, d, J 6.5Hz, pyridine $H_5$), 7.00 (1H, s, pyridine $H_3$), 7.05-7.3 (5H, m, $C_6H_5$), and 8.17 (1H, d, J 6.5Hz, pyridine $H_6$).

**EXAMPLE 27**

**(±)-3-[2-(3-Cyclopentyloxy-4-methoxyphenyl)-2-phenylethyl] aniline**

Sodium iodide (210mg, 1.4mmol) and trimethylsilyl chloride (152mg, 1.4mmol) was added to the compound of Example 13 b (620mg, 1.27mmol) in acetonitrile (20ml) and the mixture stirred at RT for 1h. The reaction mixture was poured into 10% sodium thiosulphate solution (50ml) and extracted with $CH_2Cl_2$ (2x50ml). The extract was dried ($MgSO_4$), concentrated *in vacuo* and the residue subjected to chromatography ($SiO_2$; $Et_2O$) to afford the title compound (210mg) as a colourless gum; $\delta_H$ ($CDCl_3$) 1.5-1.9 (8H, br m, $(CH_2)_4$), 3.21 (2H, d, J 7.6Hz, $PhCHCH_2$), 3.44 (2H, br s, $NH_2$), 3.75 (3H, s, OMe),4.14 (1H, t, J 7.6Hz, $PhCHCH_2$), 4.65 (1H, br m OCH), 6.3-6.45 (3H, m, $C_6H_3$), and 6.6-7.4 (9H, m, $C_6H_5$+$C_6H_4$); m/z (ESI) 410 ($M^+$+ Na, 30%), 388 ($M^+$+1, 60), 320 (58), 213 (23), and 196 (100).

**EXAMPLE 28**

**(±) -Sodium 3-[2-(3-Cyclopentyloxy-4-methoxyphenyl)-2-phenylethyl]-1,2,4-triazolyl-5-thiolate**

A mixture of thiosemicarbazide (0.43g, 4.7mmol) and Intermediate 19 (1.70g, 4.7mmol) in toluene (30ml) was heated to reflux for 4h. The reaction mixture was cooled, diluted with $Et_2O$ (30ml) and the precipitate collected by filtration. The precipitate was washed with $Et_2O$ then water to give a white solid, a portion of which (0.41g) was suspended in aqueous $Na_2CO_3$(2M; 30ml) and heated to reflux for 4h. The cooled reaction mixture was diluted with water (20ml), acidified with 10% hydrochloric acid to pH5 and extracted with $CH_2Cl_2$ (2x40ml). The extract was dried ($MgSO_4$), concentracted *in vacuo*, and the residue recrystallised ($CH_3OH$) to afford the title compound (0.31g) as a white solid (Found: C, 62.97; H, 5.98; N, 10.02. $C_{22}H_{24}N_3NaO_2S$ requires C, 63.29; H, 5.79; N, 10.07%); $\delta_H$(250MHz; DMSO-$d_6$) 1.5-2.0 (8H, br m, $(CH_2)_4$), 3.26 (2H, d, J 8.2Hz, $PhCHCH_2$), 3.67 (3H, s, OMe), 4.45 (1H, t, J 8.2Hz, $PhCHCH_2$), 4.72 (1H, br m, OCH), 6.7-6.85 (3H, m, $C_6H_3$), 7.1-7.35 (5H, m, $C_6H_5$), and 13.09 (1H, br s, NH); m/z (ESI) 419 ($M^+$+ 1+Na, 35%), 418 ($M^+$+ Na, 67), 397 ($M^+$+ 1, 95), 396 ($M^+$, 100),328 (15), 204 (25), and 60 (81).

**EXAMPLE 29**

**(-)-2-[2-(3-Cyclopentyloxy-4-methoxyphenyl)-2-phenylethyl] benzimidazole**

Intermediate 19 (2.47g, 6.9mmol) in THF (10ml) was added dropwise to a solution of 1,2-diaminobenzene (3.72g, 34.4mmol) in THF (40ml) at O°C and the mixture stirred for 2h. The reaction mixture was concentrated *in vacuo* and the residue washed with $Et_2O$ (5x50ml). The extract was washed with 10% hydrochloric acid (50ml), sodium hydrogen carbonate solution (50ml), brine (50ml), then concentrated *in vacuo*. The residue was subjected to chromatography ($SiO_2$; EtOAc-hexane, 1:1) to give a pale brown glassy solid (1.02g), a portion of which (0.44g) was heated neat at 150°C for 60h then subjected to chromatography ($SiO_2$;$Et_2O$-hexane 1:1) to afford the title compound (273mg) as an off-white solid m.p. 97.5-98°C; $\delta_H$($CDCl_3$) 1.4-1.9 (8H, br m, $(CH_2)_4$), 3.6-3.7 (2H, m, $PhCHCH_2$), 3.78 (3H, s, OMe), 4.55 (1H, br m, OCH), 4.57 (1H, t, J 8Hz, $PhCHCH_2$), 6.7-6.8 (3H, m, $C_6H_3$), and 7.15-7.5 (9H, m, $C_6H_5$ + benzimidazole $H_4$, $H_5$, $H_6$, $H_7$); m/z (ESI) 413 ($M^+$+ 1, 100%), 186 (48). (Optical rotation at 0.151g/100ml of EtOH $[\alpha]_D^{22}$=-1).

**EXAMPLE 30**

a) **(±)-4-[1-(3-Cyclopentyloxy-4-methoxyphenyl)-2-(4-pyridyl) ethyl]aniline,Dihydrochlroide, Hemihydrate**

A mixture of Intermediate 26 (3.80g), ammonium formate (1.63g), and 10% Pd/C (about 100mg) in EtOH (50ml) was heated to reflux for 2h then stirred at RT for 2 days. The reaction mixture was then filtered through Celite® and the filtrate concentrated *in vacuo.* The residue was partitioned between aqueous NaOH (1M; 50ml) and $CH_2Cl_2$ (50ml). The organic layer was separated, dried ($Na_2SO_4$), and concentrated *in vacuo.* The residue was dissolved in $Et_2O$ (50ml) and treated with ethanolic HCl (2.5M) then concentrated *in vacuo.* The residue was recrystallised (EtOH-$Et_2O$) to afford the title compound (3.4g) as an off-white solid (Found: C, 63.97; H, 6.52; N, 5.77. $C_{25}H_{28}N_2O_2$. 2HCl.

5$H_2O$ requires C, 63.83; H, 6.64; N, 5.96%); $\delta_H$ ($d_4$-MeOH) 1.5-1.9 (8H, br m, $(CH_2)_4$), 3.7-3.85 (2H, m, $CHCH_2$ pyridine), 3.74 (3H, s, OMe), 4.56 (1H, t, J 8.8Hz, $CHCH_2$ pyridine), 4.75 (1H, br m OCH), 6.8-6.85 (3H, m, $C_6H_3$), 7.35 (2H, ca. d, J 8.5Hz, ArH of $C_6H_4$), 7,55 (2H, d, J 8.5Hz, ArH of $C_6H_4$), 7.91 (2H, d, J 6.6Hz, pyridine $H_3$, $H_5$) and 8.65 (2H, d, J 6.6Hz, pyridine $H_2$, $H_6$) (N.B. $NH_2$ and 2HCl not observed); m/z (ESI) 389 ($M^+$+ 1, 11%), 297 (26), 296 (100), and 228 (11).

The following compound was prepared in a manner similar to the compound of Example 28a.

b) **(±)-2-[4-(1-(3-Cyclopentyloxy-4-methoxyphenyl)-2-(4-pyridyl) ethyl)phenyl]-4,4-dimethyl-1,3-oxazoline, Dihydrochloride Dihydrate**

From Intermediate 28b (0.27g, 0.56mmol), ammonium formate (0.70g, 11.2mmol) and 10% Pd/C (50mg). Chromatography ($SiO_2$; EtOAc) gave the title compound free base (180mg) as a clear gum.

Dissolution of the title compound free base in $Et_2O$ and treatment with ethereal HCl (1M) furnished the title compound as a white solid. (Found: C, 61.98; H, 6.53; N, 4.61. $C_{30}H_{34}N_2O_3$. $_2$HCl 2$H_2O$ requires C, 62.17; H, 6.96; N, 4.83%) $\delta_H$ ($d_4$-MeOH) 1.59 (6H, s $CMe_2$), 1.6-1.9 (8H, br m, $(CH_2)_4$), 3.75 (3H, s, OMe), 3.7-3.9 (2H, m, $CH_2$-pyridine), 4.64 (1H, t, $CHCH_2$-pyridyl), 4.73 (1H, m, OCH), 4.77 (2H, s, CH oxazolinyl), 6.8-6.9 (3H, m, $C_6H_3$), 7.68 (2H, d, J 8.4Hz, ArH meta to oxazoline), 7.90 (2H, d, J 6.6Hz, pyridine $H_3$, $H_5$), 8.01 (2H, d, J 8.4Hz, ArH ortho to oxazoline), and 8.65 (2H, d, J 6.5Hz, pyridine $H_2$, $H_6$); m/z (ESI) 471 ($M^+$+ 1, 100%), 378 (67), and 245 (20).

## EXAMPLE 31

**(±)-Ethyl N-{4-[1-(3-Cyclopentyloxy-4-methoxyphenyl)-2-(4-pyridyl) ethyl] phenyl}carbamate**

Ethyl chloroformate (81mg, 0.74mmol, 1.3eq) was added dropwise to a mixture of the compound of Example 30 (221mg, 0.57mmol) and triethylamine (75mg, 0.74mmol, 1.3eq) in $CH_2Cl_2$ (20ml). The reaction mixture was stirred overnight at RT, then concentrated *in vacuo* and the residue subjected to chromatography ($SiO_2$; hexane/EtOAc, 1:1) to afford the title compound (170mg) as a white solid; $\delta_H$ ($CDCl_3$) 1.29 (3H, t, J 7.1Hz, $OCH_2Me$), 1.5-2.0 (8H, br m, $(CH_2)_4$), 3.27 (2H, d, J 7.9Hz, $CHCH_2$ pyridine), 3.79 (3H, s, OMe), 4.10 (1H, t, J 7.9Hz, $CHCH_2$ pyridine), 4.20 (2H, q, J 7.1Hz, $OCH_2Me$), 4.64 (1H, br m OCH), 6.51 (1H, br s, NH), 6.6-6.8 (3H, m, $C_6H_3$), 6.92 (2H, d, J 5.9Hz, pyridine $H_3$, $H_5$), 7.11 (2H, d, J 8.4Hz, ArH of $C_6H_4$), 7.27 (2H, d, J 8.4Hz, ArH of $C_6H_4$), and 8.38 (2H, d, J 5.9Hz, pyridine $H_2$, $H_6$); m/z (ESI) 461 ($M^+$+ 1, 90%), 369 (25), and 368 (100).

## EXAMPLE 32

**(±)-N-{4-[1-(3-Cyclopentyloxy-4-methoxyphenyl)-2-(4-pyridyl)ethyl] phenyl}N'-ethylurea**

A mixture of the compound of Example 30 (246mg, 0.63mmol) and ethyl isocyanate (68mg, 0.95mmol, 1.5eq) in $CH_2Cl_2$ (20ml) was stirred at RT for 2 days. A further portion of ethyl isocyanate (68mg, 0.95mmol, 1.5eq) was added and the mixture allowed to stir for 20h. The reaction mixture was concentrated *in vacuo* and the residue subjected to chromatogrpahy ($SiO_2$; EtOAc) to afford the title compound (221mg) as a white solid; $\delta_H$ ($CDCl_3$) 1.14 (3H, t, J 7.2Hz, $OCH_2Me$), 1.5-2.0 (8H, br m, $(CH_2)_4$), 3.2-3.35 (4H, m, $CHCH_2$ pyridine + $OCH_2Me$), 3.79 (3H, s, OMe), 4.11 (1H, t, J 7.8Hz, $CHCH_2$ pyridine), 4.59 (1H, br m, NHCONH), 4.66 (1H, br m OCH), 6.16 (1H, br s, NHCONH), 6.65-6.7 (2H, m, ArH meta to OMe), 6.75 (1H, d,J 8.2Hz, ArH ortho to OMe) 6.93 (2H, br m, pyridine $H_3$, $H_5$), 7.12 (2H, d, J 8.6Hz, ArH of $C_6H_4$), 7.18 (2H, d, J 8.6Hz, ArH of $C_6H_4$), and 8.39 (2H, br s, pyridine $H_2$, $H_6$); m/z (ESI) 460 ($M^+$+ 1, 100%), and 117 (16).

## EXAMPLE 33

**(±)-N-[4-{1-(3-Cyclopentyloxy-4-methoxyphenyl)}-2-(4-pyridyl)ethyl] phenylacetamide**

Acetyl chloride (62mg, 0.79mmol, 1.3eq) was added dropwise to the compound of Example 30 (235mg, 0.60mmol) in $CH_2Cl_2$ (20ml) at 0°C and the mixture allowed to stir at RT overnight. The reaction mixture was concentrated *in vacuo* and the residue subjected to chromatography ($SiO_2$; $CH_2Cl_2$/MeOH, 9:1) to afford the title compound (140mg) as a white solid; $\delta_H$ ($d_4$-MeOH) 1.5-2.0 (8H, br m, $(CH_2)_4$), 2.09 (3H, s, COMe), 3.37 (2H, d, J 8.2Hz, $CHCH_2$ pyridine), 3.75 (3H, s, OMe), 4.23 (1H, t, J 8.2Hz, $CHCH_2$ pyridine), 4.69 (1H, br m OCH), 6.73 (1H, d, J 1.9Hz, ArH ortho to cyclopentyloxy), 6.77 (1H, dd, J 8.2, 1.9Hz, ArH para to OMe), 6.82 (1H, d, J 8.2Hz, ArH ortho to OMe), 7.15 (2H, d, J 5.7Hz, pyridine $H_3$, $H_5$), 7.21 (2H, d, J 8.6Hz, ArH of $C_6H_4$), 7.42 (2H, d, J 8.6Hz, ArH of $C_6H_4$), and 8.27 (2H, br s, pyridine $H_2$, $H_6$) (N.B. NH not observed); m/z (ESI) 431 ($M^+$+ 1, 100%), and 338 (22).

**EXAMPLE 34**

**(±)-4-[2-(3-Cyclopentyloxy-4-methoxyphenyl)-2-(2-furyl)-2-hydroxyethyl]pyridine**

n-Butyllithium (1.6M solution in hexane; 16.9ml, 27mmol) was added to a stirred solution of furan (1.84g, 1.96ml, 27mmol) in THF (25ml) at -70°C. After 1h at -70°C, a solution of Intermediate 1 (4.0g, 18mmol) in THF (10ml) was added over 10 min. The reaction mixture was stirred at -70°C for 0.75h, warmed to RT over 0.75h, then quenched with water (100ml) and extracted with $Et_2O$ (3x60ml). The extract was washed with brine (100ml), dried ($MgSO_4$), and concentrated *in vacuo.* The residual orange-yellow oil was subjected to chromatography ($SiO_2$; $CH_2Cl_2$/hexane, 3:1, then $Et_2O$/hexane, 1:1) to give (3-cyclopentyloxy-4-methoxyphenyl)(2-furyl)methanol (3.2g, 61%) as a colourless unstable oil; $\nu_{max}$. (neat) 3500cm$^{-1}$.

The alcohol (3.2g) was stirred with manganese (IV) oxide (10g) in $CH_2Cl_2$ (100ml) at RT for 3h. The mixture was filtered through Celite® and the filtrate concentrated *in vacuo.* The residual dark oil was subjected to chromatography ($SiO_2$) to give (3-cyclopentyloxy-4-methoxyphenyl)-(2-furyl)ketone (1.9g); $\nu_{max}$ (neat) 1620cm$^{-1}$.

n-Butyllithium (1.6M solution in hexanes; 4.2ml, 6.64mmol) was added to a solution of a 4-methylpyridine (0.62g, 0.65ml, 6.64mmol) in THF (25ml) at -70°C. After 0.5h, a solution of the crude ketone (1.9g, ca. 6.6mmol) in THF (5ml) was added, stirred for 1h at -70°C, then at RT for 0.25h. The reaction mixture was quenched with water (50ml) and extracted with EtOAc (3x50ml). The extract was dried ($MgSO_4$), concentrated *in vacuo*, and the residual red oil subjected to chromatography ($SiO_2$; EtOAc/ hexane, 3:2) to afford the title compound (1.23g, 49%) as a pale yellow oil; $\delta_H$ ($CDCl_3$) 1.5-1.9 (8H, br m, $(CH_2)_4$), 2.84 (1H, br s, OH), 3.30 (1H, d, J 13.2Hz, $CH_AH_B$ pyridine), 3,59 (1H, d, J, 13.2Hz, $CH_AH_B$ pyridine), 3.82 (3H, s, OMe), 4.65 (1H, br m OCH), 6.24 (1H, dd, J 3.3, 0.7Hz, furan $H_3$), 6.35 (1H, dd, J 3.3, 1.8Hz, furan $H_4$), 6.75-6.85 (3H, m, $C_6H_3$), 6.85 (2H, dd, J 4.5, 1.6Hz, pyridine $H_3$, $H_5$), 7.43 (1H, dd, J 1.8, 0.7Hz, furan $H_5$), and 8.33 (2H, dd, J 4.5, 1.6Hz, pyridine $H_2$, $H_6$); m/z (ESI) 402 ($M^+$+ 23, 20%), 380 ($M^+$ + 1, 35), 287 (100), 95 (28), and 94 (97).

**EXAMPLE 35**

**(±)-2-[2-(3-Cyclopentyloxy-4-methoxyphenyl)-2-phenylethyl]benz[d] oxazole**

Bromobenzene (3.47g, 22.8mmol, 3.1equiv) was added to a stirred suspension of magnesium turnings (555mg,22.1mmol, 3.0equiv) in THF (10ml) and the mixture stirred for 0.5h. A further portion of THF (30ml) was added and stirring continued for 1h. Copper (I) bromide-dimethyl sulphide complex (2.27g, 11.04mmol), 1.5equiv) was added to the Grignard solution at -70°C then allowed to quickly warm to -20°C. After 0.5h, the yellow-green slurry was cooled to -70°C and treated with a solution of Intermediate 37 (3.0g, 7.37mmol) in THF (10ml) over 10 min. After 2h at -70°C, the reaction mixture was warmed to -20°C over 0.5h then quenched with aqueous $NH_4Cl$ solution (200ml) and extracted with EtOAc (150ml, 2x50ml). The extract was washed with $NH_4Cl$ solution (40ml), and brine (50ml), then dried ($MgSO_4$), and concentrated *in vacuo.* The residue was triturated with $Et_2O$/hexane (1:2; 50ml) to afford (4S)-3-[3-(3-cyclopentyloxy-4-methoxyphenyl)-3-phenylpropanoyl]-4-phenyl-2-oxazolone (2.97g) as a white solid; $\delta_H$ ($CDCl_3$) 1.5-1.95 (8H, br m, $(CH_2)_4$), 3.6-3.85 (2H, m, $CH_2CO$), 3.79 (3H, s, OMe), 4.18 (1H, dd, J 8.7, 4Hz, CHH'O), 4.5-4.6 (1H, m, $CHCH_2CO$), 4.58 (1H, apparent t, J 8.7Hz, CHH'O), 4.70 (1H, br m, ArOCH), 5.32 (1H, dd, J 8.7,4Hz, CHN), 6.75-6.8 (3H, m, $C_6H_3$), and 7.0-7.35 (10H, m, $2xC_6H_5$).

Hydrogen peroxide (27.5% w/w; 19.1g, 17.2ml, 155mmol) was added in small portions over 0.25h to a solution of the acyloxazolidinone (15.01g, 30.9mmol) in THF-$H_2O$ (4:1, 240ml) at around 5°C. After a further 5 min, aqueous LiOH solution (1.0M; 43.5ml, 43.5mmol) was added dropwise at 0-5°C and the reaction mixture maintained at <5°C overnight.

Sodium sulphite solution (1.0M; 171ml) was added in small portions at <20°C and the THF removed *in vacuo.* The residue was filtered to remove any solid and the filtrate washed with EtOAc (2x100ml). The aqueous layer was acidified to pH2 and extracted with EtOAc (3x100ml). The extract was washed with brine (40ml), then dried ($MgSO_4$), and concentrated *in vacuo* to afford 3-(3-cyclopentyloxy-4-methoxyphenyl)-3-phenylpropanoic acid (10.2g) as a colourless viscous oil.

The crude carboxylic acid (3.19g, 9.38mmol) in $CH_2Cl_2$ (10ml) was treated with thionyl chloride (2.7ml, 37.5mmol) and the mxiture heated to reflux for 3h. The reaction mixture was concentrated *in vacuo* and the residue azeotroped with toluene (2x25ml) to afford 3-(3-cyclopentyloxy-4-methoxyphenyl)-3-phenylpropanoyl chloride (2.96g) as a brown oil.

A solution of the acid chloride (1.48g, 4.13mmol) in $CH_2Cl_2$ (5ml) was added to a suspension of 2-aminophenol (0.90g, 8.26mmol) in $CH_2Cl_2$ (15ml) and the mixture stirred for 2h. The reaction mixture was diluted with $CH_2Cl_2$ (100ml), washed with 10% HCl (2x25ml), then dried ($Na_2SO_4$), and concentrated *in vacuo.* The residue was subjected to chromatography ($SiO_2$; EtOAc/hexane, 2:3) to afford an off-white foam (0.96g). The intermediate amide (400mg) was heated

at 150°C for 60h and the residue subjected to chromatography ($SiO_2$; EtOAc/hexane, 1:4) to afford the title compound (234mg) as a white solid m.p. 91.5-92.5°C (Found: C, 78.63; H, 6.68; N, 3.23. $C_{27}H_{27}NO_3$ requires C, 78.42; H,6.58; N, 3.39%); $\delta_H$ ($CDCl_3$) 1.5-1.9 (8H, br m, $(CH_2)_4$), 3.64 (2H, d, J 8.1Hz, $PhCHCH_2$), 3.77 (3H, s, OMe), 4.61 (1H, brm, OCH), 4.74 (1H, t, J 8.1Hz, $PhCHCH_2$), 6.7-6.8 (3H, m, $C_6H_3$), 7.1-7.3 (7H, m, ArH), 7.4-7.45 (1H, m, ArH), and 7.6-7.65 (1H, m, ArH); $\nu_{max}$ (KBr) 2960, 1620, 1600, 1530, 1240, and 1140 $cm^{-1}$; m/z (ESI) 436 ($M^+$ + 23, 100%), and 414 ($M^+$ + 1, 80); $[\alpha]_D^{22}$ = +64° (0.167g/100ml EtOH).

## EXAMPLE 36

### 5-[2-(3-Cyclopentyloxy-4-methoxyphenyl)-2-phenylethyl]-1-methylimidazole

Methylamine was bubbled into a stirred mixture of Intermediate 39 (1.0g), 3A molecular sieves (ca. 5g), and 4-toluenesulphonic acid monohydrate (ca. 50mg). After stirring at RT for 1.5h, a few drops of $Et_3N$ was added and the reaction mixture filtered using $Et_2O$ washing. The filtrate was concentrated *in vacuo* to give the intermediate imine as a near colourless oil (0.88g).

A mixture of the crude imine (0.88g), t-butylamine ().51ml), and (4-toluenesulphonyl)methyl isocyanide (TosMIC) (0.63g) in dimethoxyethane (30ml) was stirred at RT for 42h. The reaction mixture was then filtered and the filtrate partitioned between 20% HCl (50ml) and $Et_2O$ (50ml). The aqueous layer was separated and combined with further 20% HCl extracts (2x25ml). The acid extract was washed with $Et_2O$ (25ml), basified with solid KOH, then extracted with $Et_2O$ (3x40ml). The organic extract was washed with brine (20ml), dried ($MgSO_4$), and concentrated *in vacuo.* The residual dark oil (0.6g) was subjected to chromatography ($SiO_2$; EtOAc to 2% MeOH-EtOAc) to afford the title compound (206mg) as a clear pale yellow oil; δH ($CDCl_3$) 1.5-2.0 (8H, br m, $(CH_2)_4$), 3.2-3.3 (5H, m, NMe + $CHCH_2$ pyridine), 3.80 (3H, s, OMe), 4.14 (1H, t, J 7.7Hz, $CHCH_2$ pyridine), 4.65 (1H, br m, OCH), 6.6-6.8 (4H, m, $C_6H_3$ + imidazole $H_4$), and 7.15-7.35 (6H, m, $C_6H_5$ + imidazole $H_2$); m/z (ESI) 377 ($M^+$ + 1, 100%).

## FORMULATION EXAMPLES

The compounds of the invention may be formulated for pharmaceutical use in a number of forms using any suitable excipients. Thus, for example, for oral use the compounds of the invention such as the compounds of the Examples may be formulated as a solid dosage form, by mixing an appropriate weight of compound (for example 50mg) with maize starch (50-99%w/w), anhydrous colloidal silica (0-10%w/w) and organic or inorganic acid (up to 1%w/w), to fill capsules of an appropriate size, e.g. white opaque hard gelatine capsules size 3. If desired the same mixture may be compressed into tablets.

The activity and selectivity of compounds according to the invention was demonstrated in the following tests. In these tests the abbreviation FMLP represents the peptide N-formyl-met-leu-phe.

### Isolated Enzyme

The potency and selectivity of the compounds of the invention was determined using distinct PDE isoenzymes as follows:

i. PDE I, rabbit heart
ii. PDE II, rabbit heart
iii. PDE III, rabbit heart, Jurkat cells
iv. PDE IV, HL60 cells, rabbit brain, rabbit kidney and human recombinant PDE IV
v. PDE V, rabbit lung, guinea pig lung

A gene encoding human PDE IV has been cloned from human monocytes (*Livi, et al., 1990, Molecular and Cellular Biology, 10, 2678*). Using similar procedures we have cloned human PDE IV genes from a number of sources including eosinophils, neutrophils, lymphocytes, monocytes, brain and neuronal tissues. These genes have been transfected into yeast using an inducible vector and various recombinant proteins have been expressed which have the biochemical characteristics of PDE IV (*Beavo and Reifsnyder, 1990, TIPS, 11, 150*). These recombinant enzymes, particularly the human eosinophil recombinant PDE IV, have been used as the basis of a screen for potent, selective PDE IV inhibitors.

The enzymes were purified to isoenzyme homogeneity using standard chromatographic techniques.

Phosphodiesterase activity was assayed as follows. The reaction was conducted in 150μl of standard mixture containing (final concentrations): 50mM 2-[[tris(hydroxymethyl)methyl]amino]-1-ethane-sulphonic acid (TES) -NaOH buffer (pH 7.5), 10mM $MgCl_2$, 0.1μM [$^3$H]-cAMP and vehicle or various concentrations of the test compounds. The

reaction was initiated by addition of enzyme and conducted at 30°C for between 5 to 30 mins. The reaction was terminated by addition of 50µl 2% trifluoroacetic acid containing [$^{14}C$]-5'AMP for determining recovery of the product. An aliquot of the sample was then applied to a column of neutral alumina and the [$^{3}H$]-cAMP eluted with 10ml 0.1 TES-NaOH buffer (pH8). The [$^{3}H$]-5'-AMP product was eluted with 2ml 2M NaOH into a scintillation vial containing 10ml of scintillation cocktail. Recovery of [$^{3}H$]-5'AMP was determined using the [$^{14}C$]-5'AMP and all assays were conducted in the linear range of the reaction.

Compounds according to the invention such as compounds of the Examples herein cause a concentration-dependent inhibition of recombinant PDE IV at 0.1 - 1000nM with little or no activity against PDE I, II, III or V at concentrations up to 100µM.

2. **The Elevation of cAMP in Leukocytes**

The effect of compounds of the invention on intracellular cAMP was investigated using human neutrophils or guinea pig eosinophils. Human neutrophils were separated from peripheral blood, incubated with dihydrocytochalasin B and the test compound for 10 min and then stimulated with FMLP. Guinea pig eosinophils were harvested by peritoneal lavage of animals previously treated with intraperitoneal injections of human serum. Eosinophils were separated from the peritoneal exudate and incubated with isoprenaline and test compound. With both cell types, suspensions were centrifuged at the end of the incubation, the cell pellets were resuspended in buffer and boiled for 10 min prior to measurement of cAMP by specific radioimmunoassay (DuPont).

The most potent compounds according to the Examples induced a concentration -dependent elevation of cAMP in neutrophils and/or eosinophils at concentrations of 0.1nM to 1µM.

3. **Suppression of Leukocyte Function**

Compounds of the invention were investigated for their effects on superoxide generation, chemotaxis and adhesion of neutrophils and eosinophils. Isolated leukocytes were incubated with dihydrocytochalasin B for superoxide generation only and test compound prior to stimulation with FMLP. The most potent compounds of the Examples caused a concentration-dependent inhibition of superoxide generation, chemotaxis and adhesion at concentrations of 0.1nM to 1µM.

Lipopolysaccharide (LPS)-induced synthesis of tumour necrosis factor (TNF) by human peripheral blood monocytes (PBM) is inhibited by compounds of the Examples at concentrations of 0.01nM to 10µM.

4. **Relaxation of Constricted Airway Smooth Muscle in vitro**

The effects of compounds of the invention on guinea-pig isolated tracheal smooth muscle were investigated. Isolated tracheal rings were suspended in organ baths and immersed in oxygenated Krebs' solution. The smooth muscle was contracted with sub-maximal concentrations of histamine or carbachol prior to the addition of increasing concentrations of test compound to the organ baths. The most potent compounds of the Examples caused a concentration-dependent reversal of both histamine and carbachol-induced contractions at concentrations of 1nM to 100µM. The compounds were generally more potent in reversing histamine-induced tone than carbachol-induced tone.

5. **Effects on Cardiac Muscle in vitro**

Compounds of the invention have been tested for their effects on isolated cardiac muscle. Right atrial and papillary muscles were dissected out from the hearts of guinea pigs and suspended in organ baths for measuring the rate (chronotropic) of spontaneously beating atria and force (inotropic) of the electrically stimulated papillary muscle. In these preparations, selective PDE IV inhibitors such as rolipram do not have any direct effects whereas selective PDE III inhibitors such as milrinone have positive chronotropic and inotropic effects. The non-specific PDE inhibitor theophylline, which is used in asthma as a bronchodilator, also causes significant cardiovascular changes such as tachycardia. Selective PDE IV inhibitors have advantage over theophylline, therefore, through reduced cardiovascular side effects. The most potent and selective compounds of the Examples had no direct effects on the atrial and papillary muscles in vitro at concentrations up to 10µM but in combination with PDE III inhibitors, these inhibitors showed an enhancement of chronotropic and inotropic activity, typical of selective type IV inhibitors.

6. **Anti-inflammatory Activity in vivo**

Interleukin-5 (IL-5)-induced pleural eosinophilia in the rat (*Lisle*, *et al*, *1993*, *Br.J. Pharmacol*. *108*, *230p*) is inhibited by compounds of the Examples given orally at doses of 0.0001 to 10.0mg/kg. The most potent compounds

cause a dose-dependent reduction in migrating eosinophils with $ED_{50}$s of 0.003 to 0.03mg/kg p.o.

Compounds of the invention also reduce the inflammatory responses induced in rats by platelet activating factor (PAF).

7. **Anti-allergic Activity in vivo**

Compounds of the invention have been tested for effects on an IgE-mediated allergic pulmonary inflammation induced by inhalation of antigen by sensitised guinea pigs. Guinea pigs were initially sensitised to ovalbumin under mild cyclophosphamide-induced immunosuppression, by intraperitoneal injection of antigen in combinations with aluminium hydroxide and pertussis vaccine. Booster doses of antigen were given two and four weeks later and at six weeks, animals were challenged with aerosolised ovalbumin whilst under cover of an intraperitoneally administered anti-histamine agent (mepyramine). After a further 48h, bronchial alveolar lavages (BAL) were performed and the numbers of eosinophils and other leukocytes in the BAL fluids were counted. The lungs were also removed for histological examination for inflammatory damage. Administration of compounds of the Examaples (0.001-10mg/kg i.p. or p.o.), up to three times during the 48h following antigen challenge, lead to a significant reduction in the eosinophilia and the accumulation of other inflammatory leukocytes. There was also less inflammatory damage in the lungs of animals treated with compounds of the Examples.

8. **Effects on Pulmonary Dynamics**

Compounds of the invention (0.001-10mg/kg by oral or other route of aministration) reduce the allergic bronchoconstruction caused by antigen in sensitized guinea pigs.

Compounds of the invention have been tested for their effects on ozone-induced hyperreactivity of the airways of guinea pigs. Following the inhalation of ozone, guinea pigs become very much more sensitive to the bronchoconstrictor effects of inhaled histamine than naive animals (*Yeadon et al*, *1992*, *Pulmonary Pharm.*, *5*, *39*). There is a pronounced shift to the left (10-30 fold) of the dose response curve to histamine and a highly significant increase in the maximum increase in pulmonary resistance. Compounds of the Examples administered 1h prior to ozone by the intraperitoneal or oral (0.001-10mg/kg) route caused a dose-dependent inhibition of ozone-induced hyperreactivity.

9. **Adverse Effects**

Compounds of the invention are free from adverse effects following repeated overdosage to rats or dogs. For example, over administration of 125mg/kg/day of active compounds of the Examples to rats for 30 days is not associated with adverse toxicity.

The most potent compounds of the invention are 20-30 times less active than rolipram in inducing behavioural changes, sedation or emesis in rats, ferrets or dogs.

**Claims**

**1.** A compound of formula (1) which is an inhibitor of PDE IV:

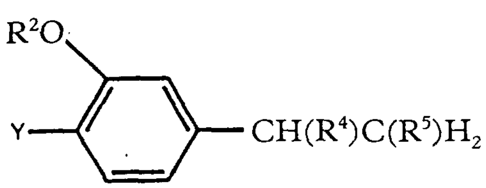

(1)

wherein

Y represents a group $OR^1$, where $R^1$ is a $C_{1-6}$ alkyl group optionally substituted by one or more halogen atoms; $R^2$ is a $C_{1-6}$ alkyl or $C_{2-6}$ alkenyl group (each optionally substituted by one , two or three halogen atoms or hydroxyl or $C_{1-6}$ alkoxy groups) or a $C_{3-8}$ cycloalkyl or $C_{3-8}$ cycloalkenyl group (each optionally substituted by one, two or three halogen atoms or $C_{1-6}$ alkyl, hydroxyl or $C_{1-6}$ alkoxy groups) ;

$R^4$ and $R^5$, which may be the same or different, is each a group Ar, where Ar is an optionally substituted monocyclic or bicyclic aryl group optionally containing one or more heteroatoms selected from oxygen, sulphur or nitrogen atoms;

and the salts, solvates, hydrates and N-oxides thereof; provided that -Y and -$OR^2$ are not both methoxy groups.

**2.** A compound according to claim 1 wherein $R^1$ is an optionally substituted methyl or ethyl group.

**3.** A compound according to claim 1 wherein $R^1$ is a methyl, -$CH_2F$, -$CH_2Cl$, -$CHF_2$, -$CF_3$ or -$CCl_3$ group.

**4.** A compound according to claim 1, wherein $R^1$ is a methyl group.

**5.** A compound according to any preceding claim wherein $R^2$ is a cyclopentyl group.

**6.** A compound according to claim 1 wherein Y is a -$OCH_3$ group and $R^2$ is a cyclopentyl group.

**7.** A compound according to any preceding claim wherein $R^4$ is a group Ar where Ar is an unsubstituted or substituted monocyclic aryl or monocyclic heteroaryl group and $R^5$ is a group Ar where Ar is an unsubstituted or substituted monocyclic nitrogen-containing heteroaryl group.

**8.** A compound according to claim 7 wherein the monocyclic aryl group is an unsubstituted or substituted phenyl group, the monocyclic heteroaryl group is an unsubstituted or substituted furyl, thienyl or pyridyl group and the monocyclic nitrogen-containing heteroaryl group is an unsubstituted or substituted pyridyl, pyridazinyl, pyrimidinyl or pyrazinyl group.

**9.** A compound according to claim 8 wherein the nitrogen-containing heteroaryl group is a substituted or unsubstituted pyridyl group.

**10.** A compound according to claim 1 selected from:

(±)-4-[2-(3-cyclopentyloxy-4-methoxyphenyl)-2-(2-furyl)ethyl]pyridine;
(±)-4-[2-(3-cyclopentyloxy-4-methoxyphenyl)-2-(2-thienyl)ethyl] pyridine;
(±)-4-[2-(3-cyclopentyloxy-4-methoxyphenyl)-2-phenylethyl]-3-methylimidazole;
(±)-4-[2-(3-cyclopentyloxy-4-methoxyphenyl)-2-phenylethyl]pyridine;
(±)-4-[1-(3-cyclopentyloxy-4-methoxyphenyl)-2-(4-pyridyl)ethyl]pyridine;
(±) -4-[2-(3-cyclopentyloxy-4-methoxyphenyl)-2-(4-fluorophenyl-ethyl] pyridine;
(±)-4-[2-(3-cyclopentyloxy-4-methoxyphenyl)-2-(4-trifluoromethyl-phenyl)ethyl]pyridine ;
(±)-4-[2-(3-cyclopentyloxy-4-methoxyphenyl)-2-(2-methoxyphenyl-ethyl)]pyridine;
(±)-4-[2-(3-cyclopentyloxy-4-methoxyphenyl)-2-(4-methoxyphenyl)-ethyl]pyridine;
(±)-4-[2-(3-cyclopentyloxy-4-methoxyphenyl)-2-(4-methylphenyl)ethyl]pyridine;
(±)-4- [2- (3-cyclopentyloxy-4-methoxyphenyl) -2- (3-methylphenyl)-ethyl] pyridine;
(±)-4-[2- (3-cyclopentyloxy-4-methoxyphenyl) -2- (3-cyclopentyloxy-4-methoxyphenyl)ethyl]pyridine;
(±)-4-[2-(3-cyclopentyloxy-4-methoxyphenyl)-2-phenylethyl]-3,5-dichloropyridine;
(±)-2-[2-(3-cyclopentyloxy-4-methoxyphenyl)-2-phenylethyl]pyridine;
(±)-4-[1-(3-cyclopentyloxy-4-methoxyphenyl)-2-(4-pyridyl)ethyl]aniline;
(±) -4-[1-(3-cyclopenxyloxy-4-methoxyphenyl)-2-(4-pyridyl)ethyl]benzoic acid;
(±) Ethyl N-{4-[1-(3-cyclopentyloxy-4-methoxyphenyl)-2-(4-pyridyl)ethyl]phenyl}carbamate;
(±) N-{4-[1-(3-cyclopentyloxy-4-methoxyphenyl)-2(4-pyridyl)ethyl]phenyl}N'-ethylurea;
(±) -N-{4-[1-(3-cyclopentyloxy-4-methoxyphenyl)]-2-(4pyridyl)ethyl}phenylacetamide;
(±) -3-[2-(3-cyclopentyloxy-4-methoxyphenyl)-2-phenylethyl]pyridine;
(±) -4-[2-(3-cyclopentyloxy-4-methoxyphenyl)-2-phenylethyl]pyrimidine;
(±) -4- [2-(3-cyclopentyloxy-4-methoxyphenyl)-2-(4-hydroxymethyl-phenyl)ethyl]pyridine;
(±) -4-[1-(3-cyclopentyloxy-4-methoxyphenyl)-2-(4-pyridyl)ethyl]benzamide;
(±) Ethyl-4-[1-(3-cyclopentyloxy-4-methoxyphenyl)-2-(4-phenyl-ethyl]benzoate;
(±) N-{4-[1-(3-cyclopentyloxy-4-methoxyphenyl)-2-(4-pyridyl)ethyl]phenyl}methanesulphonamide; or
the resolved enantiomers thereof; and the salts, solvates, hydrate and N-oxides thereof.

**11.** A compound according to claim 1 which is (+)-4-[2-(3-cyclo-pentyloxy-4-methoxyphenyl)-2-phenylethyl] pyridine;

and the salts, solvates, hydrates and N-oxides thereof.

**12.** A compound according to claim 1 which is (-)-4-[2-(3-cyclo-pentyloxy-4-methoxyphenyl)-2-phenylethyl]-pyridine; and the salts, solvates, hydrates and N-oxides thereof.

**13.** A compound according to claim 1 which is (+)-4-[1-(3-cyclo-pentyloxy-4-methoxyphenyl)-2-(4-pyridyl)ethyl]-pyridine.

**14.** A compound according to claim 1 which is (-)-4-[1-(3-cyclopentyloxy-4-methoxyphenyl)-2-(4-pyridyl)ethyl]-pyridine.

**15.** A pharmaceutical composition comprising a compound according to any preceding claim together with one or more pharmaceutically acceptable carriers, excipients or diluents.

**Patentansprüche**

**1.** Verbindung mit der Formel (1), die ein Hemmstoff der PDE IV ist,

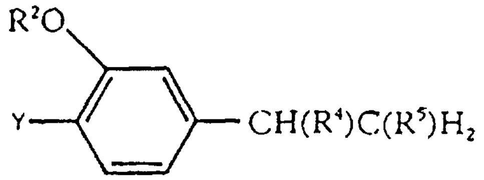

(1)

wobei

Y eine Gruppe $OR^1$ darstellt, bei der $R^1$ eine $C_{1-6}$-Alkylgruppe ist, die wahlweise durch ein oder mehrere Halogenatome substituiert ist,

$R^2$ eine $C_{1-6}$-Alkyl- oder $C_{2-6}$-Alkenylgruppe (jeweils wahlweise substituiert durch eine, zwei oder drei Halogenatome oder Hydroxyl- oder $C_{1-6}$-Alkoxygruppen) oder eine $C_{3-8}$-Cykloalkyl- oder eine $C_{3-8}$-Cykloalkenylgruppe (jeweils wahlweise substituiert durch ein bzw. eine, zwei oder drei Halogenatome oder $C_{1-6}$-Alkyl-, Hydroxyl- oder $C_{1-6}$-Alkoxygruppen) ist,

$R^4$ und $R^5$, die gleich oder unterschiedlich sein können, jeweils eine Gruppe Ar sind, bei der Ar eine wahlweise substituierte monozyklische oder bizyklische Arylgruppe ist, die wahlweise ein oder mehrere Heteroatome enthält, die aus Sauerstoff-, Schwefel- oder Stickstoffatomen ausgewählt sind,

und die Salze, Solvate, Hydrate und N-Oxide davon, vorausgesetzt, daß -Y und $-OR^2$ nicht beide Methoxygruppen sind.

**2.** Verbindung nach Anspruch 1, wobei $R^1$ eine wahlweise substituierte Methyl- oder Ethylgruppe ist.

**3.** Verbindung nach Anspruch 1, wobei $R^1$ eine Methyl-, $-CH_2F$-, $-CH_2Cl$-, $-CHF_2$-, $-CF_3$- oder $-CCl_3$-Gruppe ist.

**4.** Verbindung nach Anspruch 1, wobei $R^1$ eine Methylgruppe ist.

**5.** Verbindung nach einem der vorstehenden Ansprüche, wobei $R^2$ eine Cyclopentylgruppe ist.

**6.** Verbindung nach Anspruch 1, wobei Y eine $-OCH_3$-Gruppe und $R^2$ eine Cyclopentylgruppe ist.

**7.** Verbindung nach einem der vorstehenden Ansprüche, wobei $R^4$ eine Gruppe Ar ist, bei der Ar eine unsubstituierte oder substituierte monozyklische Aryl- oder monozyklische Heteroarylgruppe ist, und $R^5$ eine Gruppe Ar ist, bei der Ar eine unsubstituierte oder substituierte monozyklische Stickstoff enthaltende Heteroarylgruppe ist.

**8.** Verbindung nach Anspruch 7, wobei die monozyklische Arylgruppe eine unsubstituierte oder substituierte Phenylgruppe, die monozyklische Heteroarylgruppe eine unsubstituierte oder substituierte Furyl-, Thienyl- oder Pyridylgruppe und die monozyklische Stickstoff enthaltende Heteroarylgruppe eine unsubstituierte oder substituierte Pyridyl-, Pyridazinyl-, Pyrimidinyl- oder Pyrazinylgruppe ist.

**9.** Verbindung nach Anspruch 8, wobei die Stickstoff enthaltende Heteroarylgruppe eine substituierte oder unsubstituierte Pyridylgruppe ist.

**10.** Verbindung nach Anspruch 1, die ausgewählt ist aus:

(±) 4-[2-(3-Cyclopentyloxy-4-methoxyphenyl)-2-(2-furyl)-ethyl]pyridin,

(±) 4-[2-(3-Cyclopentyloxy-4-methoxyphenyl)-2-(2-thienyl)-ethyl]pyridin,

(±) 4-[2-(3-Cyclopentyloxy-4-methoxyphenyl)-2-phenylethyl]-3-methylimidazol,

(±) 4-[2-(3-Cyclopentyloxy-4-methoxyphenyl)-2-phenylethyl]-pyridin,

(±) 4-[1-(3-Cyclopentyloxy-4-methoxyphenyl)-2-(4-pyridyl)-ethyl]pyridin;

(±) 4-[2-(3-Cyclopentyloxy-4-methoxyphenyl)-2-(4-fluorphenylethyl]pyridin;

(±) 4-[2-(3-Cyclopentyloxy-4-methoxyphenyl)-2-(4-trifluormethylphenyl)ethyl]pyridin,

(±) 4-[2-(3-Cyclopentyloxy-4-methoxyphenyl)-2-(2-methoxyphenylethyl]pyridin,

(±) 4-[2-(3-Cyclopentyloxy-4-methoxyphenyl)-2-(4-methylphenyl)ethyl]pyridin,

(±) 4-[2-(3-Cyclopentyloxy-4-methoxyphenyl)-2-(4-methylphenyl)ethyl]pyridin,

(±) 4-[2-(3-Cyclopentyloxy-4-methoxyphenyl)-2-(3-methylphenyl)ethyl]pyridin,

(±) 4-[2-(3-Cyclopentyloxy-4-methoxyphenyl)-2-(3-cyclopentyloxy-4-methoxyphenyl)ethyl]pyridin,

(±) 4-[2-(3-Cyclopentyloxy-4-methoxyphenyl)-2-phenylethyl]-3,5-dichlorpyridin,

(±) 2-[2-(3-cyclopentyloxy-4-methoxyphenyl)-2-phenylethyl]-pyridin,

(±) 4- [1- (3-Cyclopentyloxy-4-methoxyphenyl) -2- (4-pyridyl)-ethyl]anilin,

(±) 4-[1-(3-Cyclopentyloxy-4-methoxyphenyl)-2-(4-pyridyl)-ethyl]benzoesäure,

(±) Ethyl-N-{4-[1-(3-cyclopentyloxy-4-methoxyphenyl)-2- (4-pyridyl)ethyl]phenyl}carbamat,

(±) N-{4-[1-(3-cyclopentyloxy-4-methoxyphenyl)-2-(4-pyridyl)ethyl]phenyl}N'-ethylharnstoff,

(±) N-{4-[1-(3-Cyclopentyloxy-4-methoxyphenyl)]-2-(4-pyridyl)ethyl}phenylacetamid,

(±) 3-[2-(3-Cyclopentyloxy-4-methoxyphenyl)-2-phenylethyl]-pyridin,

(±) 4-[2-(3-Cyclopentyloxy-4-methoxyphenyl)-2-phenylethyl]-pyrimidin,

(±) 4-[2-(3-Cyclopentyloxy-4-methoxyphenyl)-2-(4-hydroxymethylphenyl)ethyl]pyridin,

(±) 4-[1-(3-Cyclopentyloxy-4-methoxyphenyl)-2-(4-pyridyl)-ethyl]benzamid,

(±) Ethyl-4-[1-(3-cyclopentyloxy-4-methoxyphenyl)-2-(4-phenylethyl]benzoat,

(±) N-{4-[1-(3-Cyclopentyloxy-4-methoxyphenyl)-2-(4-pyridyl)ethyl]phenyl}methansulfonamid, oder

die getrennten Enantiomere davon; und die Salze, Solvate, Hydrate und N-Oxide davon.

**11.** Verbindung nach Anspruch 1, die (+) 4-[2-(3-Cyclopentyloxy-4-methoxyphenyl)-2-phenylethyl]pyridin ist, und die Salze, Solvate, Hydrate und N-Oxide davon.

**12.** Verbindung nach Anspruch 1, die (-) 4-[2-(3-Cyclopentyloxy-4-methoxyphenyl)-2-phenylethyl]pyridin ist, und die Salze, Solvate, Hydrate und N-Oxide davon.

**13.** Verbindung nach Anspruch 1, die (+) 4-[1-(3-Cyclopentyloxy-4-methoxyphenyl)-2-(4-pyridyl)ethyl]pyridin ist.

**14.** Verbindung nach Anspruch 1, die (-) 4-[1-(3-Cyclopentyloxy-4-methoxyphenyl)-2-(4-pyridyl)ethyl]pyridin ist.

**15.** Pharmazeutische Zusammensetzung, die eine Verbindung nach einem der vorstehenden Ansprüche zusammen mit einem oder mehreren pharmazeutisch zulässigen Trägern, Grundstoffen oder Verdünnungsmitteln aufweist.

**Revendications**

**1.** Composé de formule (1) qui est un inhibiteur de PDE IV :

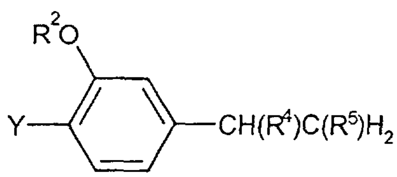

(1)

dans laquelle

Y représente un groupe $OR^1$, où $R^1$ est un groupe alkyle en $C_1$-$C_6$ éventuellement substitué par un ou plusieurs atomes d'halogène ;
$R^2$ est un groupe alkyle en $C_1$-$C_6$ ou alcényle en $C_2$-$C_6$ (chacun éventuellement substitué par un, deux ou trois atomes d'halogène, ou groupes hydroxyle ou alcoxy en $C_1$-$C_6$) ou un groupe cycloalkyle en $C_3$-$C_8$ ou cycloalcényle en $C_3$-$C_8$ (chacun éventuellement substitué par un, deux ou trois atomes d'halogène, ou groupes alkyle en $C_1$-$C_6$, hydroxyle ou alcoxy en $C_1$-$C_6$) ;
$R^4$ et $R^5$, qui peuvent être identiques ou différents, sont chacun un groupe Ar, où Ar est un groupe aryle monocyclique ou bicyclique éventuellement substitué, contenant éventuellement un ou plusieurs hétéroatomes choisis parmi les atomes d'oxygène, de soufre ou d'azote ;

et les sels, les produits de solvatation, les hydrates et les N-oxydes de ceux-ci ; à condition que -Y et -$OR^2$ ne soient pas tous deux des groupes méthoxy.

**2.** Composé selon la revendication 1, dans lequel $R^1$ est un groupe méthyle ou éthyle éventuellement substitué.

**3.** Composé selon la revendication 1, dans lequel $R^1$ est un groupe méthyle, $-CH_2F$, $-CH_2Cl$, $-CHF_2$, $-CHCl_2$, $-CF_3$ ou $-CCl_3$.

**4.** Composé selon la revendication 1, dans lequel $R^1$ est un groupe méthyle.

**5.** Composé selon l'une quelconque des revendications précédentes, dans lequel $R^2$ est un groupe cyclopentyle.

6. Composé selon la revendication 1, dans lequel Y est un groupe $-OCH_3$ et $R^2$ est un groupe cyclopentyle.

7. Composé selon l'une quelconque des revendications précédentes, dans lequel $R^4$ est un groupe Ar, où Ar est un groupe aryle monocyclique ou hétéroaryle monocyclique non substitué ou substitué et $R^5$ est un groupe Ar où Ar est un groupe hétéroaryle monocyclique azoté non substitué ou substitué.

8. Composé selon la revendication 7, dans lequel le groupe aryle monocyclique est un groupe phényle non substitué ou substitué, le groupe hétéroaryle monocyclique est un groupe furyle, thiényle ou pyridyle non substitué ou substitué, et le groupe hétéroaryle azoté monocyclique est un groupe pyridyle, pyridazinyle, pyrimidinyle ou pyrazinyle non substitué ou substitué.

9. Composé selon la revendication 8, dans lequel le groupe hétéroaryle azoté est un groupe pyridyle substitué ou non substitué.

10. Composé selon la revendication 1 choisi parmi :

la (±)-4-[2-(3-cyclopentyloxy-4-méthoxyphényl)-2-(2-furyl)éthyl]pyridine ;
la (±)-4-[2-(3-cyclopentyloxy-4-méthoxyphényl)-2-(2-thiényl)éthyl]pyridine ;
le (±) -4-[2-(3-cyclopentyloxy-4-méthoxyphényl)-2-phényléthyl]-3-méthylimidazole ;
la (±)-4-[2-(3-cyclopentyloxy-4-méthoxyphényl)-2-phényléthyl]pyridine ;
la (±)-4-[1-(3-cyclopentyloxy-4-méthoxyphényl)-2-(4-pyridyl)éthyl]pyridine ;
la (±)-4-[2-(3-cyclopentyloxy-4-méthoxyphényl)-2-(4-fluorophényl)éthyl]pyridine ;
la (±) -4- [2-(3-cyclopentyloxy-4-méthoxyphényl)-2-(4-trifluorométhylphényl)éthyl]pyridine ;
la (±)-4-[2-(3-cyclopentyloxy-4-méthoxyphényl)-2-(2-méthoxyphényl)éthyl]pyridine
la (±)-4-[2-(3-cyclopentyloxy-4-méthoxyphényl)-2-(4-méthoxyphényl)éthyl]pyridine
la (±)-4-[2-(3-cyclopentyloxy-4-méthoxyphényl)-2-(4-méthylphényl)éthyl]pyridine
la (±)-4-[2-(3-cyclopentyloxy-4-méthoxyphényl)-2-(3-méthylphényl)éthyl]pyridine ;
la (±)-4-[2-(3-cyclopentyloxy-4-méthoxyphényl)-2-(3-cyclopentyloxy-4-méthoxyphényl)éthyl]pyridine ;
la (±)-4- [2- (3-cyclopentyloxy-4-méthoxyphényl)-2-phényléthyl]-3,5-dichloropyridine ;
la (±)-2-[2-(3-cyclopentyloxy-4-méthoxyphényl)-2-phényléthyl]pyridine ;
la (±)-4-[1-(3-cyclopentyloxy-4-méthoxyphényl)-2-(4-pyridyl)éthyl]aniline ;
l'acide (±)-4-[1-(3-cyclopentyloxy-4-méthoxyphényl)-2-(4-pyridyl)éthyl]benzoïque ;
le N-{4-[1-(3-cyclopentyloxy-4-méthoxyphényl)-2-(4-pyridyl)éthyl]phényl}carbamate d'éthyle ;
la N-{4-[1-(3-cyclopentyloxy-4-méthoxyphényl)-2-(4-pyridyl)éthyl]phényl}-N-éthylurée ;
le N-{4-[1-(3-cyclopentyloxy-4-méthoxyphényl)]-2-(4-pyridyl)éthyl}phénylacétamide ;
la (±)-3-[2-(3-cyclopentyloxy-4-méthoxyphényl)-2-phényléthyl]pyridine ;
la (±)-4-[2-(3-cyclopentyloxy-4-méthoxyphényl)-2-phényléthyl]pyrimidine ;
la (±)-4-[2-(3-cyclopentyloxy-4-méthoxyphényl)-2-(4-hydroxyméthylphényl)éthyl]pyridine ;
le (±)-4-[1-(3-cyclopentyloxy-4-méthoxyphényl)-2-(4-pyridyl)éthyl]benzamide ;
le 4-[1-(3-cyclopentyloxy-4-méthoxyphényl)-2-(4-phényl)éthyl]benzoate d'éthyle ;
le (±)-N-{4-[1-(3-cyclopentyloxy-4-méthoxyphényl)-2-(4-pyridyl)éthyl]phényl}méthanesulfonamide ; ou

les énantiomères résolus de ceux-ci ; et les sels, les produits de solvatation, les hydrates et les N-oxydes de ceux-ci.

11. Composé selon la revendication 1 qui est la (+)-4-[2-(3-cyclopentyloxy-4-méthoxyphényl)-2-phényléthyl]-pyridine ; et les sels, les produits de solvatation, les hydrates et les N-oxydes de ceux-ci.

12. Composé selon la revendication 1 qui est la (-)-4-[2-(3-cyclopentyloxy-4-méthoxyphényl)-2-phényléthyl]-pyridine ; et les sels, les produits de solvatation, les hydrates et les N-oxydes de ceux-ci.

13. Composé selon la revendication 1 qui est la (+)-4-[1-(3-cyclopentyloxy-4-méthoxyphényl)-2-(4-pyridyl)éthyl]-pyridine.

14. Composé selon la revendication 1 qui est la (-)-4-[1-(3-cyclopentyloxy-4-méthoxyphényl)-2-(4-pyridyl)éthyl]-pyridine.

15. Composition pharmaceutique comprenant un composé selon l'une quelconque des revendications précédentes

avec un ou plusieurs véhicules, excipients ou diluants pharmaceutiquement acceptables.